(19) Europäisches Patentamt
European Patent Office
Office européen des brevets

(11) EP 4 144 375 A1

(12) EUROPEAN PATENT APPLICATION
published in accordance with Art. 153(4) EPC

(43) Date of publication:
08.03.2023 Bulletin 2023/10

(21) Application number: 21796251.3

(22) Date of filing: 29.04.2021

(51) International Patent Classification (IPC):
*A61K 47/64* (2017.01) *A61K 38/16* (2006.01)
*A61K 38/26* (2006.01) *A61K 38/18* (2006.01)
*A61K 38/22* (2006.01) *A61K 38/20* (2006.01)
*A61K 47/65* (2017.01) *A61K 47/68* (2017.01)
*A61K 47/60* (2017.01)

(52) Cooperative Patent Classification (CPC):
A61K 38/16; A61K 38/18; A61K 38/20; A61K 38/22; A61K 38/26; A61K 47/54; A61K 47/60; A61K 47/64; A61K 47/65; A61K 47/68; A61P 1/16

(86) International application number:
PCT/KR2021/005481

(87) International publication number:
WO 2021/221482 (04.11.2021 Gazette 2021/44)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 29.04.2020 KR 20200052866

(71) Applicant: Onegene Biotechnology Inc.
Suwon-si Gyeonggi-do 16229 (KR)

(72) Inventors:
- KIM, Yunki
Suwon-si Gyeonggi-do 16711 (KR)
- KIM, Minsun
Hwaseong-si Gyeonggi-do 18488 (KR)
- KIM, Ryuryun
Suwon-si Gyeonggi-do 16508 (KR)
- CHOI, Jaeyoung
Suwon-si Gyeonggi-do 16407 (KR)
- YIM, Yeseal
Uiwang-si Gyeonggi-do 16042 (KR)
- SHIM, Myungbo
Ansan-si Gyeonggi-do 15454 (KR)
- HAN, Daye
Hwaseong-si Gyeonggi-do 18322 (KR)
- IM, Daeseong
Yongin-si Gyeonggi-do 17084 (KR)
- PARK, Sungjin
Seongnam-si Gyeonggi-do 13540 (KR)

(74) Representative: Callaghan, Dayle Anne
Kilburn & Strode LLP
Lacon London
84 Theobalds Road
London WC1X 8NL (GB)

Remarks:
The complete document including Reference Table(s) and the Sequence Listing(s) can be downloaded from the EPO website

# (54) NOVEL PROTEIN CONJUGATE, AND USE THEREOF FOR PREVENTING OR TREATING NONALCOHOLIC STEATOHEPATITIS, OBESITY AND DIABETES

(57) The present invention relates to a protein conjugate comprising polyubiquitin, a carrier linked to the polyubiquitin, and two or more biomolecules linked to the polyubiquitin or the carrier. In addition, the present invention relates to a pharmaceutical composition for preventing or treating non-alcoholic steatohepatitis (NASH), fatty liver, liver fibrosis, cirrhosis, liver cancer, obesity, and diabetes, comprising the protein conjugate that comprises two or more biomolecules.

EP 4 144 375 A1

【Fig. 2】

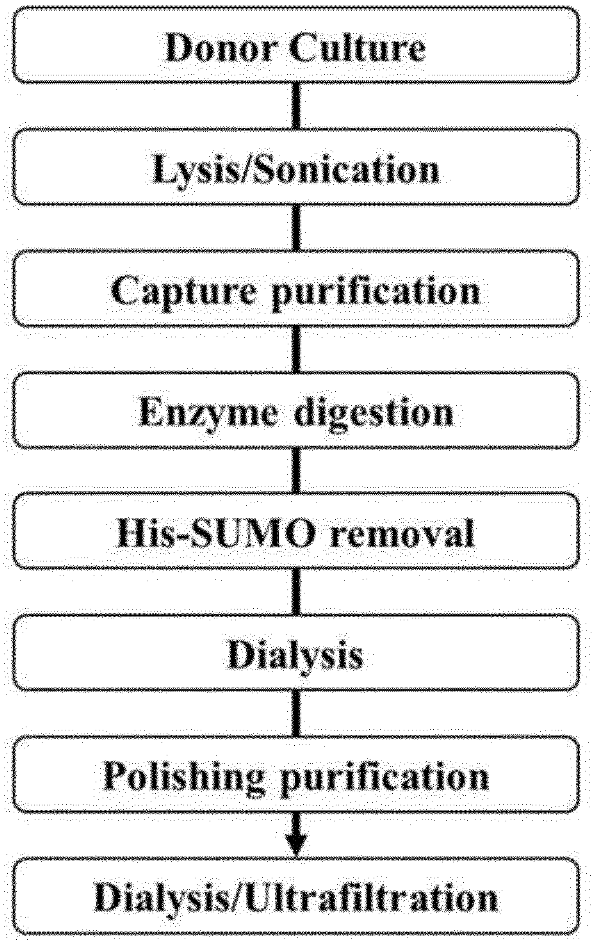
Donor Culture
Lysis/Sonication
Capture purification
Enzyme digestion
His-SUMO removal
Dialysis
Polishing purification
Dialysis/Ultrafiltration

## Description

### Technical Field

The present invention relates to a protein conjugate comprising polyubiquitin, a carrier linked to the polyubiquitin, and two or more biomolecules linked to the polyubiquitin or the carrier. In addition, the present invention relates to a pharmaceutical composition for preventing or treating non-alcoholic steatohepatitis, fatty liver, liver fibrosis, cirrhosis, liver cancer, obesity, and diabetes, comprising the protein conjugate that comprises two or more biomolecules.

### Background Art

Non-alcoholic fatty liver disease (NAFLD) is a type of disease that shows similar histological manifestation to alcoholic hepatitis even with little or no alcohol intake, and is a type of metabolic syndrome that covers non-alcoholic fatty liver (NAFL), non-alcoholic steatohepatitis (NASH), cirrhosis, and liver cancer (hepatocellular carcinomas). Non-alcoholic fatty liver disease is on the rise as the population with obesity and diabetes increases, and the annual incidence rate in Korea is about 16%.

Non-alcoholic steatohepatitis (NASH) is mainly characterized by abnormal fat accumulation or deposition in the liver (hepatic steatosis), liver inflammation and liver damage or liver tissue damage (fibrosis). The worldwide prevalence of non-alcoholic steatohepatitis is 2-4% (3-12% of adults in the United States). It is well known that non-alcoholic steatohepatitis exhibits a faster histological progression and can progress to cirrhosis, whereas simple steatosis exhibits a slow histological progression. About 5-10% of those diagnosed with fatty liver develop steatohepatitis (Metabolism Clinical and Experimental 65 (2016) 1038-1048).

Currently, there is no therapeutic agent on the market for treating non-alcoholic steatohepatitis, and due to the absence of a therapeutic agent therefor, other therapeutic agents for metabolic syndrome such as abdominal obesity, hyperlipidemia, and diabetes, for example, insulin resistance improving drugs, antioxidants (for example, Vitamins C and E), dyslipidemia therapeutic agents, hepatoprotective agents, and the like are used, but these are hardly considered to be therapeutic agents for directly treating non-alcoholic steatohepatitis.

Since non-alcoholic steatohepatitis has the nature of a complex disease, the licensing authorities in the United States, Europe, and the like have set strict drug approval requirements for therapeutic agents therefor. For this reason, recently, a number of therapeutic agents have failed in the clinical development stage, and accordingly, therapeutic agents based on a multiple agonist for simultaneously improving various indicators are emerging. Non-alcoholic steatohepatitis therapeutic agents based on a multiple agonist, which are currently in clinical trials, includes MEDI0382 from AstraZeneca, a GLP-1/glucagon (GCG) dual agonist, LY3298176 from Eli Lilly, a GIP/GLP-1 dual agonist, HM15211 from Hanmi Pharm.Co.,Ltd., a glucagon/GIP/GLP-1 triple agonist, and the like.

Obesity refers to a state of excess body fat, and can be defined as a health risk condition due to excess body fat that causes several diseases, including heart disease. The World Health Organization (WHO) has reported that there are more than 106 million overweight adults worldwide, at least 4 million are obese, and more than 2 in 3 Americans are overweight or obese (Low et al, 2009; Cooke and Bloom, 2006). Obese people have an increased risk of several diseases, including type 2 diabetes, hyperlipidemia, arthritis and apnea, as compared to those having normal body weight, and obesity is known to cause several types of cancer and heart disease.

Diabetes mellitus is a metabolic disease in which a high blood glucose level persists for a long time due to insufficient insulin secretion or insulin resistance. As the blood glucose level in the body persists for a long time, glycation products invade the retina, kidneys, nerves, or large and small blood vessels throughout the body, causing chronic complications. Since diabetes complications are more dangerous than diabetes mellitus itself, the biggest goal in diabetes treatment today is to suppress the occurrence or progression of diabetes complications. Representative complications of diabetes include diabetic retinopathy, diabetic cataract, diabetic nephropathy, diabetic neuropathy, diabetic heart disease, diabetic osteoporosis, diabetic atherosclerosis, and the like.

In the development of therapeutic agents based on a multiple agonist, due to the problem of reduced activity due to structural limitations, currently there are no studies on therapeutic agents based on a higher than quadruple agonist for non-alcoholic steatohepatitis, fatty liver, liver fibrosis, cirrhosis, liver cancer, obesity, or diabetes. Accordingly, the present inventors have worked tirelessly to develop a therapeutic agent based on a quadruple agonist/antagonist for non-alcoholic steatohepatitis, fatty liver, liver fibrosis, cirrhosis, liver cancer, obesity, and diabetes. As a result, the present invention was completed using a polyubiquitin structure.

## Prior Art Document

### Patent Document

(Patent Document 0001) Korean Patent Application Publication No. 10-2016-0032699
(Patent Document 0002) Korean Patent No. 10-2034607

## Detailed Description of Invention

### Technical Problem

An object of the present invention is to provide a protein conjugate comprising polyubiquitin, a carrier linked to the polyubiquitin, and two or more biomolecules linked to the polyubiquitin or the carrier.

In addition, the present invention provides a pharmaceutical composition for preventing or treating non-alcoholic steatohepatitis (NASH), fatty liver, liver fibrosis, cirrhosis, liver cancer, obesity, or diabetes, comprising the protein conjugate that comprises two or more biomolecules. Specifically, another object of the present invention is to provide a pharmaceutical composition for preventing or treating non-alcoholic steatohepatitis, fatty liver, liver fibrosis, cirrhosis, liver cancer, obesity, or diabetes, comprising a GCG/GLP-1/FGF21/GIP or GCG/GLP-1/FGF21/IL-1RA acceptor quadruple agonist/antagonist.

### Solution to Problem

The present invention provides a protein conjugate, characterized in that the protein conjugate comprises: polyubiquitin, a carrier linked directly or by a linker to the polyubiquitin, and a biomolecule linked directly or by a linker to the polyubiquitin or the carrier, wherein the polyubiquitin is composed of (i) an acceptor ubiquitin containing one or more unsubstituted lysines in which lysines of the ubiquitin may be substituted with arginine or alanine, and (ii) a donor ubiquitin in which all lysines of the ubiquitin are substituted with arginine or alanine, and the biomolecule is two or more selected from the group consisting of GCG, GLP-1, FGF21, GIP and IL-1RA; analogues thereof; a GCG and GLP-1 dual acceptor agonist; and a GLP-1 and GIP dual acceptor agonist.

In one embodiment, the GCG and analogue thereof may be selected from the group consisting of proteins composed of the amino acid sequences of SEQ ID NOs: 1 to 3, and preferably, the GCG analogue may be a protein composed of the amino acid sequence of SEQ ID NO: 2.

In one embodiment, the GLP-1 and analogue thereof may be selected from the group consisting of proteins composed of the amino acid sequences of SEQ ID NOs: 4 to 14, and preferably, the GLP-1 analogue may be a protein composed of the amino acid sequence of SEQ ID NO: 12.

In one embodiment, the GCG and GLP-1 dual acceptor agonist may be selected from the group consisting of proteins composed of the amino acid sequences of SEQ ID NOs: 15 and 16.

In one embodiment, the FGF21 and analogue thereof may be selected from the group consisting of proteins composed of the amino acid sequences of SEQ ID NOs: 17 to 21. Preferably, the FGF21 analogue may be a protein composed of the amino acid sequence of SEQ ID NO: 20.

In one embodiment, the GIP and analogue thereof may be selected from the group consisting of proteins composed of the amino acid sequences of SEQ ID NOs: 22 to 26. Preferably, the GIP and analogue thereof may be a protein composed of the amino acid sequence of SEQ ID NO: 24.

In one embodiment, the IL-1RA and analogue thereof may be selected from the group consisting of proteins composed of the amino acid sequences of SEQ ID NOs: 27 and 28. Preferably, the IL-1RA may be a protein composed of the amino acid sequence of SEQ ID NO: 27.

In one embodiment, the polyubiquitin may be composed of an acceptor ubiquitin in which the 6th, 11th, 27th, 29th, 33rd, and 48th lysines from the N-terminus of the ubiquitin are substituted with arginine and a donor ubiquitin in which all lysines of the ubiquitin are substituted with arginine. Preferably, the polyubiquitin may be composed of an acceptor ubiquitin composed of the amino acid sequence of SEQ ID NO: 30 and a donor ubiquitin composed of the amino acid sequence of SEQ ID NO: 31.

In one embodiment, the linker may be a polypeptide composed of an amino acid sequence in which 1 to 30 repeats of GGGGS, EAAAK or VPPPPP are combined. Preferably, the linker may be a polypeptide composed of the amino acid sequence of SEQ ID NO: 29.

In one embodiment, the carrier may be selected from the group consisting of albumin, antibody fragment, scFc (single chain Fc), scFc dimer (single chain Fc-dimer), transferrin, XTEN (genetic fusion of non-exact repeat peptide

sequence), CTP (carboxy-terminal peptide), PAS (proline-alanine-serine polymer), ELK (elastin-like peptide), HAP (homo-amino acid polymer), GLK (gelatin-like protein), PEG (polyethylene glycol), and fatty acid. Preferably, the carrier may be albumin.

The present invention provides a protein conjugate represented by the following formula:

X-L-Y-L-Ub(A)-L-A-L-Z
|
W-L-Ub(D)

in the above formula, W, X, Y and Z may be each a biomolecule selected from the group consisting of a GCG analogue composed of the amino acid sequence of SEQ ID NO: 2, a GLP-1 analogue composed of the amino acid sequence of SEQ ID NO: 12, an FGF21 analogue composed of the amino acid sequence of SEQ ID NO: 20, a GIP analogue composed of the amino acid sequence of SEQ ID NO: 24, and IL-1RA composed of the amino acid sequence of SEQ ID NO: 27, Ub(A) may be an acceptor ubiquitin composed of the amino acid sequence of SEQ ID NO: 30, Ub(D) may be a donor ubiquitin composed of the amino acid sequence of SEQ ID NO: 31, L may be each independently absent or a linker, A may be a carrier, and Ub(A) and Ub(D) may be linked by a covalent bond.

In one embodiment, X may be a GCG analogue composed of the amino acid sequence of SEQ ID NO: 2, Y may be a GLP-1 analogue composed of the amino acid sequence of SEQ ID NO: 12, Z may be an FGF21 analogue composed of the amino acid sequence of SEQ ID NO: 20, and W may be a GIP analogue composed of the amino acid sequence of SEQ ID NO: 24 or IL-1RA composed of the amino acid sequence of SEQ ID NO: 27.

In one embodiment, the linker may be a polypeptide composed of the amino acid sequence of SEQ ID NO: 29, and the carrier may be albumin.

The present invention provides a pharmaceutical composition for preventing or treating non-alcoholic steatohepatitis (NASH), fatty liver, liver fibrosis, cirrhosis, liver cancer, obesity, or diabetes, comprising the protein conjugate.

The present invention provides a method for preventing or treating non-alcoholic steatohepatitis (NASH), fatty liver, liver fibrosis, cirrhosis, liver cancer, obesity, or diabetes, comprising administering the composition to a subject other than a human.

In addition, the present invention provides a method for preparing a protein conjugate, characterized in that the method comprises the steps of:

(i) preparing an acceptor protein represented by the following formula;

X-L-Y-L-Ub(A)-L-A-L-Z

(ii) preparing a donor protein represented by the following formula; and

W-L-Ub(D)

(iii) linking Ub(A) in the acceptor protein and Ub(D) in the donor protein,

wherein W, X, Y and Z are each a biomolecule selected from the group consisting of a GCG analogue composed of the amino acid sequence of SEQ ID NO: 2, a GLP-1 analogue composed of the amino acid sequence of SEQ ID NO: 12, an FGF21 analogue composed of the amino acid sequence of SEQ ID NO: 20, a GIP analogue composed of the amino acid sequence of SEQ ID NO: 24, and IL-1RA composed of the amino acid sequence of SEQ ID NO: 27, Ub(A) is an acceptor ubiquitin composed of the amino acid sequence of SEQ ID NO: 30, Ub(D) is a donor ubiquitin composed of the amino acid sequence of SEQ ID NO: 31, L is each independently absent or a linker, and A is a carrier.

## Effects of Invention

The novel protein conjugate of the present invention comprises two or more biomolecules, and inhibits steatosis, inflammation, and fibrosis in the liver, thereby having an excellent effect on preventing or treating non-alcoholic steatohepatitis (NASH), fatty liver, liver fibrosis, cirrhosis, or liver cancer, and thus, can be also usefully used for the prevention or treatment of obesity or diabetes. In addition, the protein conjugate can be maintained for a long time in the body to exert its effect for a long time, and has excellent safety by using polyubiquitin and a carrier harmless to the human body.

## Brief Description of Drawings

Figure 1 illustrates the results obtained by confirming the purification of an acceptor protein by SDS-PAGE.
Figure 2 is a schematic diagram showing the purification process of a donor protein.
Figures 3 and 4 illustrate the results obtained by confirming the purification of a donor protein through a Ni column by SDS-PAGE.
Figures 5 and 6 illustrate the results obtained by confirming the purification of a donor protein through His-SUMO removal by SDS-PAGE.
Figures 7 and 8 illustrate the results of polishing purification of a donor protein.
Figure 9 is a schematic diagram showing a process of preparing a protein conjugate through conjugation of an acceptor protein and a donor protein.
Figures 10 and 11 illustrate the results obtained by confirming conjugation of an acceptor protein and a donor protein by SDS-PAGE.
Figure 12 illustrates the results obtained by confirming the Ni purification of a protein conjugate by chromatography.
Figures 13 and 14 illustrate the results obtained by confirming the purification of a protein conjugate by SDS-PAGE.
Figure 15 illustrates the results of SDS-PAGE of a final protein conjugate.
Figures 16 to 18 are graphs showing the results of the cAMP accumulation assay.
Figure 19 is a graph showing the results of the FGFR/KLB functional assay.
Figure 20 is a graph showing the results of the NF-κB reporter assay.
Figures 21 and 22 are graphs showing the results obtained by measuring alanine aminotransferase (ALT) and aspartate aminotransferase (AST) in mouse blood in an in-vivo efficacy test.
Figures 23 to 26 illustrate the results obtained by observing steatosis and inflammation (lobular inflammation) in the mouse liver tissue in an in-vivo efficacy test and the scores according to the NAS evaluation criteria.
Figures 27 and 28 are graphs showing the results obtained by measuring TGT-β and triglyceride concentrations in the mouse liver tissue in an in-vivo efficacy test.
Figures 29 and 30 are graphs showing the results obtained by measuring alanine aminotransferase (ALT) and aspartate aminotransferase (AST) in mouse blood in an in-vivo efficacy test.
Figures 31 to 34 illustrate the results obtained by observing steatosis and inflammation (lobular inflammation) in the mouse liver tissue in an in-vivo efficacy test and the scores according to the NAS evaluation criteria.
Figures 35 and 36 are graphs showing the results obtained by measuring TGT-β and triglyceride concentrations in the mouse liver tissue in an in-vivo efficacy test.
Figures 37 to 41 are graphs showing the changes in triglyceride (TG), free fatty acid (NEFA), total cholesterol (T-Chol), low-density lipoprotein cholesterol (LDL-cholesterol) and high-density lipoprotein cholesterol (HDL-cholesterol) in the serum of diet-induced obesity mice in an in-vivo efficacy test.
Figures 42 and 43 are graphs showing the changes in non-fasting blood glucose of type 2 diabetes mice in an in-vivo efficacy test.
Figures 44 and 45 are graphs showing the changes in body weight of type 2 diabetes mice in an in-vivo efficacy test.
Figures 46 and 47 are graphs showing the changes in food consumption of type 2 diabetes mice in an in-vivo efficacy test.
Figures 48 and 49 are graphs showing the changes in water consumption of type 2 diabetes mice in an in-vivo efficacy test.
Figures 50 to 52 illustrate the results of the *in silico* immunogenicity test in MHC Class I.
Figures 53 to 55 illustrate the results of the *in silico* immunogenicity test in MHC Class II.

## Best Mode for Carrying out the Invention

Hereinafter, with reference to the accompanying drawings, embodiments and examples of the present invention will be described in detail so that those of ordinary skill in the art to which the present invention pertains can easily practice the present invention. However, the present application may be embodied in various forms and is not limited to the embodiments and examples described herein.

Throughout the present specification, when a certain part "includes" a certain component, it means that other components may be further included, rather than excluding other components, unless otherwise stated.

As used herein, the term "prevention" refers to any action of inhibiting or delaying the onset of a disease by administration of a composition, and "treatment" refers to any action in which symptoms of a subject suspected of and suffering from a disease are improved or beneficially changed by administration of a composition.

As used herein, the term "subject" is a mammal, preferably a human, but may be an animal including a companion

animal (for example, dog, cat, etc.), a domestic animal (for example, cow, sheep, pig, horse, etc.) and a laboratory animal (for example, rat, mouse, guinea pig, etc.).

The pharmaceutical composition of the present invention may be administered parenterally or orally depending on a desired method, and the dosage may vary depending on the patient's body weight, age, sex, health status, diet, administration time, administration method, excretion rate, the severity of the disease, and the like. In addition, the therapeutically effective amount of the composition may vary depending on the administration method, the target site, and the condition of the patient, and when used in the human body, the dosage should be determined as an appropriate amount in consideration of both safety and efficiency.

As used herein, the term "GCG" may refer to a wild type glucagon (native GCG), which is a protein composed of the amino acid sequence of SEQ ID NO: 1.

As used herein, the term "GCG analogue" means that some amino acids of a wild type glucagon protein are substituted. Preferably, it may refer to a protein composed of the amino acid sequence of SEQ ID NO: 2 or 3. More preferably, it may refer to a protein composed of the amino acid sequence of SEQ ID NO: 2 in which the 16th to 20th amino acids from the N-terminus of the wild type glucagon protein are substituted from SRRAQ into ERRAK, the 23rd to 24th amino acids are substituted from VQ into IE, and the 27th to 29th amino acids are substituted from MNT into LSA.

As used herein, the term "GLP-1" may refer to a wild type GLP-1 (native GLP-1), which is a protein composed of the amino acid sequence of SEQ ID NO: 4.

As used herein, the term "GLP-1 analogue" means that some amino acids of a wild type GLP-1 protein are substituted. Preferably, it may refer to one selected from the group consisting of a protein composed of the amino acid sequences of SEQ ID NOs: 5 to 14. More preferably, it may refer to a protein composed of the amino acid sequence of SEQ ID NO: 12 in which the 2th amino acid from the N-terminus of the wild type GLP-1 protein is substituted from A into G, the 16th amino acid is substituted from G into E, and the 30th amino acid is substituted from R into GG.

As used herein, the term "FGF21" may refer to a wild type FGF21 (native FGF21), which is a protein composed of the amino acid sequence of SEQ ID NO: 17.

As used herein, the term "FGF21 analogue" means that some amino acids of a wild type FGF21 protein are substituted. Preferably, it may refer to one selected from the group consisting of a protein composed of the amino acid sequences of SEQ ID NOs: 18 to 21. More preferably, it may refer to a protein composed of the amino acid sequence of SEQ ID NO: 20 in which the 19th amino acid from the N-terminus of the wild type FGF21 protein is substituted from R into V, the 98th to 100th amino acids are substituted from LLL into DLK, the 167th to 170th amino acids are substituted from SMVG into RLVE, the 174th amino acid is substituted from G into L, and the 179th to 181th amino acids are substituted from YAS into FE.

As used herein, the term "GIP" may refer to a wild type GIP (native GIP), which is a protein composed of the amino acid sequence of SEQ ID NO: 22.

As used herein, the term "GIP analogue" means that some amino acids of a wild type GIP protein are substituted. Preferably, it may refer to one selected from the group consisting of a protein composed of the amino acid sequences of SEQ ID NOs: 23 to 26. More preferably, it may refer to a protein composed of the amino acid sequence of SEQ ID NO: 24 in which the 2th amino acid from the N-terminus of the wild type GIP protein is substituted from A into S.

As used herein, the term "IL-1RA" may refer to a wild type IL-1RA (native IL-1RA), which is a protein composed of the amino acid sequence of SEQ ID NO: 27.

As used herein, the term "IL-1RA analogue" means that some amino acids of a wild type IL-1RA protein are substituted. Preferably, it may refer to a protein composed of the amino acid sequence of SEQ ID NO: 28.

Hereinafter, the present invention will be described in more detail through the examples, but the following examples are for illustrative purposes only and are not intended to limit the scope of the present invention.

## Acceptor protein

Hereinafter, the acceptor protein prepared in the examples is a polypeptide represented by the following formula:

X-L-Y-L-Ub(A)-L-A-L-Z

in the above formula,
X is a GCG analogue composed of the amino acid sequence of SEQ ID NO: 2, Y is a GLP-1 analogue composed of the amino acid sequence of SEQ ID NO: 12, and Z is an FGF21 analogue composed of the amino acid sequence of SEQ ID NO: 20,
Ub(A) is an acceptor ubiquitin composed of the amino acid sequence of SEQ ID NO: 30,
L is a linker composed of the amino acid sequence of SEQ ID NO: 29, and
A is albumin composed of the amino acid sequence of SEQ ID NO: 32.

SEQ ID NOs of the amino acid sequences of each protein and signal peptide constituting the acceptor protein are shown in Table 1.

[Table 1]

| Protein | Amino acid sequence |
|---|---|
| GCG analogue | SEQ ID NO: 2 |
| GLP-1 analogue | SEQ ID NO: 12 |
| FGF21 analogue | SEQ ID NO: 20 |
| Linker | SEQ ID NO: 29 |
| Ub(A) | SEQ ID NO: 30 |
| Albumin | SEQ ID NO: 32 |
| HSA | SEQ ID NO: 33 |
| IgGκ | SEQ ID NO: 34 |

**Donor protein**

Hereinafter, the donor protein prepared in the examples is a polypeptide represented by the following formula:

W-L-Ub(D)

in the above formula,
W is a GIP analogue composed of the amino acid sequence of SEQ ID NO: 24 or IL-1RA composed of the amino acid sequence of SEQ ID NO: 27,
L is a linker composed of the sequence of SEQ ID NO: 29, and
Ub(D) is a donor ubiquitin composed of the sequence of SEQ ID NO: 31.

SEQ ID NO of the amino acid sequence of each protein constituting the donor protein is shown in Table 2.

[Table 2]

| Protein | Amino acid sequence |
|---|---|
| GIP | SEQ ID NO: 24 |
| IL-1RA | SEQ ID NO: 27 |
| Linker | SEQ ID NO: 29 |
| Ub(D) | SEQ ID NO: 31 |

As used herein, the terms "Donor-191" and "D-191" refer to a donor protein comprising a GIP analogue.
As used herein, the terms "Donor-192" and "D-192" refer to a donor protein comprising IL-1RA.
As used herein, the terms "RD-191" and "C-191" refer to a protein conjugate in which an acceptor protein and a donor protein comprising a GIP analogue are linked.
As used herein, the terms "RD-192" and "C-192" refer to a protein conjugate in which an acceptor protein and a donor protein comprising IL-1RA are linked.

**[Example 1]**

**Preparation of plasmid DNA for acceptor protein expression**

The acceptor gene sequence, which is a gene encoding the acceptor protein, was synthesized, and then cloned into pcDNA3.1(+), an expression vector of a simple structure having a CMV promoter, an ampicillin resistance gene, and the like.

Fast cloning was performed to clone a signal peptide that allows the protein to be secreted out of the cell into a plasmid into which the acceptor gene is inserted. Vector PCR was performed using the plasmid DNA into which the acceptor gene was inserted as a template so that a signal peptide could be inserted into 5' of the acceptor protein, and insertion PCR was performed using human serum albumin (HSA) composed of the sequence of SEQ ID NO: 33 and the IgGκ signal peptide composed of the sequence of SEQ ID NO: 34 as a template so that each signal peptide could be inserted into 5' of the acceptor protein. The PCR reaction was performed using Phusion High-Fidelity DNA polymerase (Thermo Fisher, Cat. No.: F530). In the course of the primary denaturation at 98 °C for 3 minutes, the secondary denaturation at 98 °C for 10 seconds, the primer conjugation at 60 °C for 30 seconds, and the elongation reaction at 72 °C for 3 minutes, the processes from the secondary denaturation to the elongation reaction were repeated 18 times, and then the final elongation reaction was conducted at 72 °C for 5 minutes.

After the PCR reaction was completed, it was confirmed whether the target gene is amplified through agarose gel electrophoresis. Thereafter, 10 μL of each of the vector PCR product and the insertion PCR product was added 1: 1 to a PCR tube, and then 0.5 μL of DpnI was added. The tube was treated at 37 °C for 1 hour to remove the template DNA and ligated. The ligated PCR product was added to DH5α competent cells and transformed by heat shock treatment at 42 °C for 1 minute, and plated on LB solid medium containing ampicillin, and then stationary cultured at 37 °C for at least 16 hours to obtain colonies. A single colony was taken and inoculated into 5 mL of LB medium containing ampicillin, and then cultured at 37 °C and 220 rpm for 16 hours. The culture solution was centrifuged at 3500 rpm for 20 minutes to obtain E. coli wet cells, and then S1, S2, and S3 solutions from the DNA extraction kit (COSMO Genetech, Cat. No.: CMP0112) were added to break the cell wall, and a turbid DNA solution in which proteins and DNA were separated was obtained. The plasmid DNA was purified from the obtained turbid DNA solution using the purification column from the DNA extraction kit (COSMO Genetech, Cat. No.: CMP0112). COSMO Genetech was requested to perform gene sequencing for the plasmid DNA, and two vectors were obtained in which HSA composed of the sequence of SEQ ID NO: 33 or the IgGx signal peptide composed of the sequence of SEQ ID NO: 34 were inserted into 5' of the acceptor protein.

The vector with the identified gene sequence was added to DH5α competent cells and transformed by heat shock treatment at 42 °C for 1 minute, and plated on LB solid medium containing ampicillin, and then stationary cultured at 37 °C for at least 16 hours to obtain colonies. A single colony was taken and inoculated into 5 mL of LB medium, and then cultured at 37 °C and 220 rpm for 16 hours. A part of this culture solution was again inoculated into 200 mL of LB medium containing ampicillin, and then cultured at 37 °C and 220 rpm for 16 hours. The culture solution was centrifuged at 3500 rpm for 30 minutes to obtain E. coli wet cells, and then P1, P2, and P3 solutions from the DNA extraction kit (QIAGEN, Cat. No. : 12263) were added to break the cell wall, and a turbid DNA solution in which proteins and DNA were separated was obtained. The plasmid DNA pellet was obtained from the obtained turbid DNA solution using the purification column from the DNA extraction kit (QIAGEN, Cat. No.: 12263). The pellet was dissolved in cell culture water (Sigma Aldrich, W3500) and then filtered through a 0.22 μm filter. The extracted plasmid DNA was used for protein expression after measuring the DNA concentration and purity using a nanodrop device (IMPLEN, Nanodrop NP-80).

**[Example 2]**

**Expression of acceptor protein in ExpiCHO-S cells**

24 hours before the transfection process, ExpiCHO-S (Gibco, Cat. No. : A29127, Lot : 1974423) cells were inoculated at $4\times10^6$ cells/mL and prepared, and mounted on an orbital shaker in an incubator at 37 °C, 80 % humidity or more, 8 % $CO_2$ and cultured at 95 rpm (50 mm shaking diameter) conditions. After 24 hours, the cells were filtered through a 40 μm nylon filter (BD Falcon, Cat. No.: 352340) to remove clumps, and then the cell viability and the number of cells were measured. The filtered cells were diluted with ExpiCHO-S expression medium (ExpiCHO Expression Media, Gibco, Cat. No. : A29100-01) to a final concentration of $6\times10^6$ cells/mL, and the 200 mL of the final cells was added in a 1 L flask.

120 μg of DNA was diluted in 8 mL of OptiPRO SFM (Gibco, Cat. No.: 12309-019) medium and 640 μL of ExpiFectamine CHO Reagent (Gibco, Cat. No. : 100033022) was diluted in 7.4 mL of OptiPRO SFM (Gibco, Cat. No.: 12309-019) medium, respectively, and then mixed. The mixed solution was reacted at room temperature for 3 minutes, and then dispensed into the inoculated flask to $6\times10^6$ cells/mL and transfected. It was mounted on an orbital shaker in an incubator at 37 °C, 80 % humidity or more, 8 % $CO_2$ and cultured at 95 rpm (50 mm shaking diameter) conditions for 18 hours. After 18 hours, 1200 μL of ExpiFectamine CHO Enhancer (Gibco, Cat. No. : 100033019) and 48 mL of ExpiCHO Feed (Gibco, Cat. No. : A29101-01) were added, and mounted on an orbital shaker in an incubator at 37 °C, 80 % humidity or more, 8 % $CO_2$, and cultured at 95 rpm conditions for 7-8 days. After the culture was completed, centrifugation was performed at 3500 rpm conditions for at least 30 minutes to obtain only the acceptor protein expression culture solution except for the cell pellet. The obtained culture solution was filtered through two filters (Satorius stedim, Cat. No.: DH-ST-29MDL20MC5FFV) (Satorius stedim, Cat. No.: DH-ST-5441307G4OOB) to remove impurities.

80 μL of the prepared culture solution was taken, and 20 μL of 5 X reducing sample loading dye was added

and was mixed, and the mixture was allowed to be stood at 95 °C for 5 minutes. In order to compare the expression levels on the gel, 80 μL of bovine serum albumin, which was diluted to 62.5, 125, and 250 mg/mL, and 20 μL of 5 X reducing sample loading dye were added and mixed, and the mixture was allowed to be stood at 95 °C for 5 minutes. The prepared sample and the marker protein for size checking were loaded on a 10 % Tris-Glycine gel. The gel was stained with Coomassie brilliant blue R while gently shaking, and the concentration of the acceptor protein was relatively quantified based on the bovine serum albumin protein band.

**[Example 3]**

**Purification of acceptor protein**

The culture solution of the acceptor protein expressed in Example 2 was loaded on Blue sepharose HP resin (GE, Cat. No.: 17-0413-01) column equilibrated with an equilibrium buffer (20 mM sodium phosphate, pH 7.0). The impurities was removed through pre-elution (20 mM sodium phosphate, pH 7.0, 0.15 M KCl), and the acceptor protein was recovered using an elution buffer (20 mM sodium phosphate, pH 7.0, 0.6 M KCl). The recovered acceptor protein was subjected to dialysis with 20 mM sodium phosphate, pH 7.0 buffer to remove salts, and ultrafiltration was performed so that the final sample was 5 mg/mL. The results of acceptor protein purification through SDS-PAGE are shown in Figure 1.

**[Example 4]**

**Expression of donor protein in E. coli cells**

The donor gene sequence encoding the donor protein was transformed into pET21a vector with His-SUMO tag, and then the plasmid into which the donor gene was inserted was added to BL21(DE3) competent cells and transformed by heat shock treatment at 42 °C for 1 minute, and plated on LB solid medium containing ampicillin, and then stationary cultured at 37 °C for at least 16 hours to obtain colonies. A single colony was taken and inoculated into 50 mL of LB medium, and then seed culture was performed at 37 °C and 220 rpm for 16 hours.

In the case of the donor protein (Donor-191, D-191) containing GIP as a biomolecule, the main culture was performed by inoculating the seed culture solution into 1 L of LB medium containing ampicillin at a ratio of 1:100. The culture was performed at 37 °C and 220 rpm for 2 hours. Thereafter, when the OD600nm reached 0.6, 0.25 M IPTG was added and cultured at 16 °C and 220 rpm for 20 hours. After the culture was completed, centrifugation was performed at 3500 rpm for 30 minutes to obtain E. coli wet cells.

In the case of the donor protein (Donor-192, D-192) containing IL-1RA as a biomolecule, the main culture was performed by inoculating the seed culture solution into 1 L of LB medium containing ampicillin at a ratio of 1:100. Autoinduction was performed at 37 °C and 220 rpm for 24 hours. After the culture was completed, centrifugation was performed at 3500 rpm for 30 minutes to obtain E. coli wet cells.

**[Example 5]**

**Purification of donor protein in E. coli cells**

The cultured donor protein was purified by the following method, and the purification process is shown in Figure 2.

**Lysis / sonication**

The wet cells obtained by culture were resuspended using a lysis buffer (20 mM sodium phosphate, pH 7.0, 0.5 M NaCl 0.02 M imidazole, 0.1 mM PMSF). Lysis samples were placed on ice, and sonication was performed under conditions of Pules on/off= 5 sec/3 sec and 45% amplitude for 15 minutes. Lysate was obtained by centrifugation at 14,000 rpm for 30 minutes to recover only the supernatant.

**Capture purification**

The lysate was loaded on Ni-sepharose resin (QIAGEN, Cat. No.: 30250). After the sample loading was completed, the non-specific protein was sufficiently washed and removed using a binding buffer (20 mM sodium phosphate, pH 7.0, 0.5 M NaCl, 0.02 M imidazole). Thereafter, the Hig-SUMO tagged donor protein was recovered using an elution buffer (20 mM sodium phosphate, pH 7.0, 0.5 M NaCl, 0.25 M imidazole). The recovered His-SUMO tagged donor protein was subjected to dialysis with 20 mM sodium phosphate, pH 7.0 buffer to remove salts and imidazole. The results

obtained by confirming the purification of the donor protein through a Ni column by SDS-PAGE are shown in Figures 3 and 4.

**SENP1 enzyme digestion**

The His-SUMO tagged donor protein and SENP1 were subjected to SENP1 enzyme digestion at a ratio of 100 mg : 1 mg. The concentration of the protein recovered by Ni-purification was quantified, and SNEP1 (in-house) corresponding to 1/100 w/w of the amount (mg) of the His-SUMO tagged donor protein was mixed. The reaction mixture was allowed to be stood at room temperature (15 to 25 °C) for 1 hour.

**His-SUMO removal**

The reaction mixture was loaded on Ni-sepharose resin (QIAGEN, Cat. No.: 30250) equilibrated with an equilibrium buffer (20 mM sodium phosphate, pH 7.0, 0.5 M NaCl, 0.02 M imidazole). Sample loading was performed, and the donor protein in which the His-SUMO tag was cleaved was allowed to flow through. After the sample loading was completed, the remaining donor protein was recovered using an equilibrium buffer (20 mM sodium phosphate, pH 7.0, 0.5 M NaCl, 0.02 M imidazole), and the recovered donor protein was subjected to dialysis with 20 mM sodium phosphate, pH 7.0 buffer to remove salts and imidazole. Ultrafiltration was performed so that the final donor product was 10 mg/mL. The results obtained by confirming the His-SUMO removal purification by SDS-PAGE are shown in Figures 5 and 6.

**Purification**

The donor recovered in the previous step was loaded on Capto Q ImpRes (GE, Cat. No.: 17-5470-15) column equilibrated with an equilibrium buffer (20 mM sodium phosphate, pH 7.0 buffer). The donor protein was allowed to flow through. The recovered proteins were concentrated to a high concentration. The results of polishing purification of the donor protein are shown in Figures 7 and 8.

**[Example 6]**

**Conjugation**

As shown in Figure 9, conjugation was performed using the acceptor protein produced in Example 3 and the donor protein produced in Example 5. A mixture of the conditions shown in Table 3 below was prepared, and the conjugation reaction was performed at 30 °C for 4 hours.

[Table 3]

| | Acceptor | Donor | ATP | mUBAI | UBC13 /MMS2 | Buffer | Reducing agent | Vol. |
|---|---|---|---|---|---|---|---|---|
| Condition | 10 μM | 15 μM | 4 mM | 1 μM | 10 μM | 50mM Tris pH7.6, 2.5mM MgCl2 | 0.05 mM TCEP | 100 mL |

10 μg of the acceptor was loaded on 8 % SDS-PAGE, and the degree of conjugation for the reaction was confirmed. The results are shown in Figures 10 and 11.

**[Example 7]**

**Enzyme removal with Ni-sepharose**

In order to recover only the conjugate sample, the reaction mixture was loaded on Ni-sepharose resin (QIAGEN, Cat. No.: 30250) equilibrated with an equilibrium buffer (20 mM sodium phosphate, pH 8.0, 0.15 M NaCl). After the sample loading was completed, impurities were removed using an equilibrium buffer (20 mM sodium phosphate, pH 8.0, 0.5 M NaCl). Thereafter, the conjugate was recovered using an elution buffer (25 mM Tris, pH 8.0, 0.15 M NaCl, 0.01 M imidazole). The recovered protein conjugate (C-191 and C-192) was subjected to dialysis with 20 mM sodium phosphate, pH 7.0 buffer to remove salts and imidazole. The results obtained by confirming the Ni purification by chromatography are shown in Figure 12.

**[Example 8]**

**Purification**

The conjugate recovered in the previous step was loaded on Capto Q ImpRes (GE, Cat. No.: 17-5470-15) column equilibrated with an equilibrium buffer (25 mM sodium phosphate, pH 7.0 buffer). The conjugate was recovered using an elution buffer (25 mM sodium phosphate, pH 7.0, 158 mM NaCl buffer). The recovered protein conjugate was subjected to dialysis with 20 mM sodium phosphate, pH 7.0 buffer to remove salts and imidazole. Ultrafiltration was performed so that the final conjugate product was 5 mg/mL. The results are shown in Figures 13 and 14.

**[Example 9]**

**Formulation**

The protein conjugate recovered from AEX was subjected to dialysis with a formulation buffer (4.6 mM histidine, 5.7 mM Tris, pH 7.5, 10 mM arginine, 0.1 g/mL trehalose) to remove salts and imidazole. Ultrafiltration was performed so that the final conjugate product was 5 mg/mL. The results are shown in Figure 15.

**[Test Example 1]**

**cAMP accumulation assay**

In order to test the protein conjugates C-191 and C-192, which are GCG/GLP-1/FGF21/GIP or GCG/GLP-1/FGF21/IL-1RA acceptor quadruple (tetra) agonists/antagonists prepared in Examples 1 to 9, for the activity level of the GLP-1, GCG, and GIP agonists at the cellular level (in vitro), the cAMP accumulation assay was performed in the cell line in which the GLP-1 acceptor, the GIP acceptor, and the GCG acceptor were overexpressed transiently or stably, respectively, using Cisbio cAMP Gs Dynamic kit #62AM4PEC, as follows.

**Cell preparation (Transient)**

The HEK293 cells were incubated for 2 to 3 days in an incubator at 37°C and 5% $CO_2$ so that the concentration of HEK293 cells in a T-75 flask was 70 to 80%. The medium was removed and treated with 2 mL of TryPLE Express, and then incubated in an incubator at 37°C and 5% $CO_2$ for 3 to 5 minutes, and the cells were detached. It was diluted by adding 6 mL of the culture medium (MEM, 10 % FBS, 1 % Anti-anti), and transferred to a 15 mL tube, and then centrifuged at 1000 rpm for 3 minutes. The supernatant was discarded and released in 5 mL of the medium. The number of cells was counted, and the concentration was allowed to be $3 \times 10^5$ cells/mL. 2 mL was dispensed into a 6-well plate and incubated in an incubator at 37°C and 5% $CO_2$ for 24 hours. The cultured medium was removed, and 1.7 mL of the medium without an antibiotic medium was dispensed. FuGENE6 and each acceptor plasmid were added to Opti-MEM in an appropriate amount and cultured at room temperature for 5 minutes, respectively. Each was mixed and cultured at room temperature for 15 minutes. The mixed solution was dispensed into the corresponding well and incubated in an incubator at 37°C and 5% $CO_2$ for 24 hours. The medium was removed, and the cells were washed with 2 mL of the pre-warmed PBS. 0.5 mL of Accutase per well was dispensed and incubated in an incubator at 37°C and 5% $CO_2$ for 5 minutes, and the cells were detached. At this time, it was checked under a microscope whether the cells were completely detached, and the plate was struck to prevent the cells from detaching. 2.5 mL of the 37°C pre-warmed assay buffer (0.5% BSA, 2 mM IBMX in PBS) per well was added, transferred to a 15 mL tube, and centrifuged at 1,000 rpm for 3 minutes, and then again released in 2 mL of the assay buffer. The number of cells was counted, and the concentration was allowed to be 400,000 cells/mL in the assay buffer.

**Cell preparation (Stable)**

The cells in which the GLP-1 acceptor (Genscript, Cat. No. M00451), the GIP acceptor (Genscript, Cat. No. M00486), and the GCG acceptor (Genscript, Cat. No. M00345) were overexpressed were cultured so that the concentration of the cells in a T-75 flask was 70 to 80%. The medium was removed, and the cells were washed with 2 mL of the 37°C pre-warmed PBS. 1 mL of Accutase was dispensed and incubated in an incubator at 37°C and 5% $CO_2$ for 5 minutes, and the cells were detached. At this time, it was checked under a microscope whether the cells were completely detached, and the plate was struck to prevent the cells from detaching. 3 mL of the 37°C pre-warmed assay buffer per well was added, transferred to a 15 mL tube, and centrifuged at 1,000 rpm for 3 minutes, and then again released in 2 mL of the assay buffer. The number of cells was counted, and the concentration was allowed to be 400,000 cells/mL in

the assay buffer.

**Procedure**

5 μL of the prepared cells was dispensed into a 96-well low volume white plate. 5 μL of each of the reference and sample (2X) prepared by 4-fold serial dilution was dispensed in duplicate and incubated in an incubator at 5% $CO_2$ for 30 minutes. At this time, 5 μL of the assay buffer was added to the control and blank wells. 5 μL of 1 X cAMP-d2 solution was dispensed. At this time, 5 μL of Lysis & Detection buffer was added to the blank well. 5 μL of 1X Anti-cAMP-Cryptate solution was dispensed and cultured for 1 hour at room temperature in a state where light was blocked, and then the fluorescence was measured with a plate reader.

**Measurement and analysis**

The fluorescence of the sample in the plate was measured with a Synergy Neo2 instrument (Excitation wavelength : 330 nm, Emission wavelength : 665 nm and 620 nm).

The HTRF ratio was calculated as follows.

$$HTRF\ Ratio = \frac{Signal\ 665\ nm}{Signal\ 620\ nm} \times 10^4$$

In addition, the Delta ratio (△R ) was calculated as follows.

$$\triangle R = \text{Ratio}_{\text{sample}} - \text{Ratio}_{\text{blank}} = \text{Signal - background fluorescence}$$

The EC50 values were obtained as HTRF ratio plot values using GraphPad Prism 8 (curve-fitting of the log (agonist) vs. normalized response - variable slope equation).

HTRF ratio plots for GLP-1R (GLP-1 acceptor), GIPR (GIP acceptor) and GCGR (GCG acceptor) are shown in Figures 16 to 18, and the EC50 values were calculated and are shown in Table 4 below.

[Table 4]

| EC50 (pM) | GLP-1R | | | | GIPR | | | | GCGR | | | |
|---|---|---|---|---|---|---|---|---|---|---|---|---|
| | 1 | 2 | Mean | SD | 1 | 2 | Mean | SD | 1 | 2 | Mean | SD |
| Liraglutide | 40.7 | 36.7 | 38.7 | 2.83 | - | - | - | - | - | - | - | - |
| Dulaglutide | 1.23 | 0.9 | 1.07 | 0.23 | - | - | - | - | - | - | - | - |
| GIP(1-39) | - | - | - | - | 15.4 | 14.1 | 14.8 | 0.92 | - | - | - | - |
| GCG | - | - | - | - | - | - | - | - | 39.3 | 30.4 | 34.9 | 6.29 |
| C-191 | 25.4 | 36.5 | 31.0 | 7.85 | 110 | 85 | 97.5 | 17.7 | 4487 | 3832 | 4160 | 463 |
| C-192 | 13.2 | 15.9 | 14.6 | 1.91 | - | - | - | - | 286 | 251 | 269 | 24.7 |

As shown in Table 4, it was confirmed that C-191 had activity on each of the GLP-1 acceptor, the GIP acceptor, and the GCG acceptor. In addition, it was confirmed that C-192 also had activity on each of the GLP-1 acceptor and the GCG acceptor.

In particular, it was confirmed that in terms of activity on the GLP-1 acceptor, C-191 and C-192 exhibited more excellent activity than liraglutide used as a control group. In addition, it was confirmed that C-192 had more excellent activity by about 2 times than C-191 in terms of activity on the GLP-1 acceptor, and had more excellent activity by about 15 times than C-191 in terms of activity on the GCG acceptor.

**[Test Example 2]**

**FGFR1/KLB functional assay**

In order to test the protein conjugates C-191 and C-192, which are GCG/GLP-1/FGF21/GIP or GCG/GLP-1/FGF21/IL-1RA acceptor quadruple (tetra) agonists/antagonists prepared in Examples 1 to 9, for the activity level of the FGF21 agonist at the cellular level (in vitro), the FGFR1/KLB functional assay was performed in the cell line (Discover X, Cat. No. 93-118C3) in which FGFR1/KLB was overexpressed using PathHunter Detection Kit (Discover X, Cat. No. 93-0001), as follows.

**Cell seeding**

The cells were cultured to 70-80 % full in a T-75 flask. The medium was removed and washed with 5 mL of the pre-warmed PBS. PBS was removed, and 2 mL of AssayComplete cell detachment reagent was added, and then incubated in an incubator at 37°C and 5% $CO_2$ for 3 minutes, and the attached cells were detached. It was mixed with 6 mL of AssayComplete cell plating 0 reagent, and the mixture was transferred to a 15 mL tube, centrifuged at 1,000 rpm for 3 minutes, and then again released in 5 mL of cell plating reagent. The number of cells was counted, and the concentration was allowed to be 500,000 cells/mL. It was transferred to a reservoir, and 40 μL per well was dispensed into a white 96-well half-area cell culture plate using a multi-channel pipette (20,000 cells/well). Only 40 μL of cell plating reagent was dispensed into the blank well. The cells were incubated in an incubator at 37°C and 5% $CO_2$ for 24 hours to allow to be attached.

**Procedure**

10 μL of each of rhFGF21 (5X) and test sample (5X) prepared by 4-fold serial dilution was dispensed in duplicate and cultured at room temperature for 4 hours. 10 μL of AssayComplete cell plating 0 reagent was added to the control and blank wells. After the culture was completed, the medium was removed, and 50 μL of AssayComplete cell plating 0 reagent was added. 30 μL of Detection reagent was dispensed and reacted in a state where light was blocked at room temperature for 1 hour, and then the luminescence was measured with a plate reader.

**Measurement and analysis**

The luminescence of the sample in the plate was measured with a Synergy Neo2 instrument.

The Delta RLU (relative luminescence unit) (△RLU) was calculated as follows.

$$\triangle RLU = RLU_{sample} - RLU_{blank} = \text{Signal - background luminescence}$$

In addition, the fold induction relative to the control group was calculated as follows.

$$Fold\ induction = \frac{\Delta RLU_{sample}}{\Delta RLU_{control}}$$

The EC50 values were obtained based on fold induction values by each concentration using GraphPad Prism 8, as follows (curve-fitting of the log (agonist) vs. normalized response - variable slope equation).

The plots are shown in Figure 19, and the EC50 values were calculated and are shown in Table 5 below.

[Table 5]

| EC50 (nM) | rhFGF21 | C-191 | C-192 |
|---|---|---|---|
| 1 | 0.75 | 92.2 | 42.2 |
| 2 | 0.88 | 45.1 | 40.5 |
| Mean | 0.82 | 68.7 | 41.4 |
| SD | 0.09 | 33.3 | 1.20 |

As shown in Table 5 above, it was confirmed that both C-191 and C-192 had activity on the FGFR1/KLB acceptor. In addition, it was confirmed that in terms of activity on the FGFR1/KLB acceptor, C-192 had more excellent activity by about 40% than C-191.

**[Test Example 3]**

**NF-κB reporter gene luciferase assay (reporter gene assay)**

In order to test the protein conjugate C-192, which is a GCG/GLP-1/FGF21/IL-1RA acceptor quadruple (tetra) agonist/antagonist prepared in Examples 1 to 9, for the ability of IL-1RA to inhibit NF- κB activity by IL-1β at the cellular level (in vitro), the luciferase assay was performed in the NF-κB reporter (Luc) cell line (BPS Bioscience, Cat. No. 60650), as follows.

**Cell seeding**

The cells were cultured to 70-80 % full in a T-75 flask. The medium was removed and washed once with 5 mL of the pre-warmed PBS. PBS was removed, and 1 mL of TryPLE Express was added, and incubated for 5 minutes in an incubator at 37°C and 5% $CO_2$, and the cells were detached. It was mixed with 4 mL of assay medium (10 % FBS, 1% antibiotic in DMEM), and the mixture was transferred to a 15 mL tube, centrifuged at 1,000 rpm for 3 minutes, and then again released in 5 mL of assay medium. The number of cells was counted, and the concentration was allowed to be 500,000 cells/mL. It was transferred to a reservoir, and 40 μL per well was dispensed into a white 96-well half-area cell culture plate using a multi-channel pipette (20,000 cells/well). Only 40 μL of assay medium was dispensed into the blank well. The cells were incubated in an incubator at 37°C and 5% $CO_2$ for 24 hours to allow to be attached.

**Procedure**

5 μL of each of the reference (10X) and sample (10X) prepared by 4-fold serial dilution was dispensed in duplicate. Thereafter, 5 μL of 50 pM IL-1β (10X) was dispensed and incubated in an incubator at 5% $CO_2$ for 4 hours. 50 μL of ONE-Glo reagent (Promega, Cat. No. E6120) was each dispensed and cultured at room temperature for 5 minutes, and then the luminescence was measured with a Synergy Neo2 instrument. At this time, before treatment with ONE-Glo reagent, the plate was removed from the incubator for 15 minutes and allowed to reach room temperature.

The Delta RLU (relative luminescence unit) (△RLU) was calculated as follows.

$$\triangle RLU = RLU_{sample} - RLU_{blank} = \text{Signal - background luminescence}$$

The IC50 values were obtained as △RLU by each concentration using GraphPad Prism 8, as follows (curve-fitting of the log (antagonist) vs. normalized response - variable slope equation).

The plots are shown in Figure 20, and the IC50 values were calculated and are shown in Table 6 below.

[Table 6]

| IC50 (pM) | rhIL-1RA | C-192 |
|---|---|---|
| 1 | 39.9 | 561 |
| 2 | 51.5 | 594 |
| 3 | 46.6 | 708 |
| 4 | 51.9 | 568 |
| Mean | 47.5 | 607 |
| SD | 4.85 | 68.4 |

As shown in Table 6 above, it was confirmed that C-192 inhibited the activity of NF-κB by IL-1β.

**[Test Example 4]**

***In-vivo* efficacy test : Efficacy evaluation against non-alcoholic steatohepatitis**

The *in vivo* efficacy of the protein conjugate C-192, which is a GCG/GLP-1/FGF21/IL-1RA acceptor quadruple (tetra) agonist/antagonist prepared in Examples 1 to 9, against non-alcoholic steatohepatitis was verified in C57BL/6J mice in which fatty liver was induced by feeding MCD (Methionine and Choline Deficient L-Amino Acid Diet). The MCD

mouse model is the most typically used method for efficacy testing against non-alcoholic steatohepatitis, and it is known that the histological appearance of the induced liver tissue from week 2 to week 4 is histologically similar to that of human non-alcoholic steatohepatitis. There are many precedent cases in which a number of pharmaceutical companies and academia conducted tests with various substances.

Fatty liver was induced by feeding MCD to male C57BL/6J mice for 8 weeks, and then the mice were divided into 6 groups shown in Table 7 below, and drug administration was performed for 4 weeks. As a comparative substance, two currently commercially available drugs, liraglutide (Saxenda) and dulaglutide (Trulycity), were used. The administered composition was repeatedly administered subcutaneously using a disposable syringe according to each administration cycle. During the 8-week diet period, body weight was measured once a week, and group separation was performed significantly by the body weight after 8 weeks. The normal control group was fed with MCS (Methionine and Choline Sufficient L-Amino Acid Diet).

[Table 7]

| No | Group | Diet | Administered composition | Administered dose | Administration cycle | Number of administrati on |
|---|---|---|---|---|---|---|
| 1 | Normal control group | MCS | - | - | - | - |
| 2 | Negative control group | MCD | Excipient | - | - | - |
| 3 | Experimental group A | MCD | C-192 | 5 nmol/kg | 1 time/2 days | 14 times |
| 4 | Experimental group B | MCD | C-192 | 40 nmol/kg | 1 time/2 days | 14 times |
| 5 | Comparative group A | MCD | Liraglutide | 50 nmol/kg | 2 times/1 day | 56 times |
| 6 | Comparative group B | MCD | Dulaglutide | 2 nmol/kg | 1 time/2 days | 14 times |

During the 4-week administration period, general symptoms and behavior were observed, and body weight was measured once a week after the start of administration and on the day of tissue extraction. Blood collection and liver tissue extraction were performed on the day of the end of observation, and blood was subjected to a hematological-biochemical test, and the extracted liver tissue was subjected to a histopathological test.

Alanine aminotransferase (ALT), which is used as the most basic indicator of liver disease, was measured through a hematological-biochemical test, and the results are shown in Table 8 below and Figure 21.

[Table 8]

| No | Group | ALT |
|---|---|---|
| 1 | Normal control group | 18.3 |
| 2 | Negative control group | 230.6 |
| 3 | Experimental group A | 178.3 |
| 4 | Experimental group B | 126.3 |
| 5 | Comparative group A (liraglutide administration group) | 223.0 |
| 6 | Comparative group B (dulaglutide administration group) | 220.5 |

As shown in Table 8 above, it was found that the ALT value was hardly reduced in the group administered with liraglutide and the group administered with dulaglutide (Comparative groups A and B), but the ALT value was significantly reduced in the group administered with C-192.

Since ALT indicates the degree of liver damage, it can be seen that when the protein conjugate of the present invention is administered, the degree of liver damage is reduced.

In addition, the ALT/AST values are shown in Table 9 below and Figure 22.

[Table 9]

| No | Group | ALT/AST value |
|---|---|---|
| 1 | Normal control group | 1.77 |
| 2 | Negative control group | 0.98 |
| 3 | Experimental group A | 1.35 |
| 4 | Experimental group B | 1.53 |
| 5 | Comparative group A (liraglutide administration group) | 0.99 |
| 6 | Comparative group B (dulaglutide administration group) | 0.79 |

As shown in Table 9 above, it was confirmed that the ALT/AST value was less than 1.0 in the group administered with liraglutide and the group administered with dulaglutide (Comparative groups A and B), but it was found that the ALT/AST value was greater than 1.0 in the group administered with C-192.

Since the ALT/AST value is often less than 1 in liver disease, it can be seen that when the protein conjugate of the present invention is administered, the degree of liver damage is reduced.

In addition, histopathological test was performed by observation through H&E and Masson's trichrome staining, and the results are shown in Figure 23. In the case of non-alcoholic steatohepatitis, steatosis and inflammation in lobules occurs, and inflammatory cells can be identified by tissue staining. As shown in Figure 23, it can be seen that the group administered with the protein conjugate at 40 nmol/kg (Experimental group B) showed improvements in fat and inflammatory cells to that of the normal control group, compared to the negative control group.

In addition, based on the evaluation criteria shown in Table 10 below, steatosis score, lobular inflammation score, and NAS (NAFLD activity score) were evaluated.

[Table 10]

| Item | Definition | Score |
|---|---|---|
| Steatosis | Low to medium power evaluation of parenchymal involvement by steatosis | |
| | <5% | 0 |
| | 5~33% | 1 |
| | >33~66% | 2 |
| | >66% | 3 |
| Lobular inflammation | Overall assessment of all inflammatory foci | |
| | No foci | 0 |
| | <2 foci per 200Yfield | 1 |
| | 2~4 foci per 200Yfield | 2 |
| | >4 foci per 200Yfield | 3 |
| Ballooning | None | 0 |
| | Few balloon cells | 1 |
| | Many cells/prominet ballooning | 2 |

The evaluation results are shown in Tables 11 to 13 below and Figures 24 to 26.

[Table 11]

| No | Group | Steatosis score (Steatosis) |
|---|---|---|
| 1 | Normal control group | 0 |
| 2 | Negative control group | 2.1 |
| 3 | Experimental group A | 1.7 |

(continued)

| No | Group | Steatosis score (Steatosis) |
|---|---|---|
| 4 | Experimental group B | 0.9 |
| 5 | Comparative group A (liraglutide administration group) | 1.6 |
| 6 | Comparative group B (dulaglutide administration group) | 1.9 |

[Table 12]

| No | Group | Lobular inflammation score (Lobular inflammation) |
|---|---|---|
| 1 | Normal control group | 0.3 |
| 2 | Negative control group | 1.3 |
| 3 | Experimental group A | 1.2 |
| 4 | Experimental group B | 0.2 |
| 5 | Comparative group A (liraglutide administration group) | 0.5 |
| 6 | Comparative group B (dulaglutide administration group) | 1.5 |

[Table 13]

| No | Group | NAS (NAFLD Activity Score) |
|---|---|---|
| 1 | Normal control group | 0.3 |
| 2 | Negative control group | 3.4 |
| 3 | Experimental group A | 2.9 |
| 4 | Experimental group B | 1.2 |
| 5 | Comparative group A (liraglutide administration group) | 2.2 |
| 6 | Comparative group B (dulaglutide administration group) | 3.4 |

As shown in Tables 11 to 13 above, all groups administered with the protein conjugate of the present invention exhibited excellent results, and in particular, the group administered with the protein conjugate at 40 nmol/kg (Experimental group B) showed significantly more excellent evaluation results than the group administered with liraglutide and the group administered with dulaglutide.

The liver tissue was analyzed by ELISA for TGF-β, a marker of liver fibrosis, and the results are shown in Table 14 below and Figure 27.

[Table 14]

| No | Group | Hepatic TGF-β |
|---|---|---|
| 1 | Normal control group | 74.40 |
| 2 | Negative control group | 164.10 |
| 3 | Experimental group A | 135.50 |
| 4 | Experimental group B | 98.70 |
| 5 | Comparative group A (liraglutide administration group) | 136.40 |
| 6 | Comparative group B (dulaglutide administration group) | 159.60 |

As shown in Table 14 above, the group administered with the protein conjugate of the present invention exhibited the reduced TGF-β value, and in particular, the group administered with the protein conjugate at 40 nmol/kg (Experimental

group B) exhibited the value almost similar to that of the normal control group.

In addition, triglyceride accumulation in the liver tissue was analyzed using the Triglyceride Assay Kit, and the results are shown in Table 15 below and Figure 28. The amount of triglyceride in the liver tissue was hardly reduced in the group administered with liraglutide and the group administered with dulaglutide, but the group administered with the protein conjugate of the present invention exhibited the value almost similar to that of the normal control group.

[Table 15]

| No | Group | Hepatic Triglyceride |
|---|---|---|
| 1 | Normal control group | 17.76 |
| 2 | Negative control group | 40.31 |
| 3 | Experimental group A | 25.69 |
| 4 | Experimental group B | 24.43 |
| 5 | Comparative group A (liraglutide administration group) | 40.69 |
| 6 | Comparative group B (dulaglutide administration group) | 40.78 |

As a result, it was found that the group administered with the protein conjugate C-192 of the present invention exhibited a significantly more excellent effect in all experiments compared to the group administered with liraglutide and the group administered with dulaglutide.

**[Test Example 5]**

***In-vivo* efficacy test : Efficacy evaluation against non-alcoholic steatohepatitis**

The *in vivo* efficacy of the protein conjugate C-191, which is a GCG/GLP-1/FGF21/GIP acceptor quadruple (tetra) agonist/antagonist prepared in Examples 1 to 9, against non-alcoholic steatohepatitis was verified in C57BL/6J mice in which fatty liver was induced by feeding MCD (Methionine and Choline Sufficient L-Amino Acid Diet).

The mice were divided into 4 groups shown in Table 16 below, and the experiment was conducted in the same manner as in Test Example 4 above. As a comparative substance, the currently commercially available dulaglutide (Trulycity) was used.

[Table 16]

| No | Group | Diet | Administered composition | Administered dose | Administration cycle | Number of administration |
|---|---|---|---|---|---|---|
| 1 | Normal control group | MCS | | - | - | - |
| 2 | Negative control group | MCD | Excipient | - | - | - |
| 3 | Experimental group A | MCD | C-191 | 10 nmol/kg | 1 time/2 days | 14 times |
| 4 | Comparative group B | MCD | Dulaglutide | 2 nmol/kg | 1 time/2 days | 14 times |

During the 4-week administration period, general symptoms and behavior were observed, and body weight was measured once a week after the start of administration and on the day of tissue extraction. Blood collection and liver tissue extraction were performed on the day of the end of observation, and blood was subjected to a hematological-biochemical test, and the extracted liver tissue was subjected to a histopathological test.

Alanine aminotransferase (ALT), which is used as the most basic indicator of liver disease, was measured through a hematological-biochemical test, and the results are shown in Table 17 below and Figure 29.

[Table 17]

| No | Group | ALT |
|---|---|---|
| 1 | Normal control group | 19.1 |
| 2 | Negative control group | 312.4 |
| 3 | Experimental group A | 151.9 |
| 4 | Comparative group A (dulaglutide administration group) | 246.8 |

As shown in Table 17 above, it was confirmed that the ALT value was hardly reduced in the group administered with dulaglutide, but the ALT value was significantly reduced in the group administered with C-191.

Since ALT indicates the degree of liver damage, it can be seen that when the protein conjugate of the present invention is administered, the degree of liver damage is reduced.

In addition, the AST/ALT values are shown in Table 18 below and Figure 30.

[Table 18]

| No | Group | AST/ALT value |
|---|---|---|
| 1 | Normal control group | 1.84 |
| 2 | Negative control group | 0.82 |
| 3 | Experimental group A | 0.98 |
| 4 | Comparative group A (dulaglutide administration group) | 0.76 |

As shown in Table 18 above, it was confirmed that the ALT/AST value was 0.76 in the group administered with dulaglutide, but it was confirmed that the ALT/AST value was 0.98 in the group administered with C-191, which is higher than that of the group administered with dulaglutide.

Since the ALT/AST value is often less than 1 in liver disease, it can be seen that when the protein conjugate of the present invention is administered, the degree of liver damage is reduced.

In addition, histopathological test was performed by observation through H&E and Masson's trichrome staining, and the results are shown in Figure 31. In the case of non-alcoholic steatohepatitis, steatosis and inflammation in lobules occurs, and inflammatory cells can be identified by tissue staining. As shown in Figure 31, it can be seen that the group administered with the protein conjugate at 10 nmol/kg showed improvements in fat and inflammatory cells to that of the normal control group, compared to the negative control group.

In addition, based on the evaluation criteria shown in Table 10 above, steatosis score, lobular inflammation score, and NAS (NAFLD activity score) were evaluated.

The evaluation results are shown in Tables 19 to 21 below and Figures 32 to 34.

[Table 19]

| No | Group | Steatosis score (Steatosis) |
|---|---|---|
| 1 | Normal control group | 0 |
| 2 | Negative control group | 2.4 |
| 3 | Experimental group A | 1.4 |
| 4 | Comparative group A (dulaglutide administration group) | 1.8 |

[Table 20]

| No | Group | Lobular inflammation score (Lobular inflammation) |
|---|---|---|
| 1 | Normal control group | 0.2 |
| 2 | Negative control group | 1.6 |
| 3 | Experimental group A | 1.0 |

(continued)

| No | Group | Lobular inflammation score (Lobular inflammation) |
|---|---|---|
| 4 | Comparative group A (dulaglutide administration group) | 1.6 |

[Table 21]

| No | Group | NAS (NAFLD Activity Score) |
|---|---|---|
| 1 | normal control group | 0.2 |
| 2 | negative control group | 4.0 |
| 3 | Experimental group A | 2.4 |
| 6 | Comparative group A (dulaglutide administration group) | 3.4 |

As shown in Tables 19 to 21 above, all groups administered with the protein conjugate of the present invention exhibited excellent results, and showed significantly more excellent evaluation results than the group administered with dulaglutide.

The liver tissue was analyzed by ELISA for TGF-β, a marker of liver fibrosis, and the results are shown in Table 22 below and Figure 35.

[Table 22]

| No | Group | Hepatic TGF-β |
|---|---|---|
| 1 | Normal control group | 77.3 |
| 2 | Negative control group | 177.9 |
| 3 | Experimental group A | 117.4 |
| 4 | Comparative group A (dulaglutide administration group) | 165.3 |

As shown in Table 22 above, it was confirmed that the group administered with the protein conjugate of the present invention exhibited the reduced TGF-β value, and the value was significantly reduced compared to the group administered with dulaglutide.

In addition, triglyceride accumulation in the liver tissue was analyzed using the Triglyceride Assay Kit, and the results are shown in Table 23 below and Figure 36. The amount of triglyceride in the liver tissue was hardly reduced in the group administered with dulaglutide, but the group administered with the protein conjugate of the present invention exhibited the value almost similar to that of the normal control group.

[Table 23]

| No | Group | Hepatic Triglyceride |
|---|---|---|
| 1 | Normal control group | 17.13 |
| 2 | Negative control group | 40.09 |
| 3 | Experimental group A | 24.02 |
| 6 | Comparative group A (dulaglutide administration group) | 36.08 |

As a result, it can be seen that the group administered with the protein conjugate C-191 of the present invention exhibited a significantly more excellent effect in all experiments compared to the group administered with dulaglutide.

**[Test Example 6]**

***In-vivo* efficacy test : Efficacy evaluation against obesity**

In order to confirm the effect of the protein conjugate C-192, which is a GCG/GLP-1/FGF21/IL-1RA acceptor

quadruple (tetra) agonist/antagonist prepared in Examples 1 to 9, to reduce blood lipid level, diet-induced obesity mice were used to perform the evaluation. In order to induce obesity by a dietary method, 5-week-old C57BL/6J mice were purchased from Central Lab. Animal Inc., and after the acclimatization period of about 7 days was completed, the western diet was fed for 16 weeks to induce obese mice. As shown in Table 24 below, animals without abnormalities were selected with reference to the average body weight and body weight change, and group separation was performed by 6 animals per group so that the average body weight of each group was equal. For the route of administration, subcutaneous administration was carried out in the same way as the planned clinical application route, and the administration frequency was 1 time/2 days for 8 weeks, i.e., a total of 28 times.

[Table 24]

| No | Group | Average body weight(g) | Diet | Administered composition | Administered dose | Administration cycle | Number of administration |
|---|---|---|---|---|---|---|---|
| 1 | Normal control group | 27.1 ± 1.3 | Solid feed for experimental animals | Excipient | - | 1 time/2 days | 28 times |
| 2 | Negative control group | 44.7 ± 23 | RD western diet | Excipient | - | 1 time/2 days | 28 times |
| 3 | Experimental group A | 43.9 ± 1.1 | RD western diet | C-192 | 10 nmol/kg | 1 time/2 days | 28 times |
| 4 | Experimental group B | 42.1 ± 4.1 | RD western diet | C-192 | 30 nmol/kg | 1 time/2 days | 28 times |
| 5 | Experimental group C | 41.7 ± 4.4 | RD western diet | C-192 | 60 nmol/kg | 1 time/2 days | 28 times |

For the administered composition, an excipient was administered to the normal control group and the negative control group, and C-192 was administered at a dose of 10, 30 and 60 nmol/kg to the experimental group. During the 8-week administration period, general symptoms such as appearance, behavior and excretion were observed once a day. On the day of the end of administration, blood was collected from the abdominal vena cava, put in an SST tube, and centrifuged at 3,000 rpm for 15 minutes to separate the serum, and then a hematological-biochemical analyzer (7180, HTTACHI, Japan) was used to measure the biomarkers shown in Table 25 below.

[Table 25]

| Lipid-related biomarker | Unit | Measurement method |
|---|---|---|
| Triglyceride (TG) | mg/dL | GPO-HMMPS Glycerol blanking |
| Free fatty acid (Non esterified fatty acid, NEFA) | μEq/L | ACS·ACOD |
| Total cholesterol (T-Chol) | mg/dL | Cholesterol oxidase-HMMPS |
| Low-density lipoprotein cholesterol (LDL-cholesterol) | mg/dL | Selective protection enzymatic |
| High-density lipoprotein cholesterol (HDL-cholesterol) | mg/dL | Direct |

In order to evaluate the effect of C-192 on lipid improvement, triglyceride (TG), free fatty acid (NEFA), total cholesterol (T-Chol), low-density lipoprotein cholesterol (LDL), and high-density lipoprotein cholesterol (HDL) in the serum were measured, and the results are shown in Table 26 below and Figures 37 to 41.

Table 26]

| No | Group | TG (mg/dL) | NEFA (pEq/L) | T-Chol (mg/dL) | LDL (mg/dL) | HDL (mg/dL) |
|---|---|---|---|---|---|---|
| 1 | Normal control group | 14.7 | 1.2 | 105.3 | 2.9 | 62.8 |
| 2 | Negative control group | 26.0 | 1.5 | 241.8 | 21.7 | 99.2 |
| 3 | Experimental group A | 24.2 | 1.3 | 227.0 | 19.9 | 102.8 |
| 4 | Experimental group B | 18.0 | 1.1 | 162.0 | 13.6 | 74.7 |
| 5 | Experimental group C | 16.0 | 1.2 | 167.2 | 12.7 | 79.8 |

As shown in Table 26 above and Figures 37 to 41, it was confirmed that the levels of lipid-related biomarkers in the blood were reduced in the group administered with C-192. In particular, it was confirmed that in the case of the group administered with C-192 at 60 nmol/kg (Experimental group C), triglyceride (TG) was reduced by 38.5%, free fatty acid (NEFA) was reduced by 20%, total cholesterol (T-Chol) was reduced by 30.9%, low-density lipoprotein cholesterol (LDL-Chol) was reduced by 41.5%, and high-density lipoprotein cholesterol (HDL-Chol) was reduced by 19.6% compared to the negative control group. In addition, there was a statistically significant difference in all biomarkers except high-density lipoprotein cholesterol when compared to the negative control group. Therefore, it can be seen that the protein conjugate of the present invention reduces blood lipids in diet-induced obese mice.

**[Test Example 7]**

***In-vivo* efficacy test : Efficacy evaluation against diabetes**

In order to confirm the anti-diabetic efficacy of the protein conjugate C-192, which is a GCG/GLP-1/FGF21/IL-1RA acceptor quadruple (tetra) agonist/antagonist prepared in Examples 1 to 9, db/db mice, which are type 2 diabetes model mice, were used to perform the evaluation. The db/db mouse is an animal model in which obesity and type 2 diabetes mellitus are induced because the db/db mouse has a mutation in Lepr, a leptin acceptor gene on chromosome 4, resulting in no signal transduction of leptin, a hormone secreted by adipocytes. C57BL/6J mice were used as normal animals, and 7-week-old male mice were purchased from Japan SLC and subjected to quarantine and acclimatization for 7 days to obtain 8-week-old mice, which were used as experimental animals.

As shown in Table 27 below, the composition of the experimental group was classified into the normal control group, the negative control group, the group administered with a test substance, and the positive control group. After quarantine and acclimatization were completed, group separation was performed by 5 animals per group so that the body weight and the blood glucose were equal. For the route of administration, subcutaneous administration was carried

out in the same way as the planned clinical application route, and administration was carried out for 8 days. The administration frequency was 1 time/1 day for the normal control group, the negative control group and the experimental group, and 1 time/2 days for the positive control group.

Table 27

| No | Lineage and Species | Group | Administered composition | Administered dose | Administration cycle | Number of administration |
|---|---|---|---|---|---|---|
| 1 | C57BL/6J mouse | Normal control group | Excipient | - | 1 time/1 day | 8 times |
| 2 | db/db mouse | Negative control group | Excipient | - | 1 time/1 day | 8 times |
| 3 | | Experimental group A | C-192 | 10 nmol/kg | 1 time/1 day | 8 times |
| 4 | | Experimental group B | C-192 | 60 nmol/kg | 1 time/1 day | 8 times |
| 5 | | Positive control group | Semaglutide | 10 nmol/kg | 1 time/2 days | 4 times |

For the administered composition, an excipient was administered to the normal control group and the negative control group, and C-192 was administered at a dose of 10 and 60 nmol/kg to the experimental group. During the 8-day administration period, general symptoms were observed once a day. In order to confirm the anti-diabetic effect in db/db mice, blood glucose, body weight, food, and water were measured. For the blood glucose, non-fasting blood glucose was measured, and blood was collected from the tail vein 1 time/2 days before administration, and the blood glucose was measured using a blood glucose meter (G-Doctor). In order to confirm changes in body weight, food, and water, measurements were carried out on the 1st day of administration, 4th day of administration, and 8th day of administration, and the results are shown in Table 28 below and Figures 42 to 49.

[Table 28]

| No | Group | Glucose (mg/dL) | Weight (g) | Food (g) | Water (mL) |
|---|---|---|---|---|---|
| 1 | Normal control group | 161.0 | 23.6 | 15.4 | 33.1 |
| 2 | Negative control group | 561.9 | 33.2 | 26.6 | 97.2 |
| 3 | Experimental group A | 409.6 | 33.7 | 20.4 | 61.5 |
| 4 | Experimental group B | 216.8 | 30.6 | 16.7 | 48.6 |
| 5 | Positive control | 444.5 | 32.3 | 21.9 | 41.3 |
| | group | | | | |

As shown in Table 28 above and Figures 42 to 49, it was confirmed that blood glucose, food consumption, and water consumption were reduced in the group administered with C-192. In particular, in the case of the group administered with C-192 at 60 nmol/kg (Experimental group B), it was confirmed that the blood glucose level on the 9th day was significantly reduced by 61.4 %, the body weight on the 8th day was significantly reduced by 7.8 %, the food consumption was significantly reduced by 37.1 %, and the water consumption was significantly reduced by 50.0 % compared to the negative control group. Therefore, it can be seen that the protein conjugate of the present invention exhibits an anti-diabetic effect.

**[Test Example 8]**

***In silica* immunogenicity analysis**

The *in silica* immunogenicity analysis was performed for each acceptor and donor constituting the protein conjugates C-191 and C-192, which are GCG/GLP-1/FGF21/GIP or GCG/GLP-1/FGF21/IL-1RA acceptor quadruple (tetra) agonists/antagonists prepared in Examples 1 to 9. Immunogenicity refers to a property that induces an immune

response when a drug is administered into the human body, and is a factor that affects not only drug efficacy but also safety. The *in silica* analysis was performed using an immunogenicity-inducing sequence database and computer analysis program, and the *in vitro* immunogenicity evaluation was performed using human-derived PBMCs.

As a result, as shown in Figures 50 to 55, the level of induction of immunogenicity was predicted with respect to the 27 HLA types shown in Table 29 below, which are the most frequent worldwide.

[Table 29]

| Seque nce No. | HLA type | | Seque nce No. | HLA type |
|---|---|---|---|---|
| 1 | HLA-DRB1*01:01 | | 15 | HLA-DRB*501:01 |
| 2 | HLA-DRB1*03:01 | | 16 | HLA-DQA1*05:01/DQB1*02:01 |
| 3 | HLA-DRB1*04:01 | | 17 | HLA-DQA1*05:01/DQB1*03:01 |
| 4 | HLA-DRB1*04:05 | | 18 | HLA-DQA1*03:01/DQB1*03:02 |
| 5 | HLA-DRB1*07:01 | | 19 | HLA-DQA1*04:01/DQB1*04:02 |
| 6 | HLA-DRB1*08:02 | | 20 | HLA-DQA1*01:01/DQB1*05:01 |
| 7 | HLA-DRB1*09:01 | | 21 | HLA-DQA1*01:02/DQB1*06:02 |
| 8 | HLA-DRB1*11:01 | | 22 | HLA-DPA1*02:01/DPB1*01:01 |
| 9 | HLA-DRB1*12:01 | | 23 | HLA-DPA1*01:03/DPB1*02:01 |
| 10 | HLA-DRB1*13:02 | | 24 | HLA-DPA1*01/DPB1*04:01 |
| 11 | HLA-DRB1*15:01 | | 25 | HLA-DPA1*03:01/DPB1*04:02 |
| 12 | HLA-DRB3*01:01 | | 26 | HLA-DPA1*02:01/DPB1*05:01 |
| 13 | HLA-DRB3*02:02 | | 27 | HLA-DPA1*02:01/DPB1*14:01 |
| 14 | HLA-DRB4*01:01 | | | |

The protein sequences of each acceptor and donor were predicted to have a low probability of binding to the antigen binding sites of MHC I and MHC II for each of the 27 HLA types. It was predicted that safety was high as each acceptor and donor had low immunogenicity.

**[Test Example 9]**

**API Screening**

**GLP-1 acceptor agonist screening**

In order to test the activity levels of the native GLP-1 and the GLP-1 analogue at the cellular level (in vitro), the cAMP accumulation assay was performed in the same manner as in Test Example 1.

The EC50 values of the GLP-1 acceptor single agonists were calculated and are shown in Table 30 below.

[Table 30]

| Item | Cell line | EC50 (pM) | Comments | SEQ ID NO |
|---|---|---|---|---|
| Liraglutide | Transient | 43.0 ± 10.6 | n=11 | - |
| | Stable | 50.3 ± 21.9 | n=8 | |
| Dulaglutide | Stable | 1.45 ± 0.45 | n=6 | - |
| GLP-1(7-36)_native | Transient | 400 | n=1 | SEQ ID NO: 4 |
| GLP#1 | Transient | 159 | n=1 | SEQ ID NO: 5 |
| GLP#3 | Transient | 2.25 ± 0.19 | n=2 | SEQ ID NO: 6 |
| GLP#4 | Transient | 3.19 ± 0.74 | n=2 | SEQ ID NO: 7 |

(continued)

| Item | Cell line | EC50 (pM) | Comments | SEQ ID NO |
|---|---|---|---|---|
| GLP#5 | Transient | 7.52 | n=1 | SEQ ID NO: 8 |
| GLP#6 | Transient | 1.50 ± 0.06 | n=2 | SEQ ID NO: 9 |
| GLP#7 | Transient | 23.0 | n=1 | SEQ ID NO: 10 |
| GLP#8 | Transient | 1.95 ± 0.03 | n=2 | SEQ ID NO: 11 |
| GLP#9 | Transient | 0.71 ± 0.37 | n=3 | SEQ ID NO: 12 |
| | Stable | 2.08 ± 1.60 | n=4 | |
| GLP#10 | Transient | 8.26 ± 4.02 | n=2 | SEQ ID NO: 13 |
| GLP#11 | Transient | 6.05 | n=1 | SEQ ID NO: 14 |
| GLP#12 | Transient | 559 | n=1 | - |
| GLP#13 | Transient | 31.9 ± 7.28 | n=2 | - |
| GLP#14 | Transient | >5000 | n=1 | - |
| GLP#15 | Transient | >5000 | n=1 | - |

The EC50 values of the GLP-1/GCG acceptor dual agonists were calculated and are shown in Table 31 below.

[Table 31]

| Item | Cell line | EC50 (pM) | Comments | SEQ ID NO |
|---|---|---|---|---|
| GCG#2 | Transient | 52.0 ± 27.4 | n=2 | SEQ ID NO: 2 |
| | Stable | 10.1 ± 1.23 | n=2 | |
| GCG#3 | Transient | 43.9 ± 28.3 | n=2 | SEQ ID NO: 3 |
| | Stable | 25.6 ± 26.9 | n=3 | |
| GLP/GCG#1 | Transient | 588 | n=1 | - |
| GLP/GCG#2 | Transient | 201 | n=1 | - |
| GLP/GCG#3 | Transient | 13 | n=1 | SEQ ID NO: 15 |
| GLP/GCG#4 | Transient | 15.0 ± 7.00 | n=2 | - |
| GLP/GCG#5 | Transient | 165 | n=1 | - |
| GLP/GCG#6 | Transient | 129 | n=1 | - |
| GLP/GCG#7 | Transient | 3860 | n=1 | - |
| GLP/GCG#8 | Transient | 12.5 | n=1 | - |
| GLP/GCG#9 | Transient | 1589 | n=1 | - |
| GLP/GCG#10 | Transient | 9742 | n=1 | - |
| GLP/GCG#11 | Transient | >5000 | n=1 | - |
| GLP/GCG#12 | Transient | 80.1 | n=1 | - |
| GLP/GCG#13 | Transient | 604 | n=1 | - |
| GLP/GCG#14 | Transient | 221 | n=1 | - |
| GLP/GCG#15 | Transient | 16 | n=1 | SEQ ID NO: 16 |

The EC50 values of the GLP-1/GIP acceptor dual agonists were calculated and are shown in Table 32 below.

[Table 32]

| Item | Cell line | EC50 (pM) | Comments | SEQ ID NO |
|---|---|---|---|---|
| GLP/GIP#2 | Transient | 57.8 | n=1 | - |
| GLP/GIP#3 | Transient | >5000 | n=1 | - |
| GLP/GIP#4 | Transient | >5000 | n=1 | - |

## GIP acceptor agonist screening

In order to test the activity levels of the native GIP and the GIP analogue at the cellular level (in vitro), the cAMP accumulation assay was performed in the same manner as in Test Example 1.

The EC50 values of the GIP acceptor single agonists and the GLP-1/GIP acceptor dual agonists were calculated and are shown in Table 33 below.

[Table 33]

| Item | Cell line | EC50 (pM) | Comments | SEQ ID NO |
|---|---|---|---|---|
| GIP native-Ub | Transient | 8.93 ± 5.77 | n=4 | SEQ ID NO: 22 |
| | Stable | 35.0 | n=1 | |
| GIP#1 | Transient | 17.5 ± 10.6 | n=3 | SEQ ID NO: 23 |
| GIP#2 | Transient | 6.63 ± 3.87 | n=3 | SEQ ID NO: 24 |
| | Stable | 12.0 ± 5.73 | n=2 | |
| GIP#3 | Transient | 171 | n=1 | - |
| GIP#4 | Transient | >5000 | n=1 | - |
| GIP#5 | Transient | 81.9 | n=1 | SEQ ID NO: 25 |
| GIP#6 | Transient | 398 | n=1 | - |
| GIP#7 | Transient | 769 | n=1 | SEQ ID NO: 26 |
| GIP#8 | Transient | 560 | n=1 | - |
| GLP/GIP#2 | Transient | 128 | n=1 | - |
| GLP/GIP#3 | Transient | 566 | n=1 | - |
| GLP/GIP#4 | Transient | 1283 | n=1 | - |

## GCG acceptor agonist screening

In order to test the activity levels of the native GCG and the GCG analogue at the cellular level (in vitro), the cAMP accumulation assay was performed in the same manner as in Test Example 1.

The EC50 values of the GCG acceptor single agonists were calculated and are shown in Table 34 below.

[Table 34]

| Item | Cell line | EC50 (pM) | Comments | SEQ ID NO |
|---|---|---|---|---|
| GCG native-Ub | Transient | 41.7 ± 29.9 | n=3 | SEQ ID NO: 1 |
| | Stable | 35.3 ± 32.7 | n=2 | |
| GCG#1 | Transient | >5000 | n=1 | - |
| GCG#2 | Transient | 41.7 ± 5.51 | n=3 | SEQ ID NO: 2 |
| | Stable | 68.5 ± 41.8 | n=2 | |
| GCG#3 | Transient | 47.8 ± 21.9 | n=3 | SEQ ID NO: 3 |
| | Stable | 21.1 ± 12.3 | n=3 | |

(continued)

| Item | Cell line | EC50 (pM) | Comments | SEQ ID NO |
|---|---|---|---|---|
| GCG#4 | Transient | >5000 | n=1 | - |
| GCG#5 | Transient | 661 ± 193 | n=3 | - |
| GCG#6 | Transient | 8400 | n=1 | - |
| GCG#7 | Transient | 956 | n=1 | - |
| GCG#8 | Transient | 761 ± 73.5 | n=2 | - |
| GCG#9 | Transient | 1073 | n=1 | - |
| GCG#10 | Transient | >5000 | n=1 | - |
| GCG#11 | Transient | >5000 | n=1 | - |

The EC50 values of the GLP-1/GCG acceptor dual agonists were calculated and are shown in Table 35 below.

[Table 35]

| Item | Cell line | EC50 (pM) | Comments | SEQ ID NO |
|---|---|---|---|---|
| GLP/GCG#1 | Transient | >5000 | n=1 | - |
| GLP/GCG#2 | Transient | >5000 | n=1 | - |
| GLP/GCG#3 | Transient | >5000 | n=1 | SEQ ID NO: 15 |
| GLP/GCG#4 | Transient | >5000 | n=1 | - |
| GLP/GCG#5 | Transient | >5000 | n=1 | - |
| GLP/GCG#6 | Transient | >6250 | n=1 | - |
| GLP/GCG#7 | Transient | >5000 | n=1 | - |
| GLP/GCG#8 | Transient | >5000 | n=1 | - |
| GLP/GCG#9 | Transient | >5000 | n=1 | - |
| GLP/GCG#10 | Transient | >5000 | n=1 | - |
| GLP/GCG# 11 | Transient | >5000 | n=1 | - |
| GLP/GCG#12 | Transient | 321 | n=1 | - |
| GLP/GCG#13 | Transient | >5000 | n=1 | - |
| GLP/GCG#14 | Transient | >5000 | n=1 | - |
| GLP/GCG#15 | Transient | 424 | n=1 | SEQ ID NO: 16 |

**FGF21 acceptor agonist screening**

In order to test the activity levels of the native FGF21 and the FGF21 analogue at the cellular level (in vitro), the FGFR1/KLB functional assay was performed in the same manner as in Test Example 2.

The EC50 values of the FGF21 acceptor single agonists were calculated and are shown in Table 36 below.

[Table 36]

| Item | EC50 (nM) | Comments | SEQ ID NO |
|---|---|---|---|
| rhFGF21 (Native) | 1.17 ± 0.56 | n=4 | SEQ ID NO: 17 |
| FGF#1 | 21.0 ± 0.49 | n=2 | SEQ ID NO: 18 |
| FGF#5 | 22.3 ± 0.71 | n=2 | SEQ ID NO: 19 |
| FGF#7 | 17.7 ± 2.55 | n=2 | SEQ ID NO: 20 |

(continued)

| Item | EC50 (nM) | Comments | SEQ ID NO |
|---|---|---|---|
| FGF#9 | 19.1 ± 0.49 | n=2 | SEQ ID NO: 21 |
| FGF#11 | > 5000 | n=1 | - |
| FGF#12 | > 5000 | n=1 | - |
| FGF#13 | > 5000 | n=1 | - |
| FGF#14 | > 5000 | n=1 | - |
| FGF#15 | > 5000 | n=1 | - |

**IL-1RA acceptor antagonist screening**

In order to test the ability of the native IL-1RA and the IL-1RA analogue to inhibit NF-κB activity by IL-1β at the cellular level (in vitro), the NF-xB reporter gene luciferase assay was performed in the same manner as in Test Example 3.

The IC50 values of the IL-1RA acceptor single antagonists were calculated and are shown in Table 37 below.

[Table 37]

| Item | IC50 (pM) | Comments | SEQ ID NO |
|---|---|---|---|
| rhIL-1RA (Native) | 68.6 ± 7.71 | n=2 | SEQ ID NO: 27 |
| II,-1RA-Ub (Donor) | 236 ± 43.8 | n=2 | SEQ ID NO: 28 |

As a result of screening the above various agonists and antagonists, the biomolecules having excellent effects were selected to prepare the protein conjugate of the present invention. As a result, it was confirmed that the protein conjugate of the present invention has excellent effects in the prevention and treatment of non-alcoholic steatohepatitis (NASH), fatty liver, liver fibrosis, cirrhosis, liver cancer, obesity, or diabetes, and also has excellent stability.

**Claims**

**1.** A protein conjugate, **characterized in that** the protein conjugate comprises:

polyubiquitin,
a carrier linked directly or by a linker to the polyubiquitin, and
a biomolecule linked directly or by a linker to the polyubiquitin or the carrier,
wherein the polyubiquitin is composed of (i) an acceptor ubiquitin containing one or more unsubstituted lysines in which lysines of the ubiquitin may be substituted with arginine or alanine, and (ii) a donor ubiquitin in which all lysines of the ubiquitin are substituted with arginine or alanine, and
the biomolecule is two or more selected from the group consisting of GCG, GLP-1, FGF21, GIP and IL-1RA; analogues thereof; a GCG and GLP-1 dual acceptor agonist; and a GLP-1 and GIP dual acceptor agonist.

**2.** The protein conjugate according to claim 1, **characterized in that** the GCG and analogue thereof are selected from the group consisting of proteins composed of the amino acid sequences of SEQ ID NOs: 1 to 3.

**3.** The protein conjugate according to claim 2, **characterized in that** the GCG analogue is a protein composed of the amino acid sequence of SEQ ID NO: 2.

**4.** The protein conjugate according to claim 1, **characterized in that** the GLP-1 and analogue thereof are selected from the group consisting of proteins composed of the amino acid sequences of SEQ ID NOs: 4 to 14.

**5.** The protein conjugate according to claim 4, **characterized in that** the GLP-1 analogue is a protein composed of the amino acid sequence of SEQ ID NO: 12.

**6.** The protein conjugate according to claim 1, **characterized in that** the GCG and GLP-1 dual acceptor agonist is

selected from the group consisting of proteins composed of the amino acid sequences of SEQ ID NOs: 15 and 16.

**7.** The protein conjugate according to claim 1, **characterized in that** the FGF21 and analogue thereof are selected from the group consisting of proteins composed of the amino acid sequences of SEQ ID NOs: 17 to 21.

**8.** The protein conjugate according to claim 7, **characterized in that** the FGF21 analogue is a protein composed of the amino acid sequence of SEQ ID NO: 20.

**9.** The protein conjugate according to claim 1, **characterized in that** the GIP and analogue thereof are one or more selected from the group consisting of proteins composed of the amino acid sequences of SEQ ID NOs: 22 to 26.

**10.** The protein conjugate according to claim 9, **characterized in that** the GIP and analogue thereof are a protein composed of the amino acid sequence of SEQ ID NO: 24.

**11.** The protein conjugate according to claim 1, **characterized in that** the IL-1RA and analogue thereof are one or more selected from the group consisting of proteins composed of the amino acid sequences of SEQ ID NOs: 27 and 28.

**12.** The protein conjugate according to claim 11, **characterized in that** the IL-1RA is a protein composed of the amino acid sequence of SEQ ID NO: 27.

**13.** The protein conjugate according to claim 1, **characterized in that** the polyubiquitin is composed of an acceptor ubiquitin in which the 6th, 11th, 27th, 29th, 33rd, and 48th lysines from the N-terminus of the ubiquitin are substituted with arginine and a donor ubiquitin in which all lysines of the ubiquitin are substituted with arginine.

**14.** The protein conjugate according to claim 1, **characterized in that** the polyubiquitin is composed of an acceptor ubiquitin composed of the amino acid sequence of SEQ ID NO: 30 and a donor ubiquitin composed of the amino acid sequence of SEQ ID NO: 31.

**15.** The protein conjugate according to claim 1, **characterized in that** the linker is a polypeptide composed of an amino acid sequence in which 1 to 30 repeats of GGGGS, EAAAK or VPPPPP are combined.

**16.** The protein conjugate according to claim 15, **characterized in that** the linker is a polypeptide composed of the amino acid sequence of SEQ ID NO: 29.

**17.** The protein conjugate according to claim 1, **characterized in that** the carrier is selected from the group consisting of albumin, antibody fragment, scFc (single chain Fc), scFc dimer (single chain Fc-dimer), transferrin, XTEN (genetic fusion of non-exact repeat peptide sequence), CTP (carboxy-terminal peptide), PAS (proline-alanine-serine polymer), ELK (elastin-like peptide), HAP (homo-amino acid polymer), GLK (gelatin-like protein), PEG (polyethylene glycol), and fatty acid.

**18.** The protein conjugate according to claim 17, **characterized in that** the carrier is albumin.

**19.** A protein conjugate represented by the following formula:

$$\begin{array}{c} \text{X-L-Y-L-Ub(A)-L-A-L-Z} \\ | \\ \text{W-L-Ub(D)} \end{array}$$

in the above formula,

W, X, Y and Z are each a biomolecule selected from the group consisting of a GCG analogue composed of the amino acid sequence of SEQ ID NO: 2, a GLP-1 analogue composed of the amino acid sequence of SEQ ID NO: 12, an FGF21 analogue composed of the amino acid sequence of SEQ ID NO: 20, a GIP analogue composed of the amino acid sequence of SEQ ID NO: 24, and IL-1RA composed of the amino acid sequence of SEQ ID NO: 27,

Ub(A) is an acceptor ubiquitin composed of the amino acid sequence of SEQ ID NO: 30,

Ub(D) is a donor ubiquitin composed of the amino acid sequence of SEQ ID NO: 31,

L is each independently absent or a linker,
A is a carrier, and
Ub(A) and Ub(D) are linked by a covalent bond.

**20.** The protein conjugate according to claim 19, **characterized in that** X is a GCG analogue composed of the amino acid sequence of SEQ ID NO: 2, Y is a GLP-1 analogue composed of the amino acid sequence of SEQ ID NO: 12, Z is an FGF21 analogue composed of the amino acid sequence of SEQ ID NO: 20, and W is a GIP analogue composed of the amino acid sequence of SEQ ID NO: 24 or IL-1RA composed of the amino acid sequence of SEQ ID NO: 27.

**21.** The protein conjugate according to claim 19, **characterized in that** the linker is a polypeptide composed of the amino acid sequence of SEQ ID NO: 29.

**22.** The protein conjugate according to claim 19, **characterized in that** the carrier is albumin.

**23.** A pharmaceutical composition for preventing or treating non-alcoholic steatohepatitis, fatty liver, liver fibrosis, cirrhosis, liver cancer, obesity, or diabetes, comprising the protein conjugate according to any one of claims 1 to 22.

**24.** A method for preventing or treating non-alcoholic steatohepatitis, fatty liver, liver fibrosis, cirrhosis, liver cancer, obesity, or diabetes, comprising administering the composition according to claim 23 to a subject other than a human.

**25.** A method for preparing a protein conjugate, **characterized in that** the method comprises the steps of:

(i) preparing an acceptor protein represented by the following formula;

X-L-Y-L-Ub(A)-L-A-L-Z

(ii) preparing a donor protein represented by the following formula; and

W-L-Ub(D)

(iii) linking Ub(A) in the acceptor protein and Ub(D) in the donor protein,
wherein W, X, Y and Z are each a biomolecule selected from the group consisting of a GCG analogue composed of the amino acid sequence of SEQ ID NO: 2, a GLP-1 analogue composed of the amino acid sequence of SEQ ID NO: 12, an FGF21 analogue composed of the amino acid sequence of SEQ ID NO: 20, a GIP analogue composed of the amino acid sequence of SEQ ID NO: 24, and IL-1RA composed of the amino acid sequence of SEQ ID NO: 27,
Ub(A) is an acceptor ubiquitin composed of the amino acid sequence of SEQ ID NO: 30,
Ub(D) is a donor ubiquitin composed of the amino acid sequence of SEQ ID NO: 31,
L is each independently absent or a linker, and
A is a carrier.

**26.** The method for preparing a protein conjugate according to claim 25, **characterized in that** X is a GCG analogue composed of the amino acid sequence of SEQ ID NO: 2, Y is a GLP-1 analogue composed of the amino acid sequence of SEQ ID NO: 12, Z is an FGF21 analogue composed of the amino acid sequence of SEQ ID NO: 20, and W is a GIP analogue composed of the amino acid sequence of SEQ ID NO: 24 or IL-1RA composed of the amino acid sequence of SEQ ID NO: 27.

【Fig. 1】

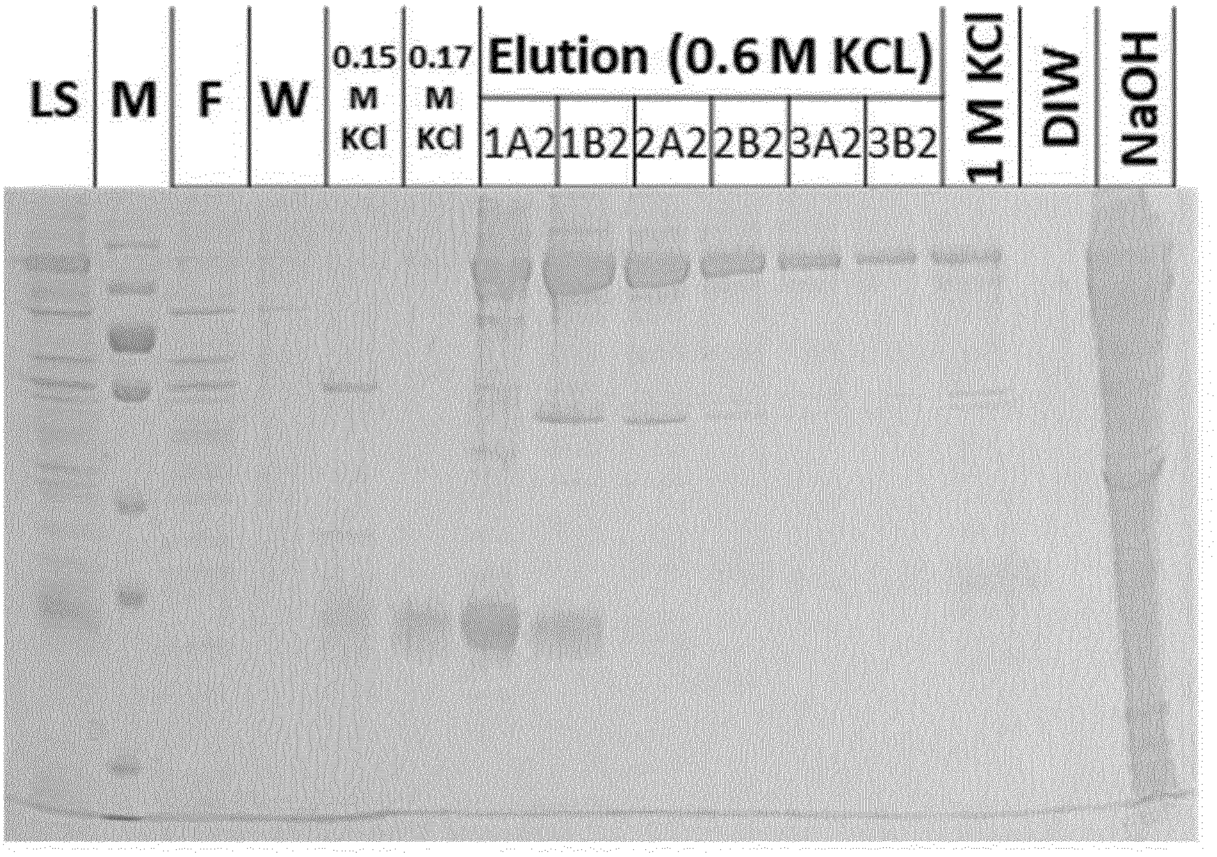
LS
M
F
W
0.15 M KCl
0.17 M KCl
Elution (0.6 M KCL)
1A2
1B2
2A2
2B2
3A2
3B2
1 M KCl
DIW
NaOH

【Fig. 2】

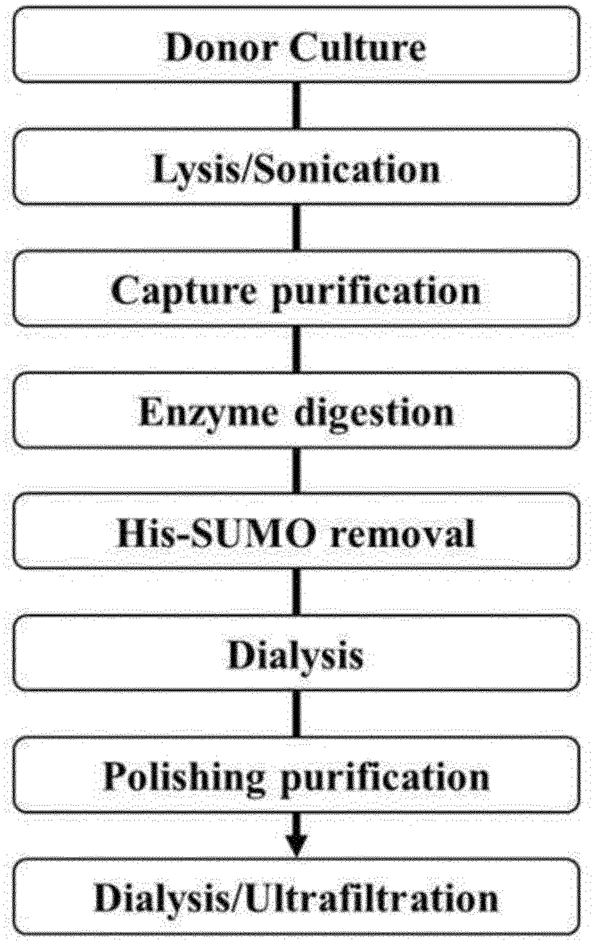
Donor Culture
Lysis/Sonication
Capture purification
Enzyme digestion
His-SUMO removal
Dialysis
Polishing purification
Dialysis/Ultrafiltration

【Fig. 3】

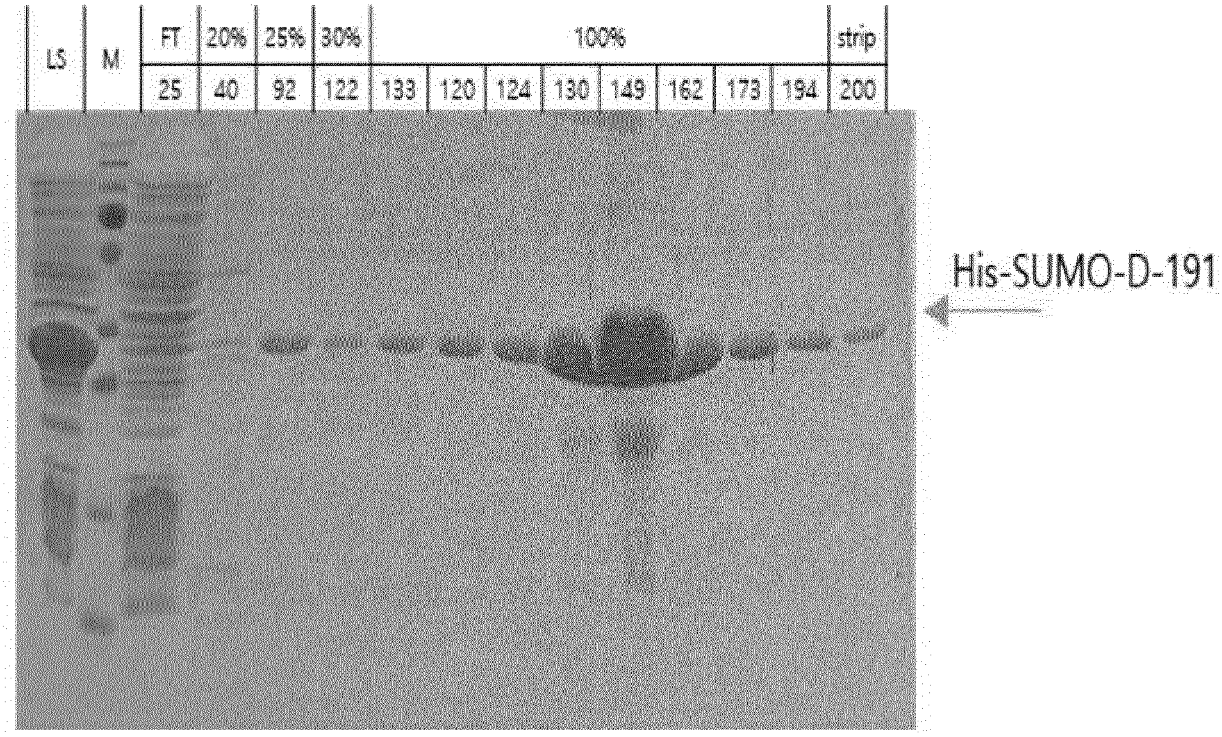
LS
M
FT
20%
25%
30%
100%
strip
25
40
92
122
133
120
124
130
149
162
173
194
200
His-SUMO-D-191

【Fig. 4】

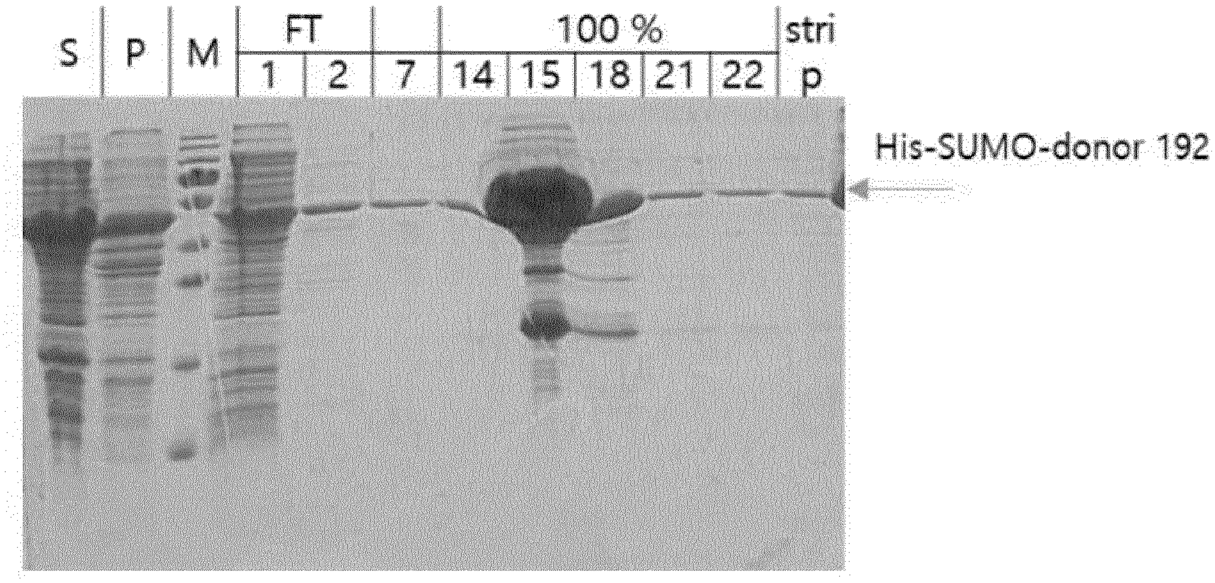

【Fig. 5】

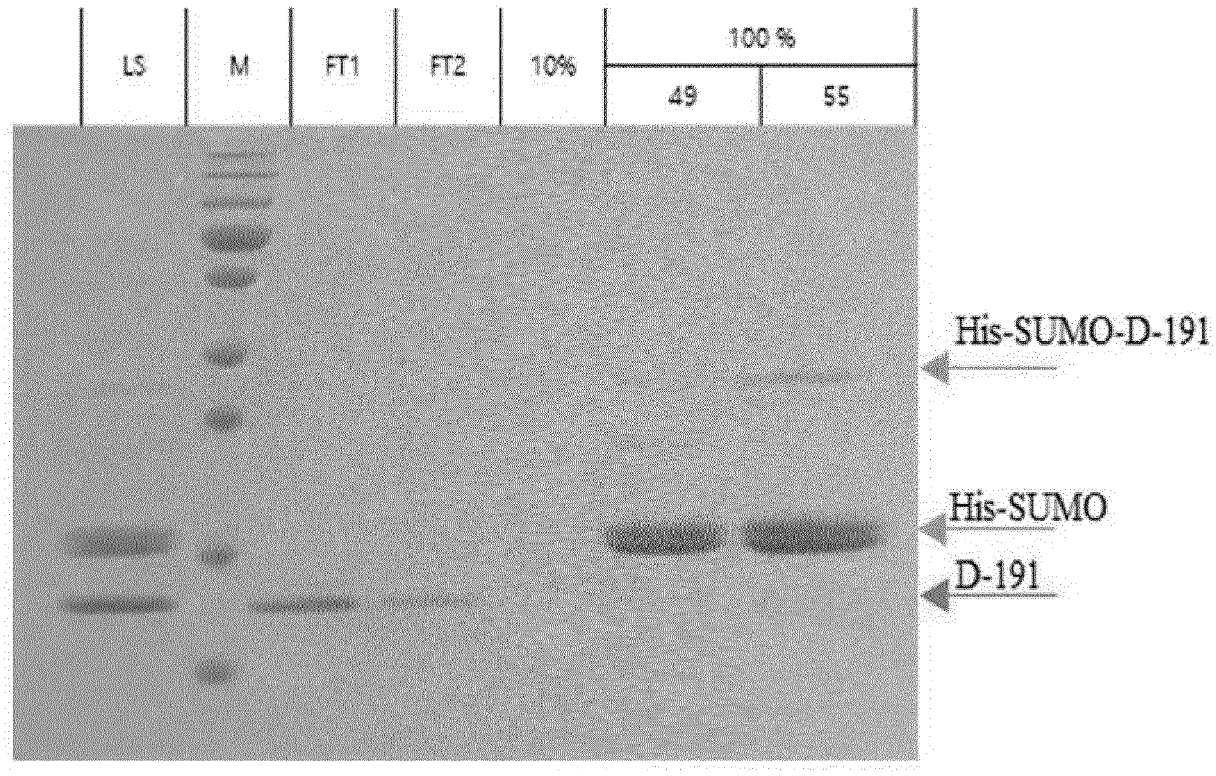

【Fig. 6】

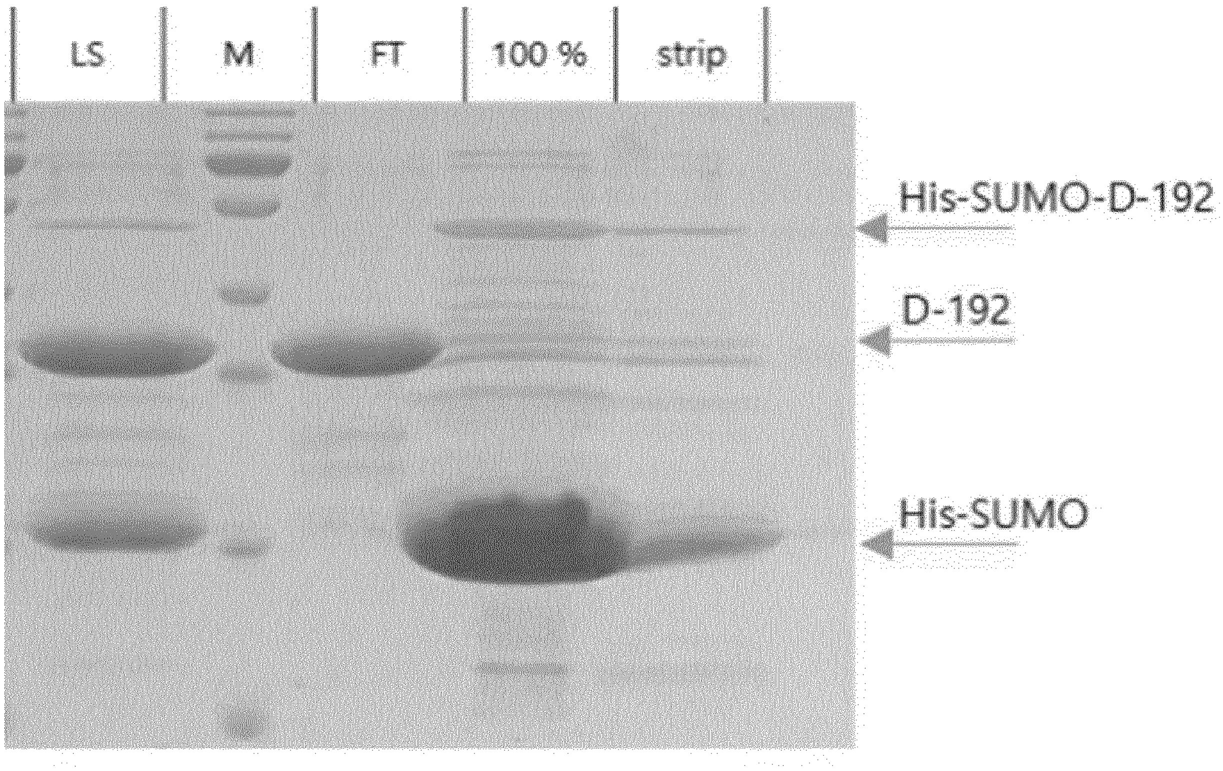

【Fig. 7】

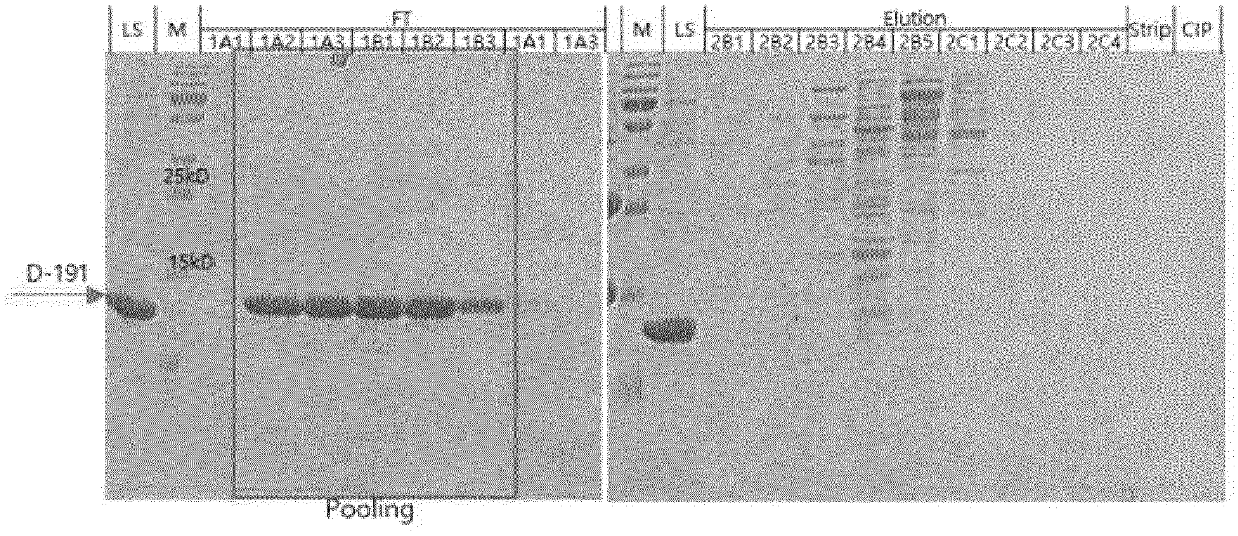
LS
M
FT
1A1
1A2
1A3
1B1
1B2
1B3
1A1
1A3
M
LS
Elution
2B1
2B2
2B3
2B4
2B5
2C1
2C2
2C3
2C4
Strip
CIP
25kD
15kD
D-191
Pooling

【Fig. 8】

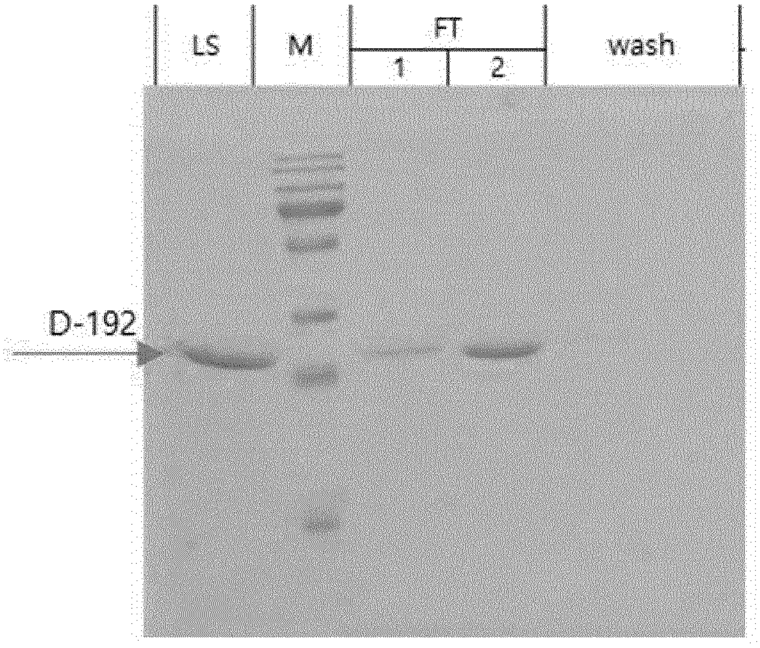

【Fig. 9】

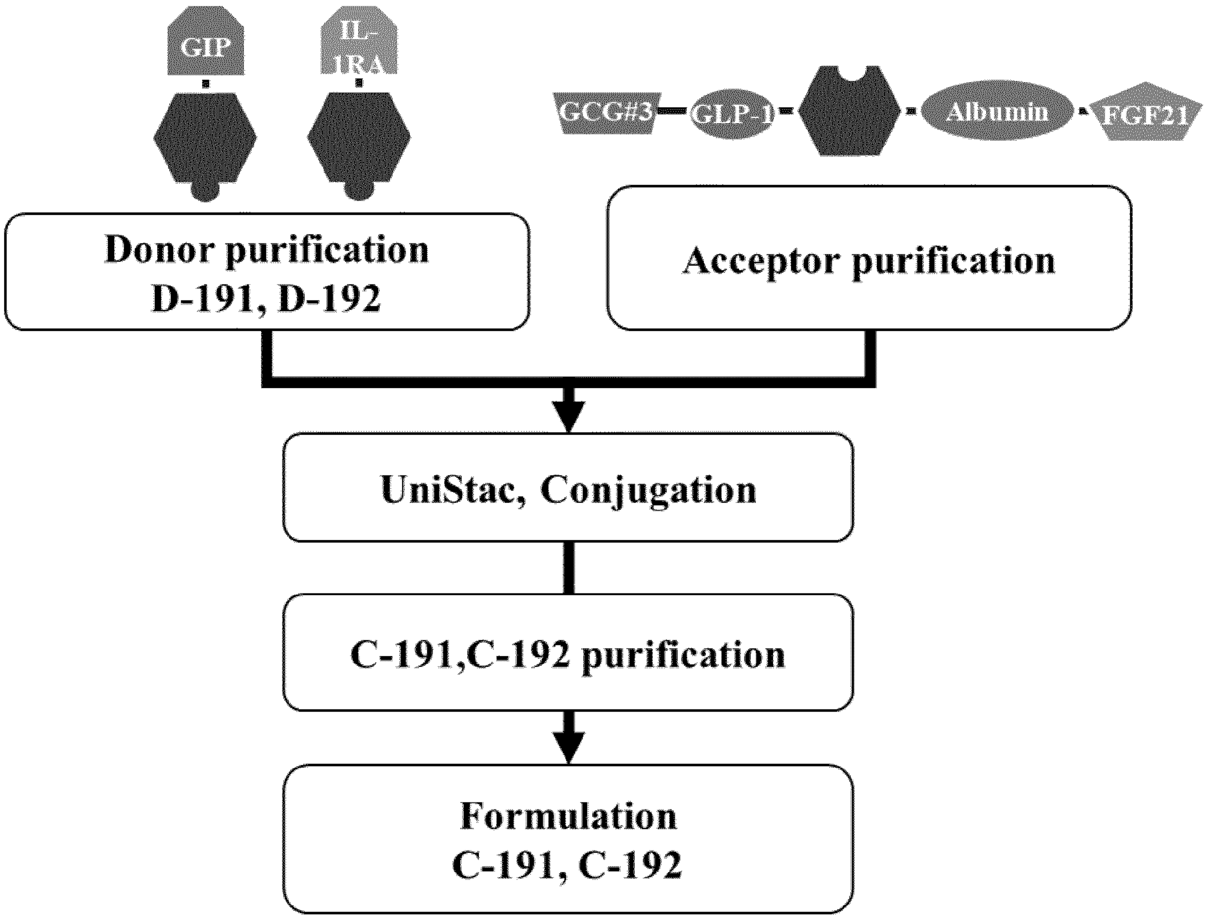
GIP
IL-1RA
GCG#3
GLP-1
Albumin
FGF21
Donor purification
D-191, D-192
Acceptor purification
UniStac, Conjugation
C-191,C-192 purification
Formulation
C-191, C-192

【Fig. 10】

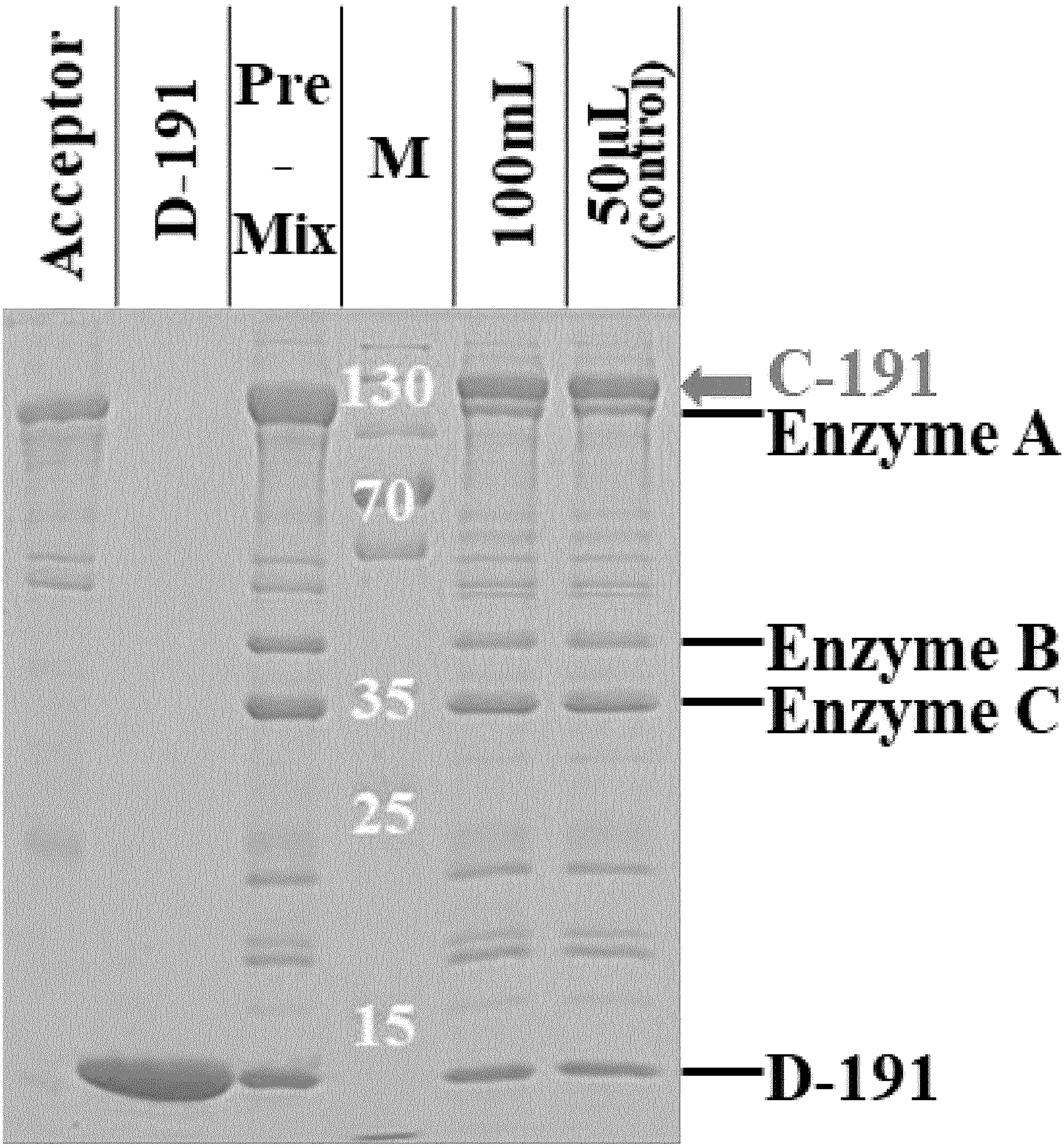
Acceptor
D-191
Pre-Mix
M
100mL
50μL (control)
130
70
35
25
15
C-191
Enzyme A
Enzyme B
Enzyme C
D-191

【Fig. 11】

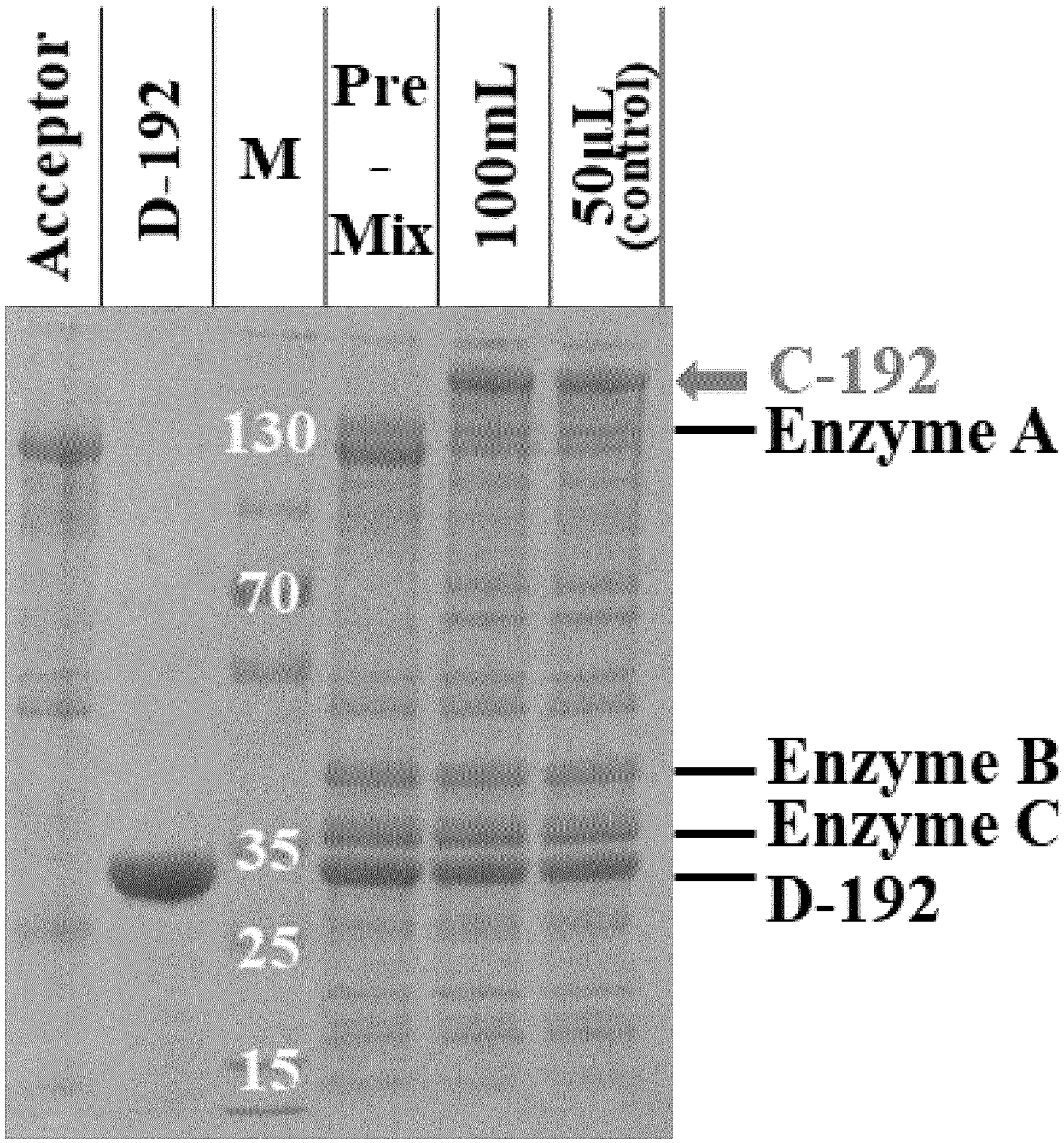
Acceptor
D-192
M
Pre
-
Mix
100mL
50μL
(control)
130
70
35
25
15
C-192
Enzyme A
Enzyme B
Enzyme C
D-192

【Fig. 12】

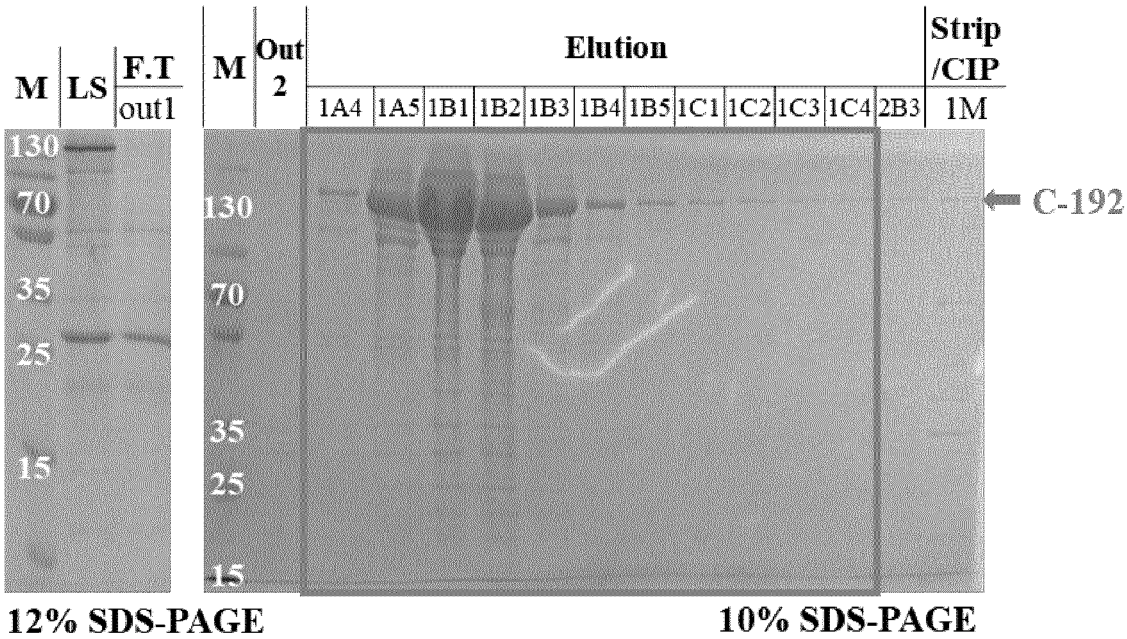
M
LS
F.T
out1
130
70
35
25
15
12% SDS-PAGE
M
Out 2
Elution
1A4
1A5
1B1
1B2
1B3
1B4
1B5
1C1
1C2
1C3
1C4
2B3
Strip /CIP
1M
130
70
35
25
15
C-192
10% SDS-PAGE

【Fig. 13】

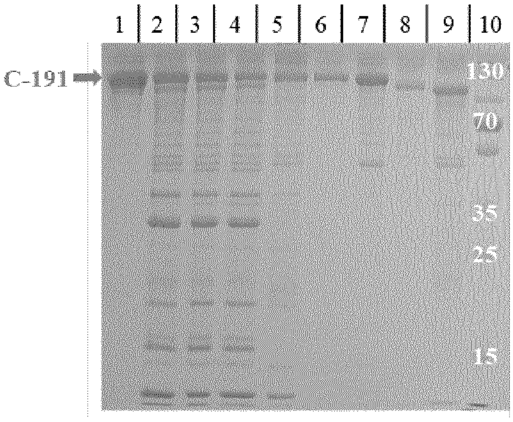


Lane 1: RD191-003-DS, positive control (Lot. No.: S191031)
2: RD191-004-C-01
3: RD191-004-C-02
4: RD191-004-C-03
5: RD191-004-C-NF-01
6: RD191-004-C-AX-02
7: RD191-004-C-NB-01
8: UCTE1 (S191109-01)
9: Acceptor (RD191-A-004-AF-06)
10: Marker (thermo fisher, #26619)

【Fig. 14】

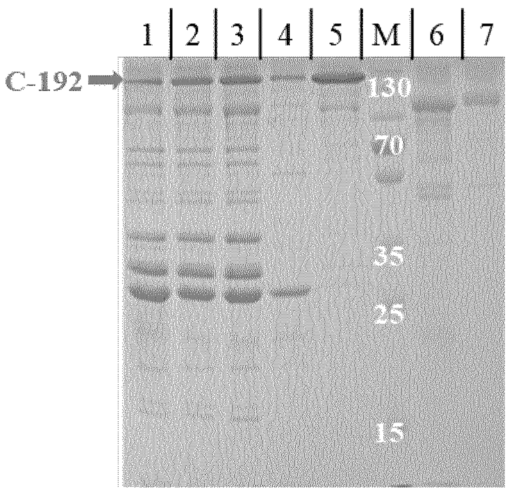


Lane 1: RD192-001-C-03
2: RD192-001-C-02
3: RD192-001-C-01
4: RD192-001-C-NF-01
5: RD192-001-C-AX-01
M: Marker (thermo fisher, #26619)
6: Acceptor (RD191-A-004-AF-06)
7: Enzyme 1 (Lot. No: S191109-01)

【Fig. 15】

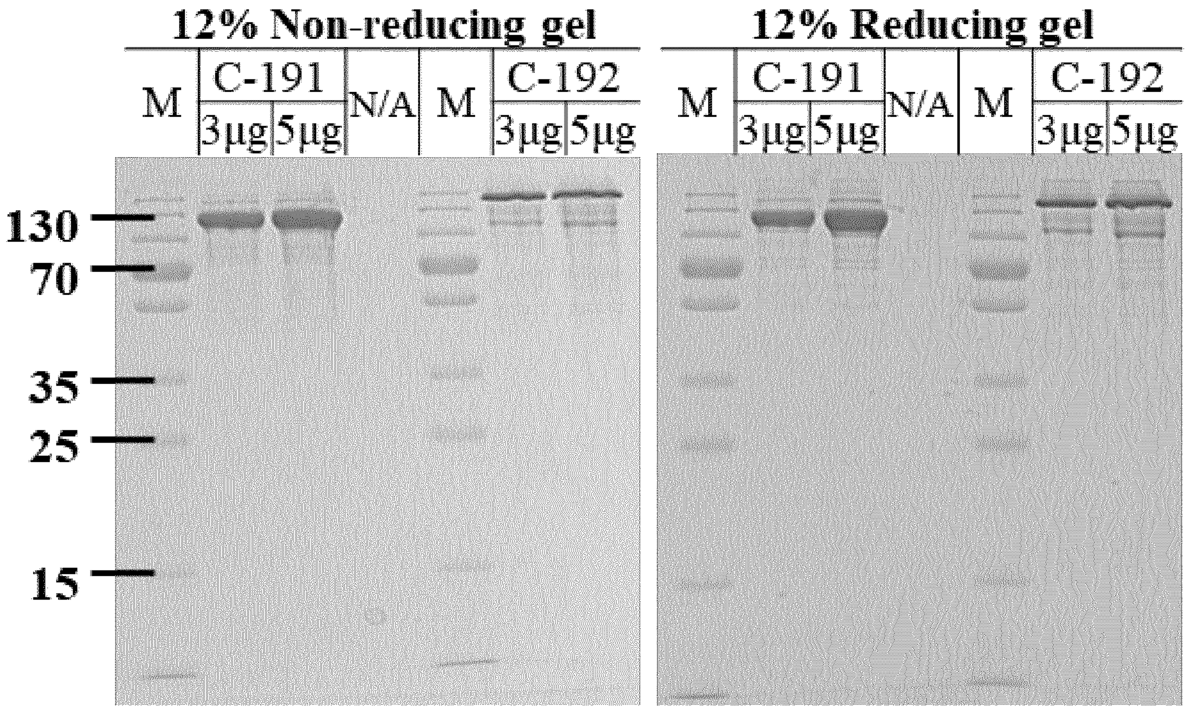
12% Non-reducing gel
M
C-191
3μg
5μg
N/A
M
C-192
3μg
5μg
12% Reducing gel
M
C-191
3μg
5μg
N/A
M
C-192
3μg
5μg
130
70
35
25
15

【Fig. 16】

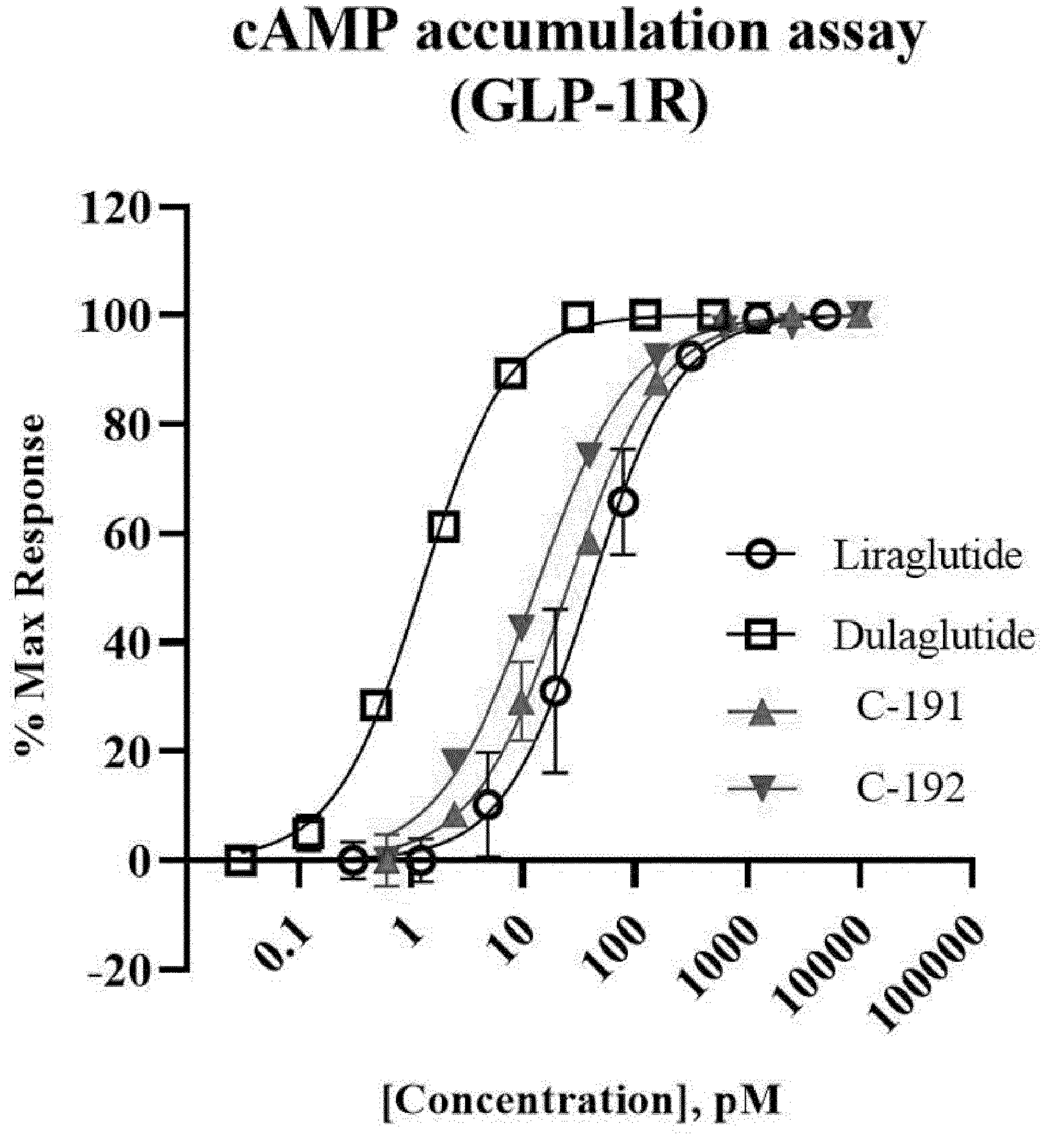
cAMP accumulation assay
(GLP-1R)
% Max Response
120
100
80
60
40
20
0
-20
0.1
1
10
100
1000
10000
100000
[Concentration], pM
Liraglutide
Dulaglutide
C-191
C-192

【Fig. 17】

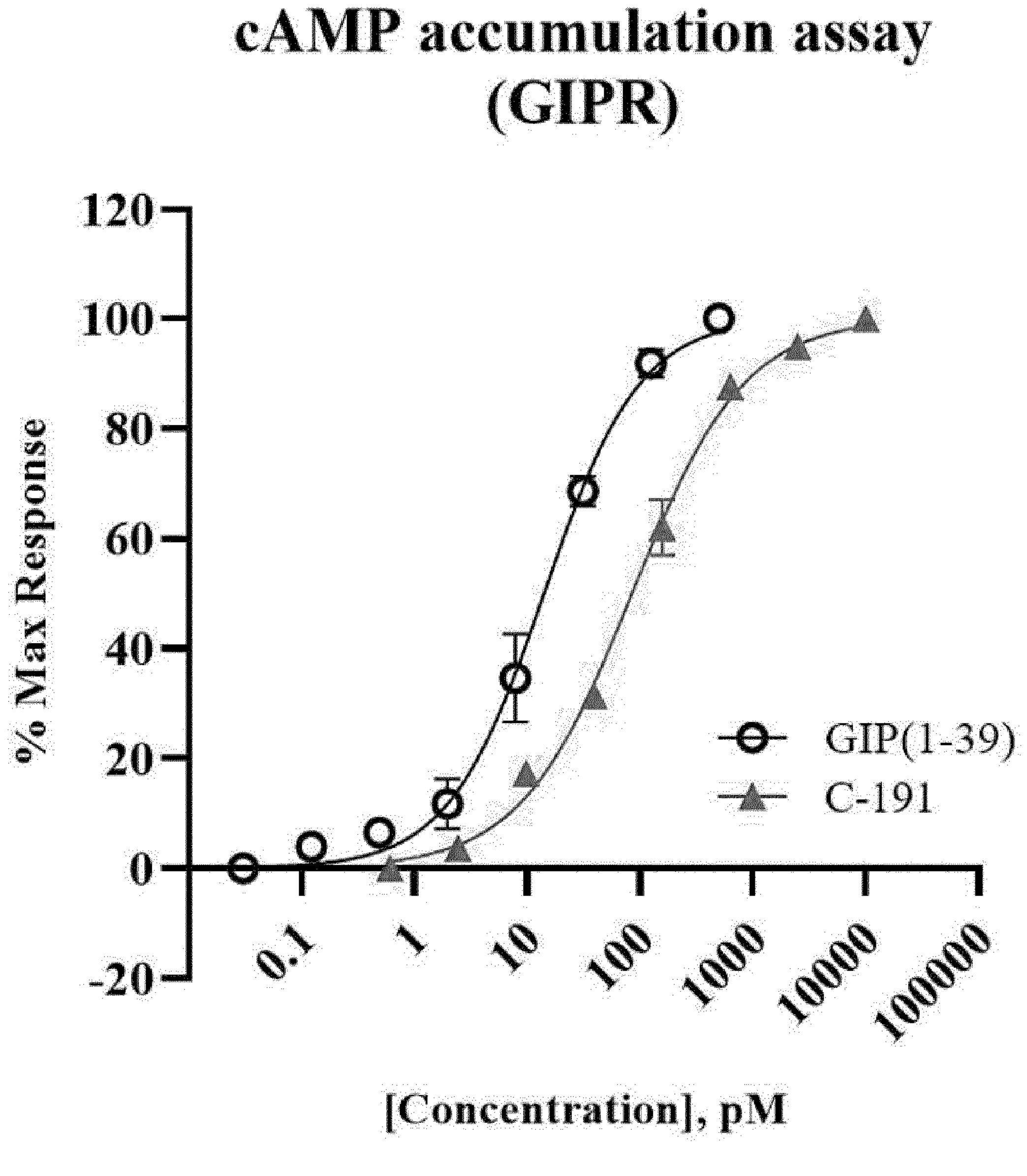
cAMP accumulation assay
(GIPR)
120
100
80
60
40
20
0
-20
% Max Response
0.1
1
10
100
1000
10000
100000
[Concentration], pM
GIP(1-39)
C-191

【Fig. 18】

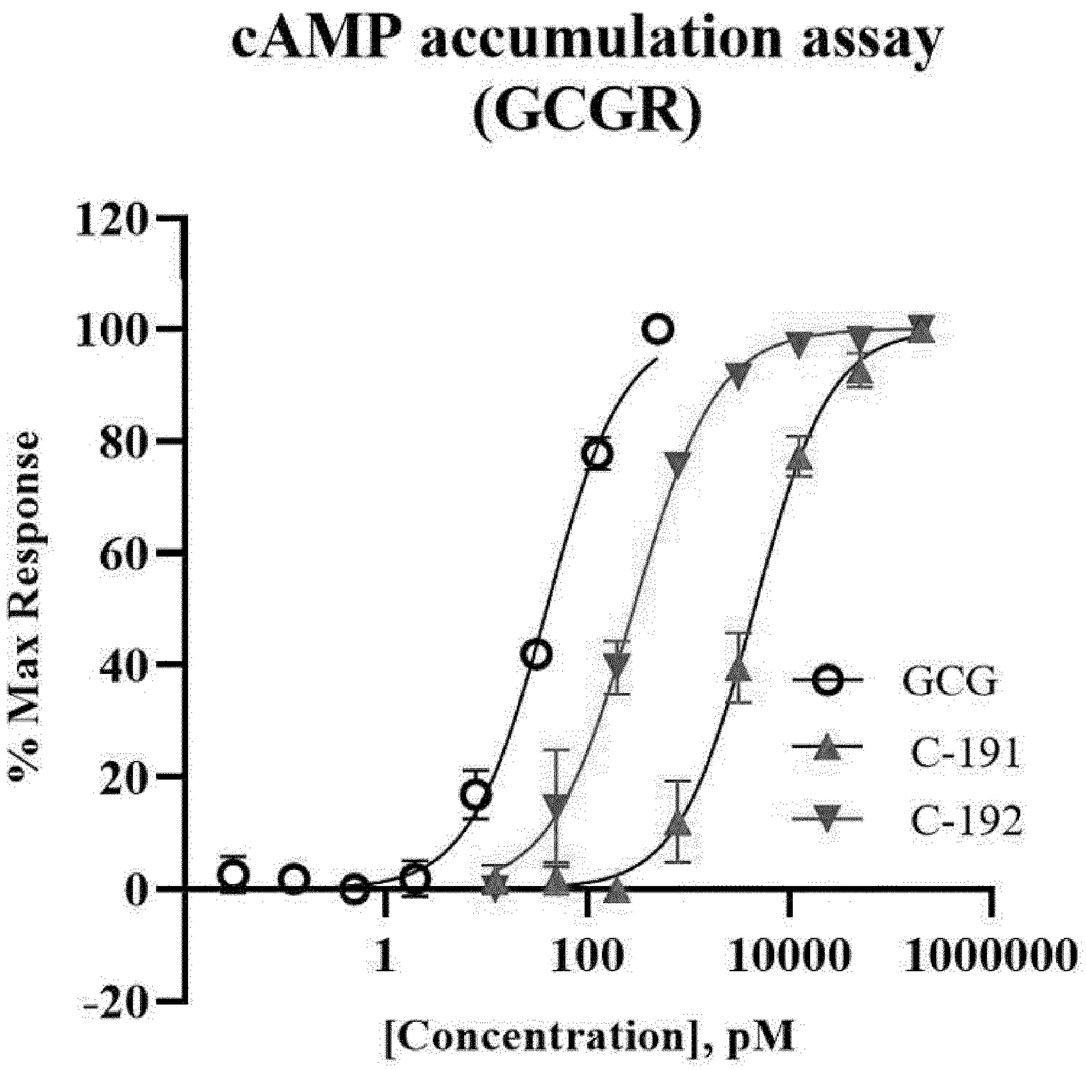
cAMP accumulation assay
(GCGR)
120
100
80
60
40
20
0
-20
% Max Response
1
100
10000
1000000
[Concentration], pM
GCG
C-191
C-192

【Fig. 19】

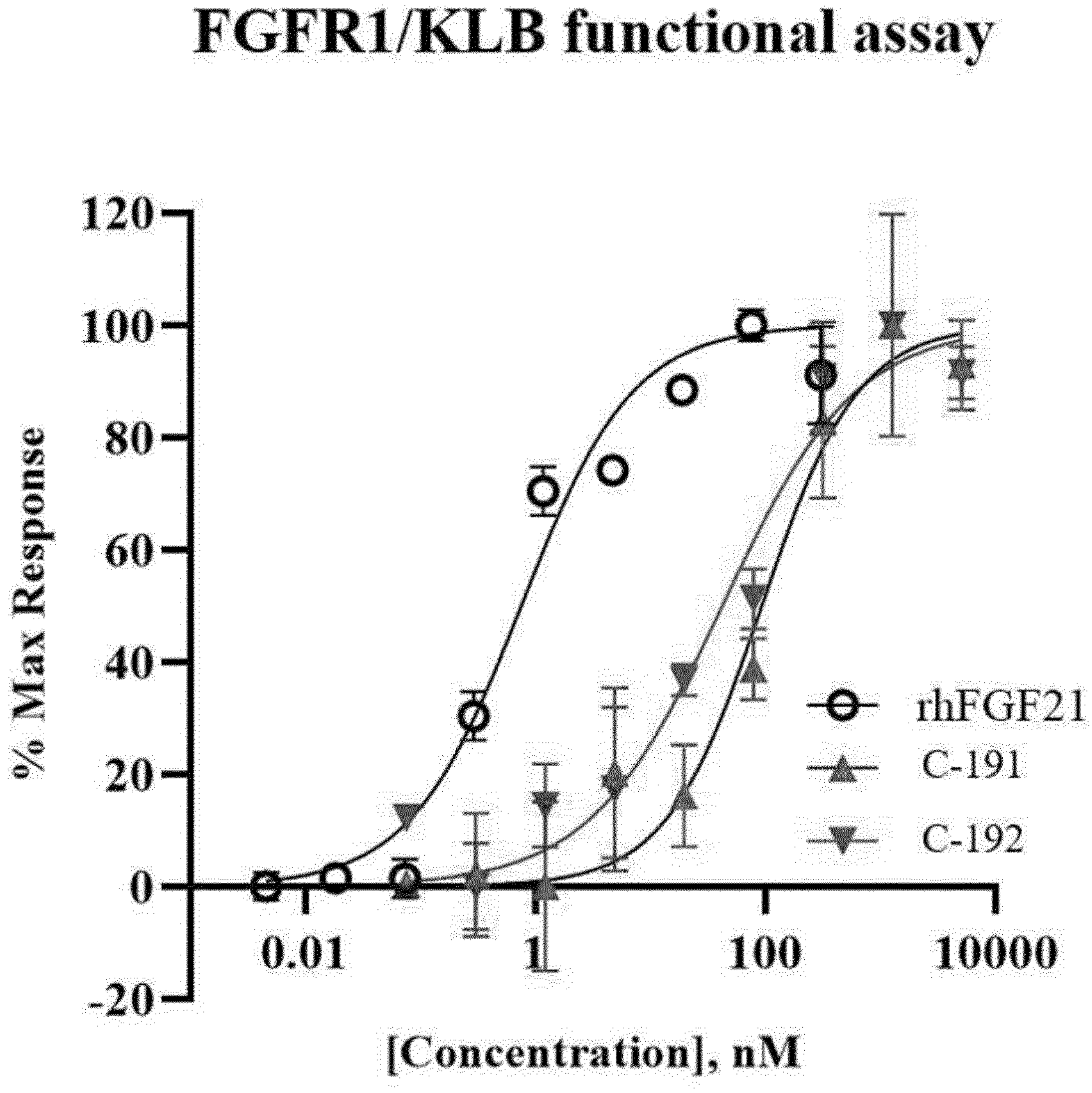
FGFR1/KLB functional assay
% Max Response
120
100
80
60
40
20
0
-20
0.01
1
100
10000
[Concentration], nM
rhFGF21
C-191
C-192

【Fig. 20】

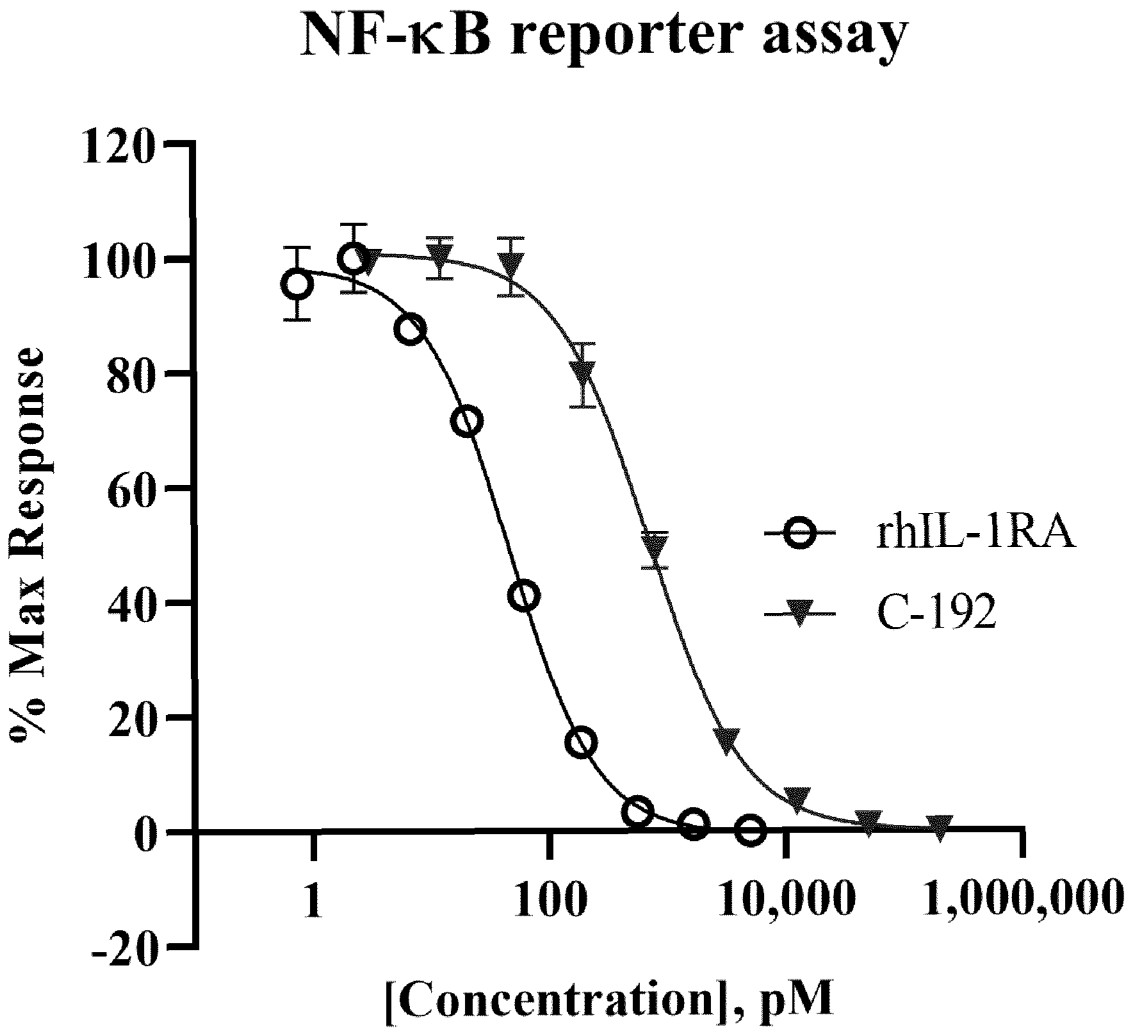
NF-κB reporter assay
120
100
80
60
40
20
0
-20
% Max Response
1
100
10,000
1,000,000
[Concentration], pM
rhIL-1RA
C-192

【Fig. 21】

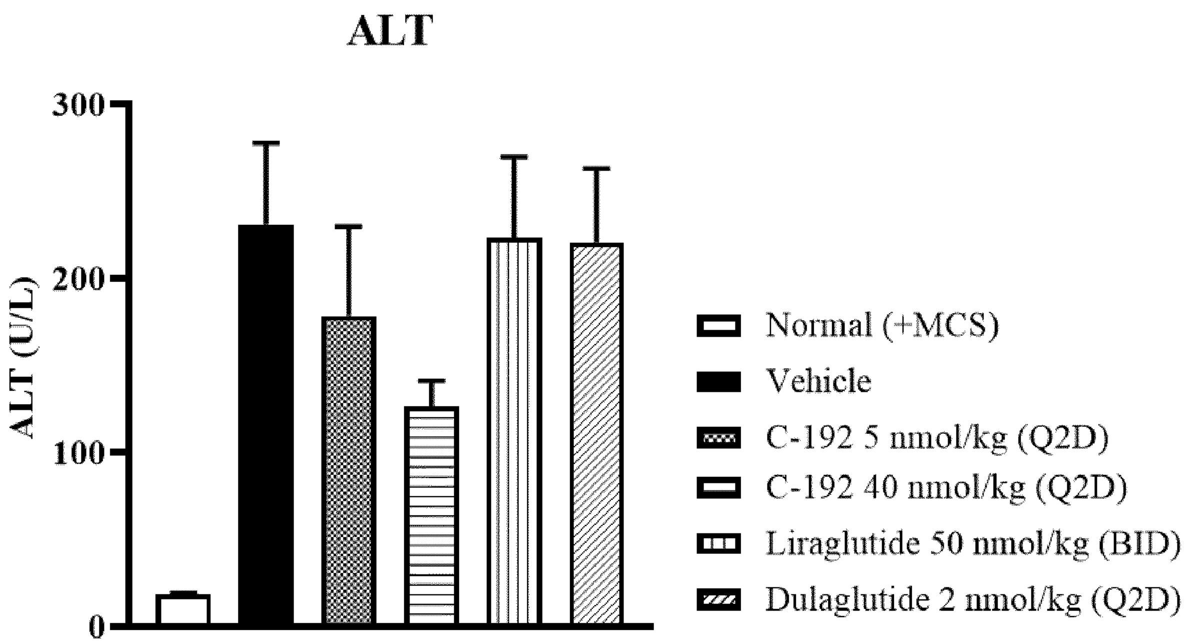
ALT
300
200
100
0
ALT (U/L)
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)

【Fig. 22】

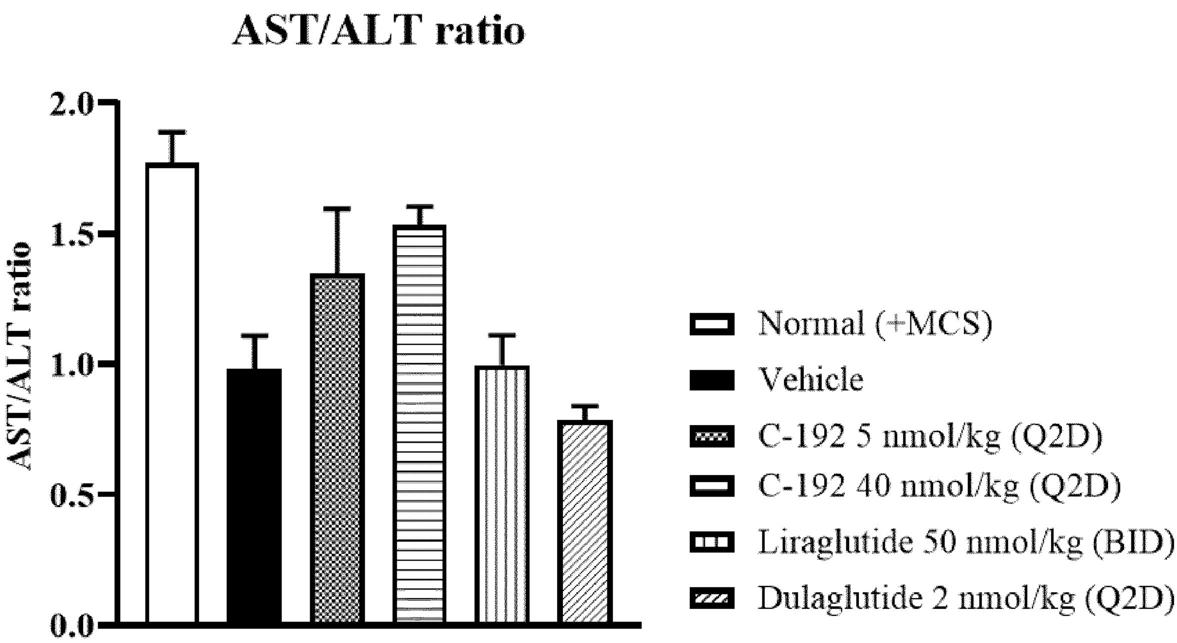
AST/ALT ratio
AST/ALT ratio
2.0
1.5
1.0
0.5
0.0
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)

【Fig. 23】

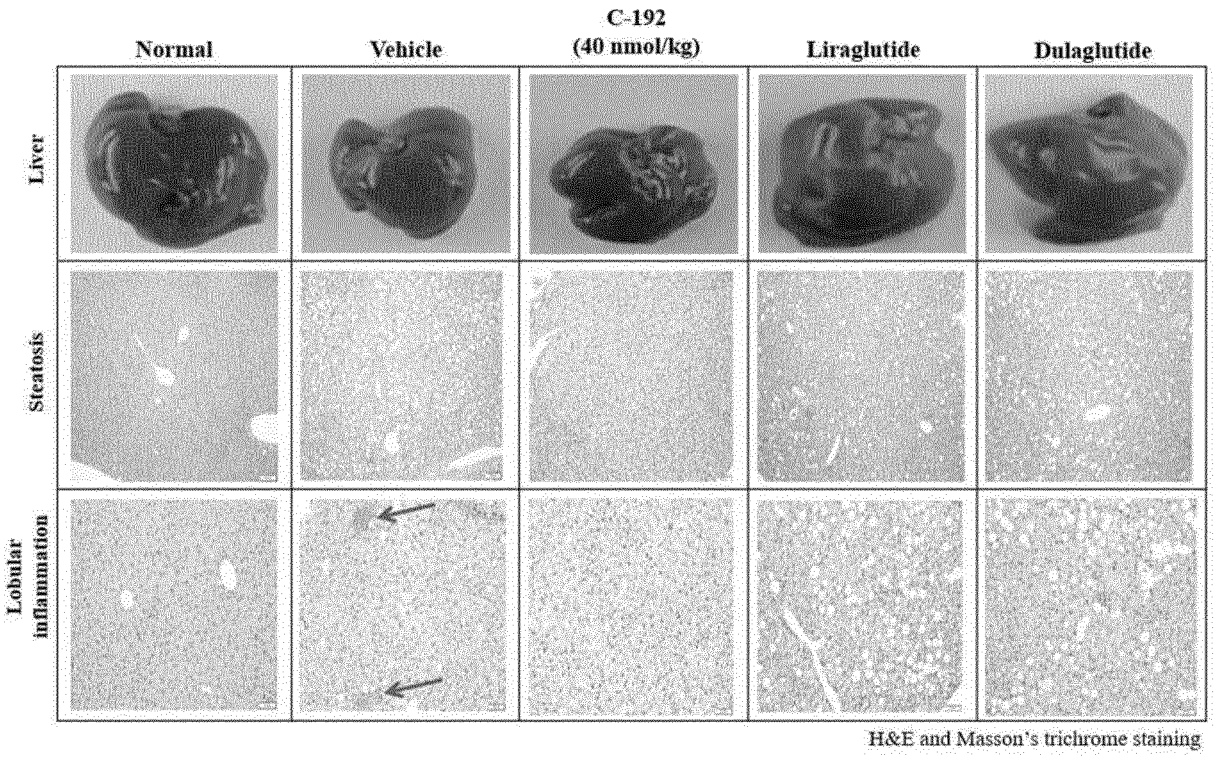
Normal
Vehicle
C-192
(40 nmol/kg)
Liraglutide
Dulaglutide
Liver
Steatosis
Lobular
inflammation
H&E and Masson's trichrome staining

【Fig. 24】

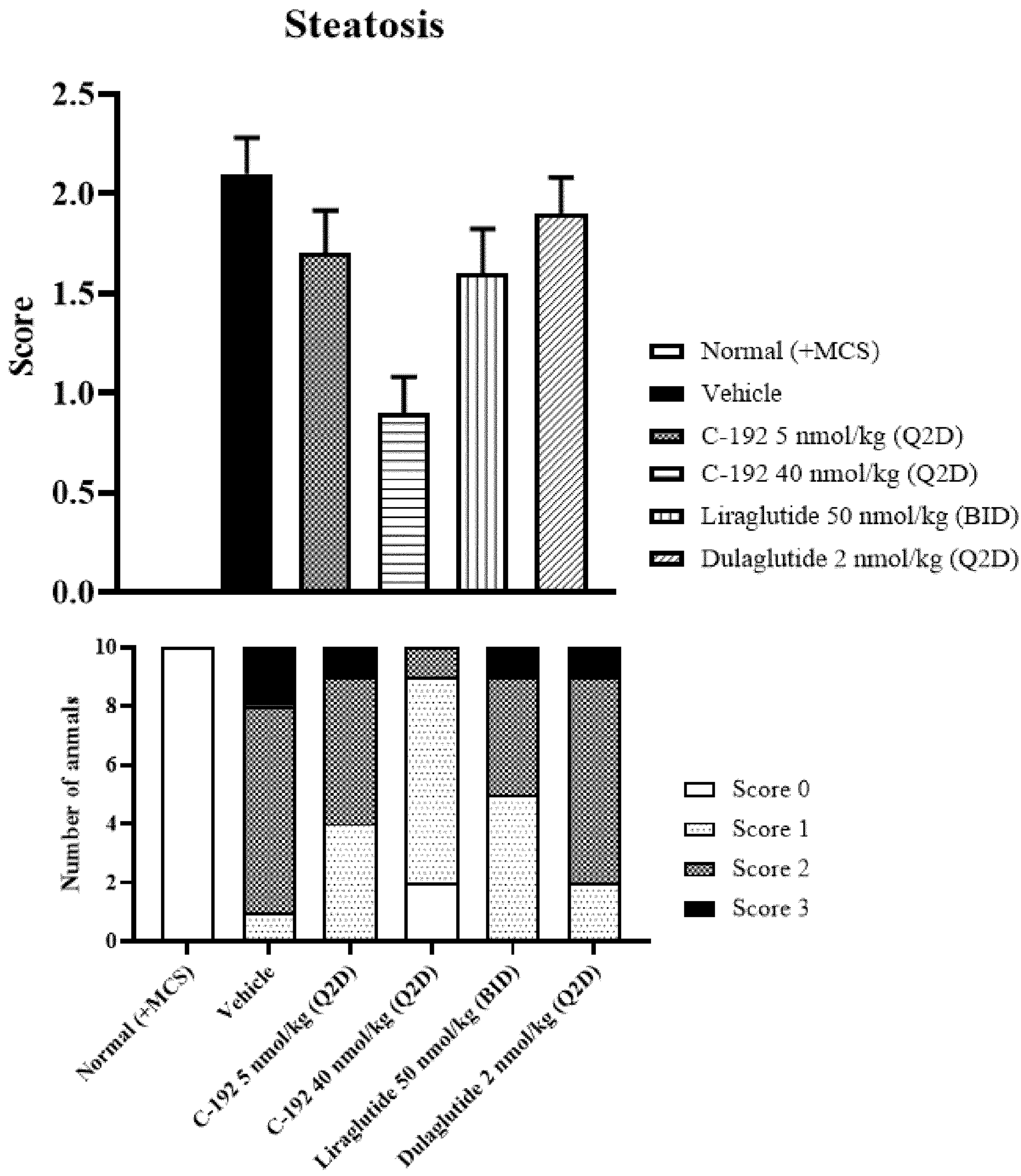
Steatosis
Score
2.5
2.0
1.5
1.0
0.5
0.0
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)
Number of animals
10
8
6
4
2
0
Score 0
Score 1
Score 2
Score 3
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)

【Fig. 25】

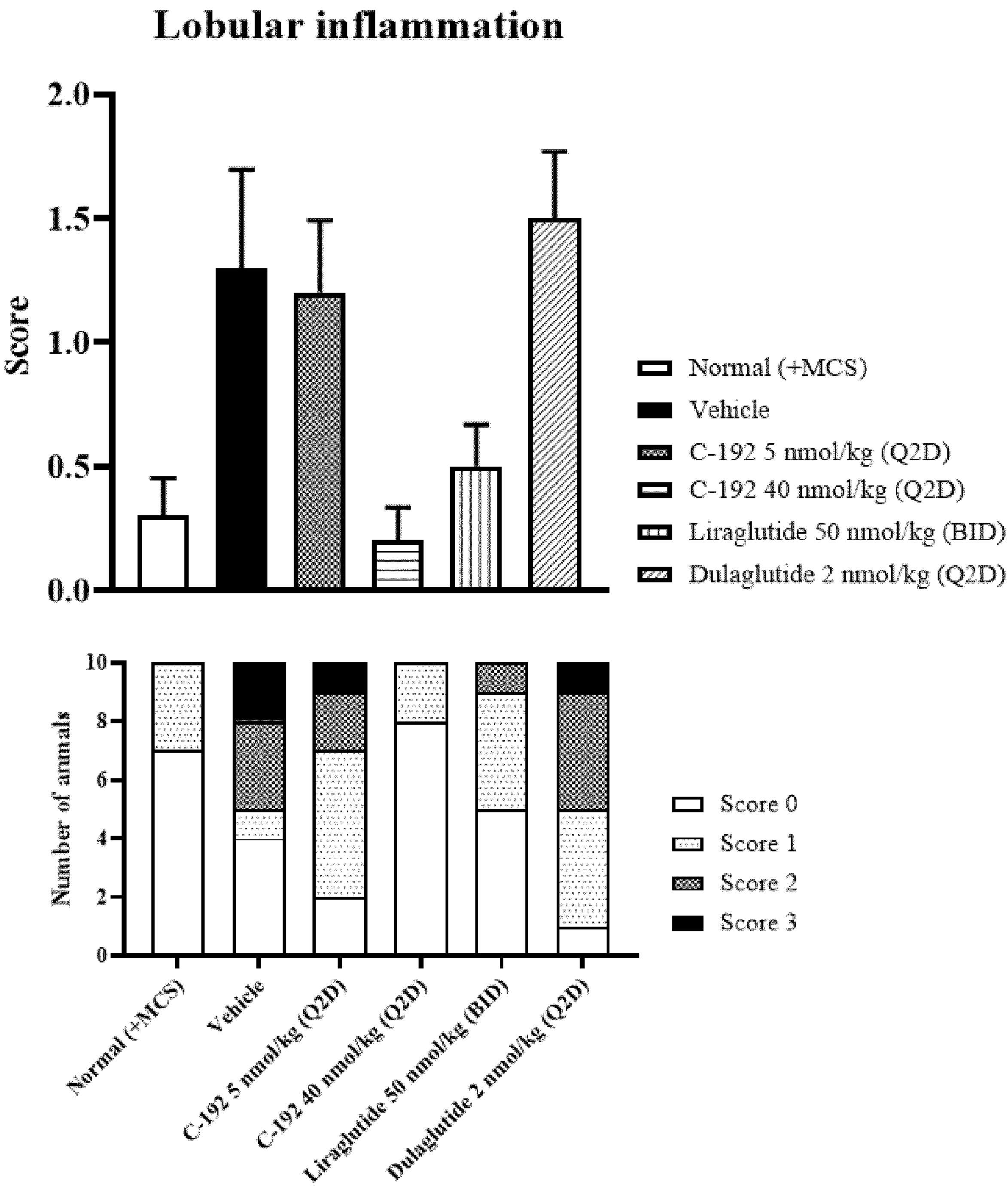
Lobular inflammation
Score
2.0
1.5
1.0
0.5
0.0
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)
Number of animals
10
8
6
4
2
0
Score 0
Score 1
Score 2
Score 3

【Fig. 26】

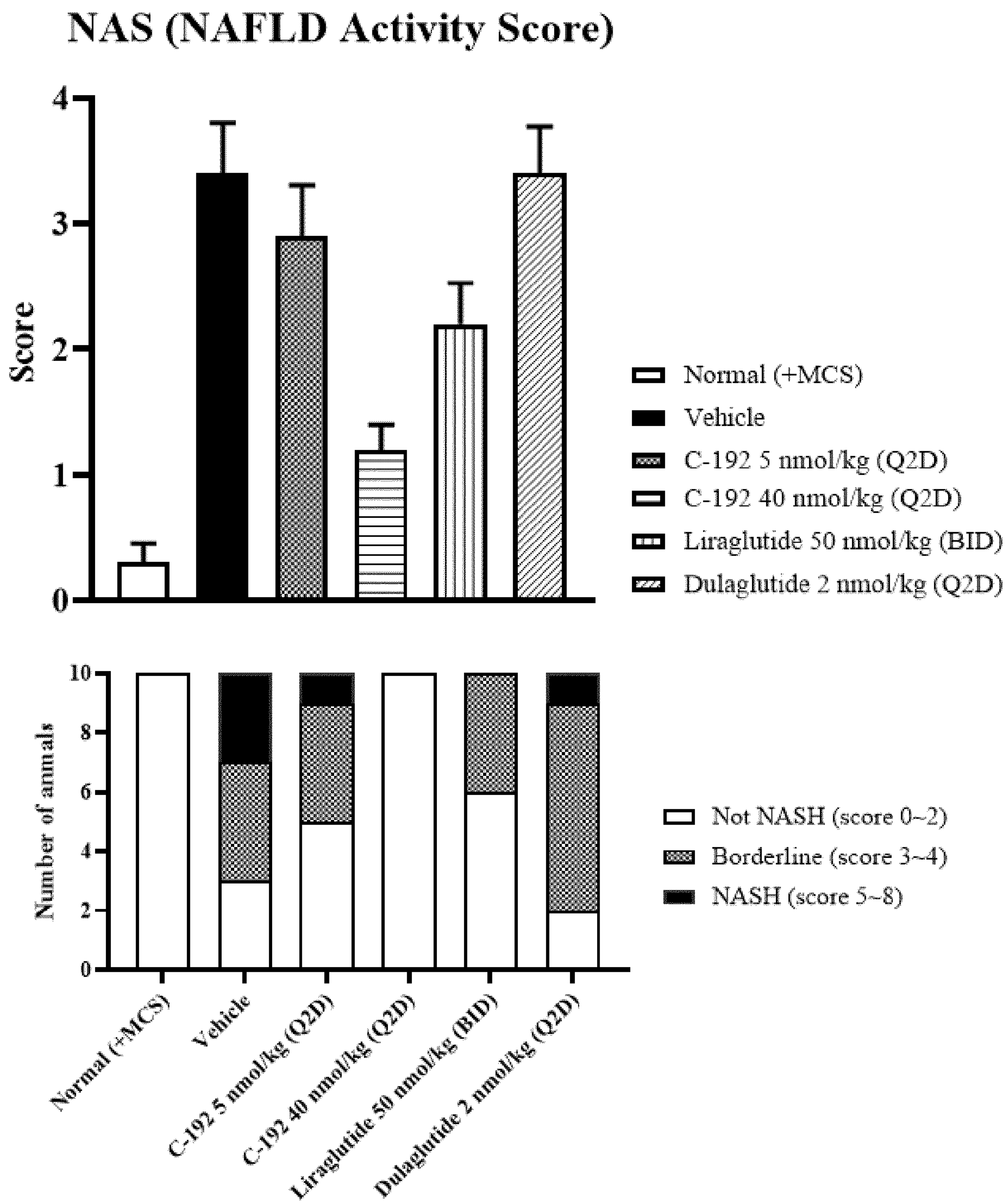
NAS (NAFLD Activity Score)
Score
0
1
2
3
4
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)
Number of animals
0
2
4
6
8
10
Not NASH (score 0~2)
Borderline (score 3~4)
NASH (score 5~8)
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)

【Fig. 27】

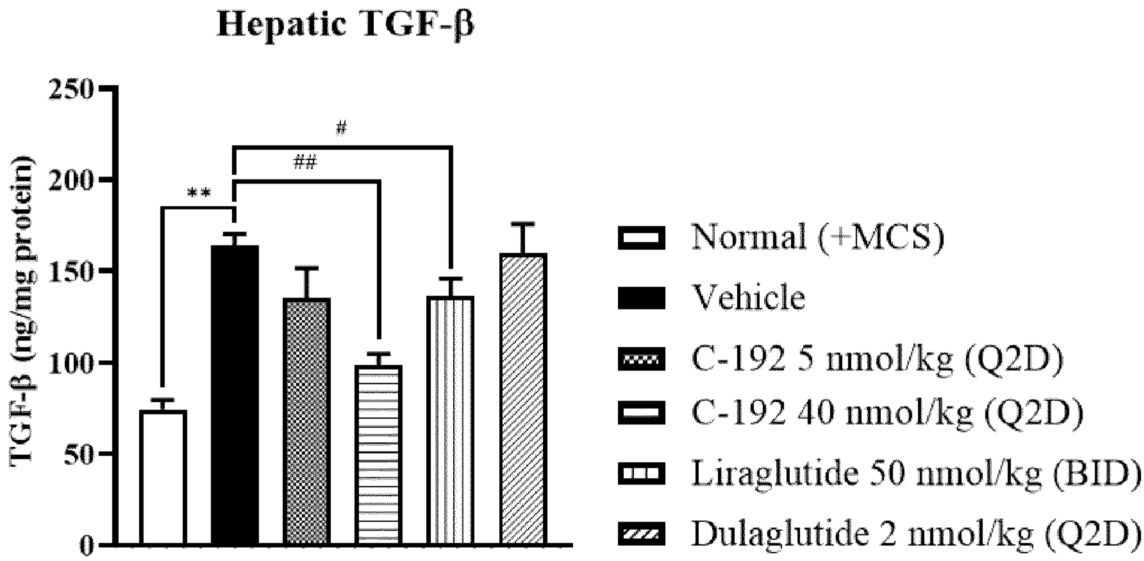


** $p < 0.01$, Significant difference from the normal control by Student t-test.
# $p < 0.05$, Significant difference from the negative control by Steel's t-test.
## $p < 0.01$, Significant difference from the negative control by Steel's t-test.

【Fig. 28】

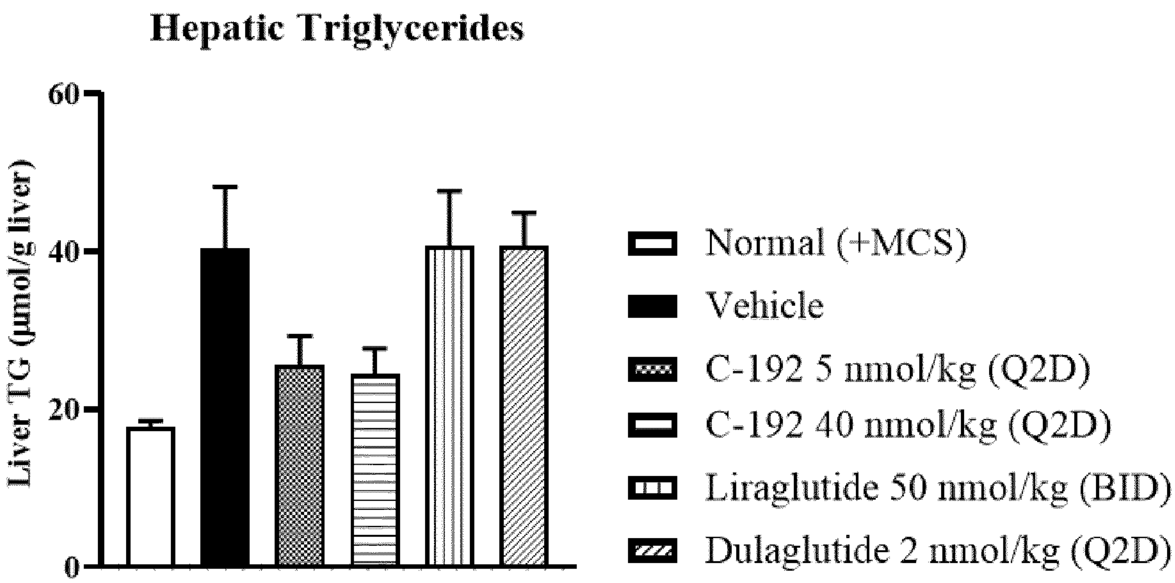
Hepatic Triglycerides
Liver TG (μmol/g liver)
60
40
20
0
Normal (+MCS)
Vehicle
C-192 5 nmol/kg (Q2D)
C-192 40 nmol/kg (Q2D)
Liraglutide 50 nmol/kg (BID)
Dulaglutide 2 nmol/kg (Q2D)

【Fig. 29】

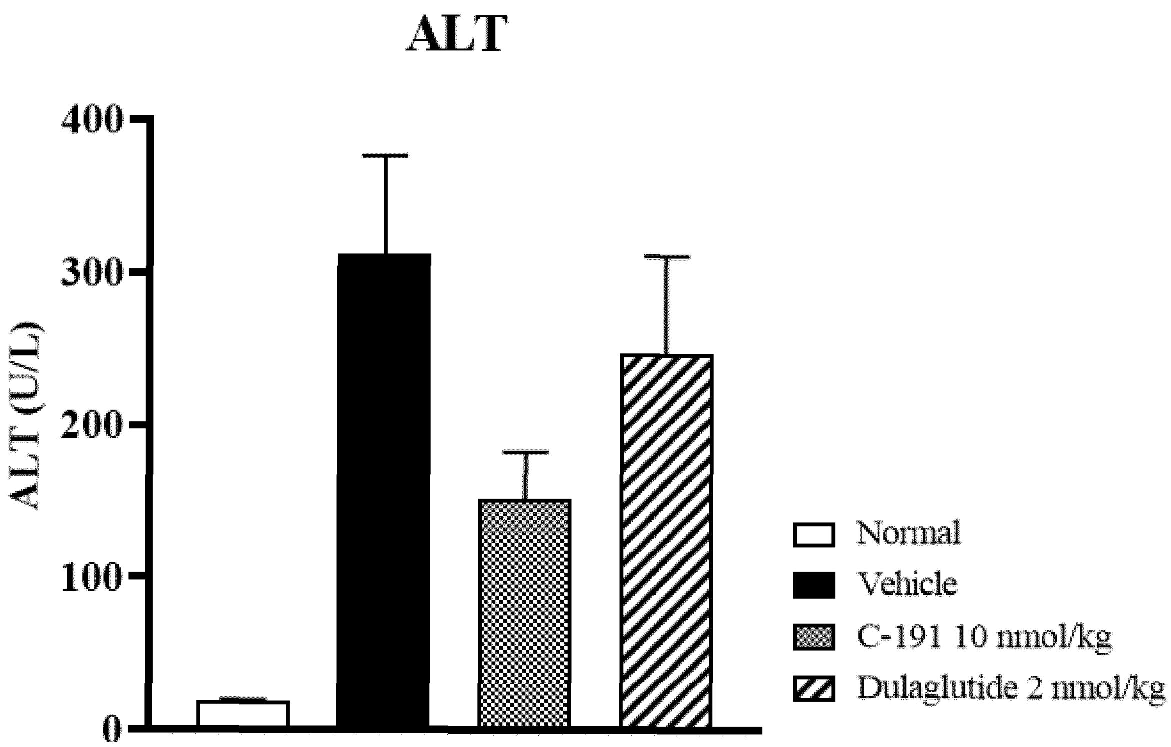
ALT
ALT (U/L)
400
300
200
100
0
Normal
Vehicle
C-191 10 nmol/kg
Dulaglutide 2 nmol/kg

【Fig. 30】

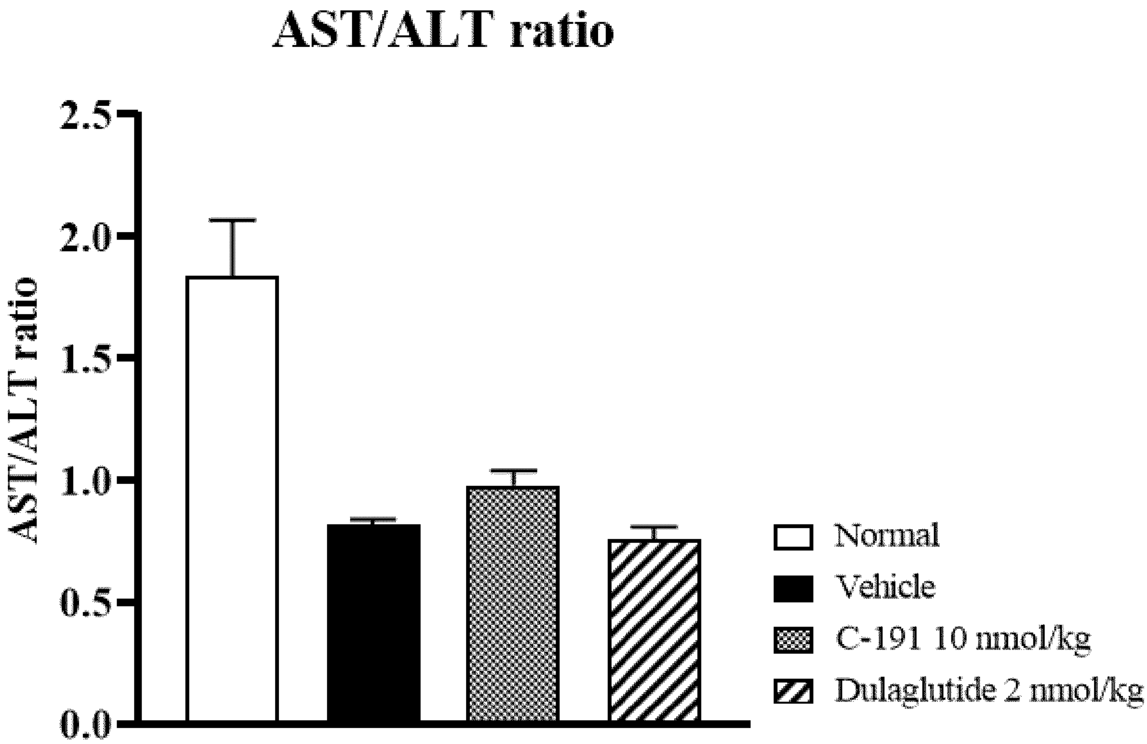
AST/ALT ratio
2.5
2.0
1.5
1.0
0.5
0.0
AST/ALT ratio
Normal
Vehicle
C-191 10 nmol/kg
Dulaglutide 2 nmol/kg

【Fig. 31】

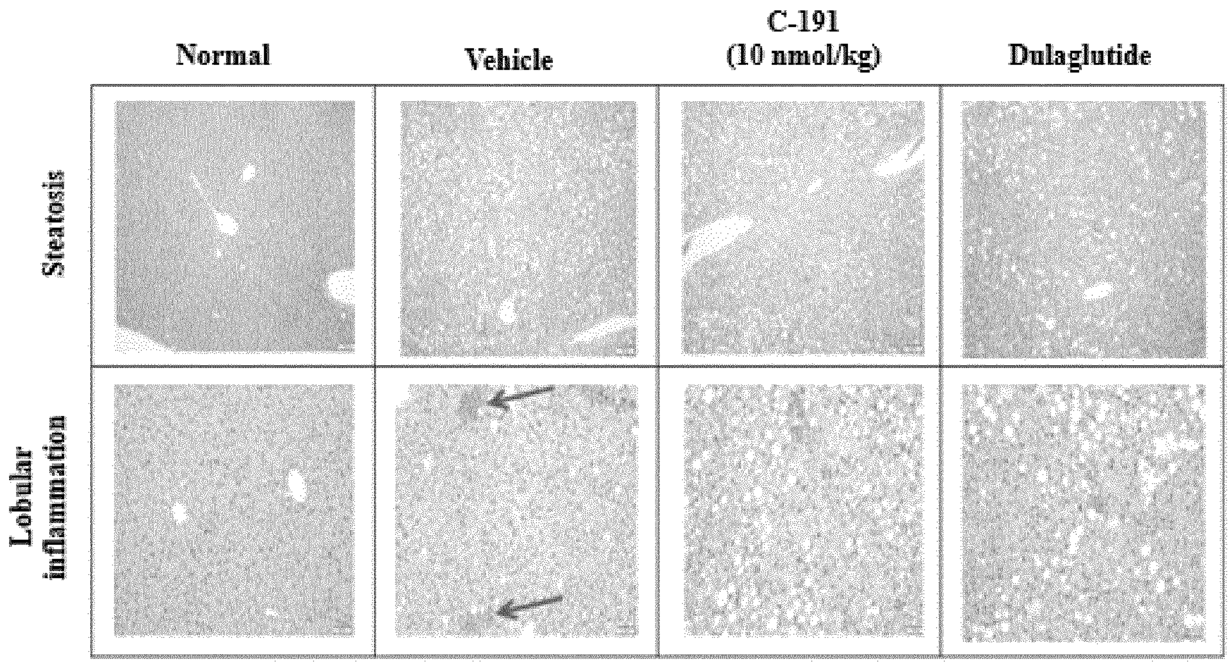
Normal
Vehicle
C-191
(10 nmol/kg)
Dulaglutide
Steatosis
Lobular
inflammation

【Fig. 32】

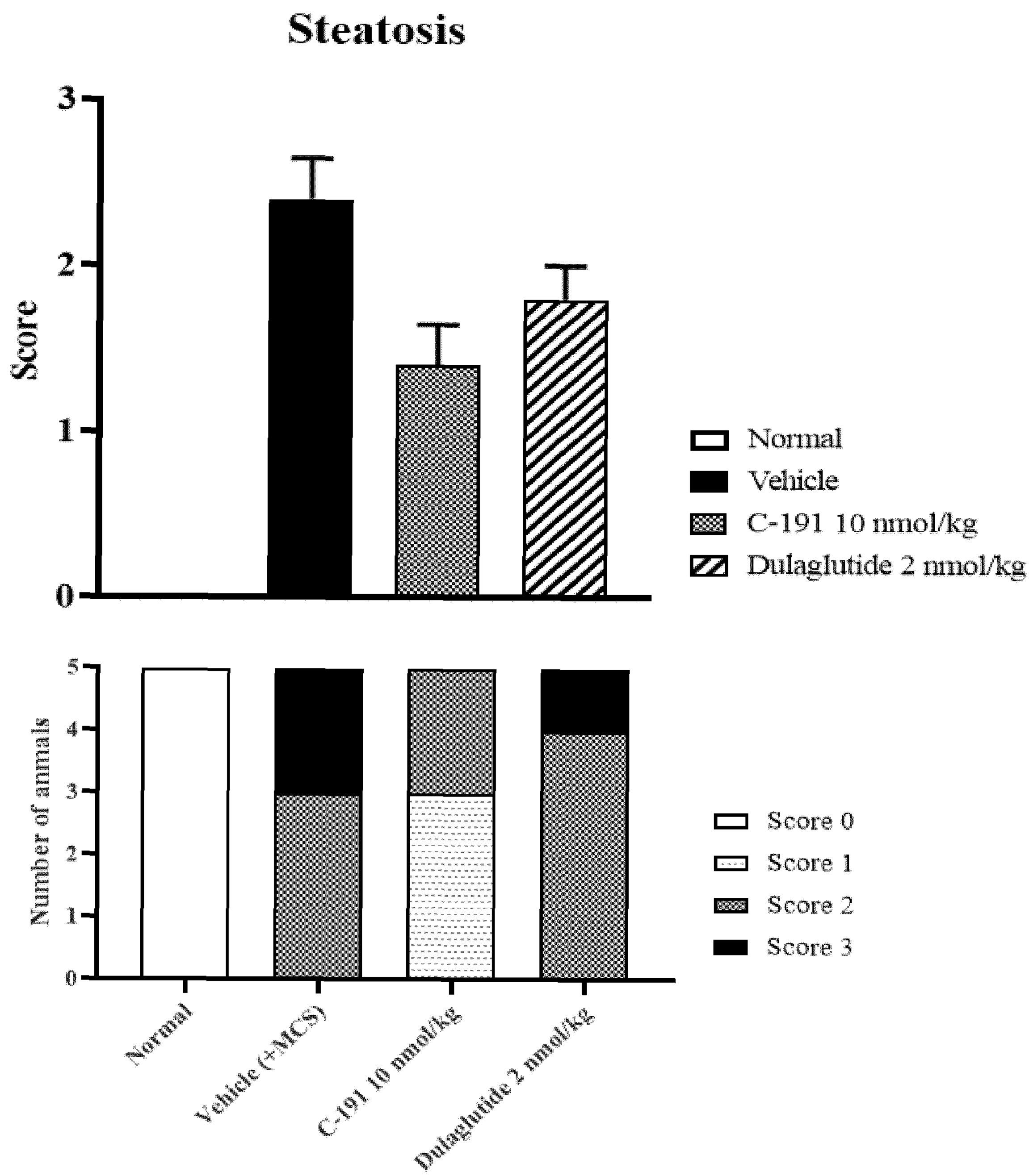
Steatosis
Score
3
2
1
0
Normal
Vehicle
C-191 10 nmol/kg
Dulaglutide 2 nmol/kg
Number of animals
5
4
3
2
1
0
Score 0
Score 1
Score 2
Score 3
Normal
Vehicle (+MCS)
C-191 10 nmol/kg
Dulaglutide 2 nmol/kg

【Fig. 33】

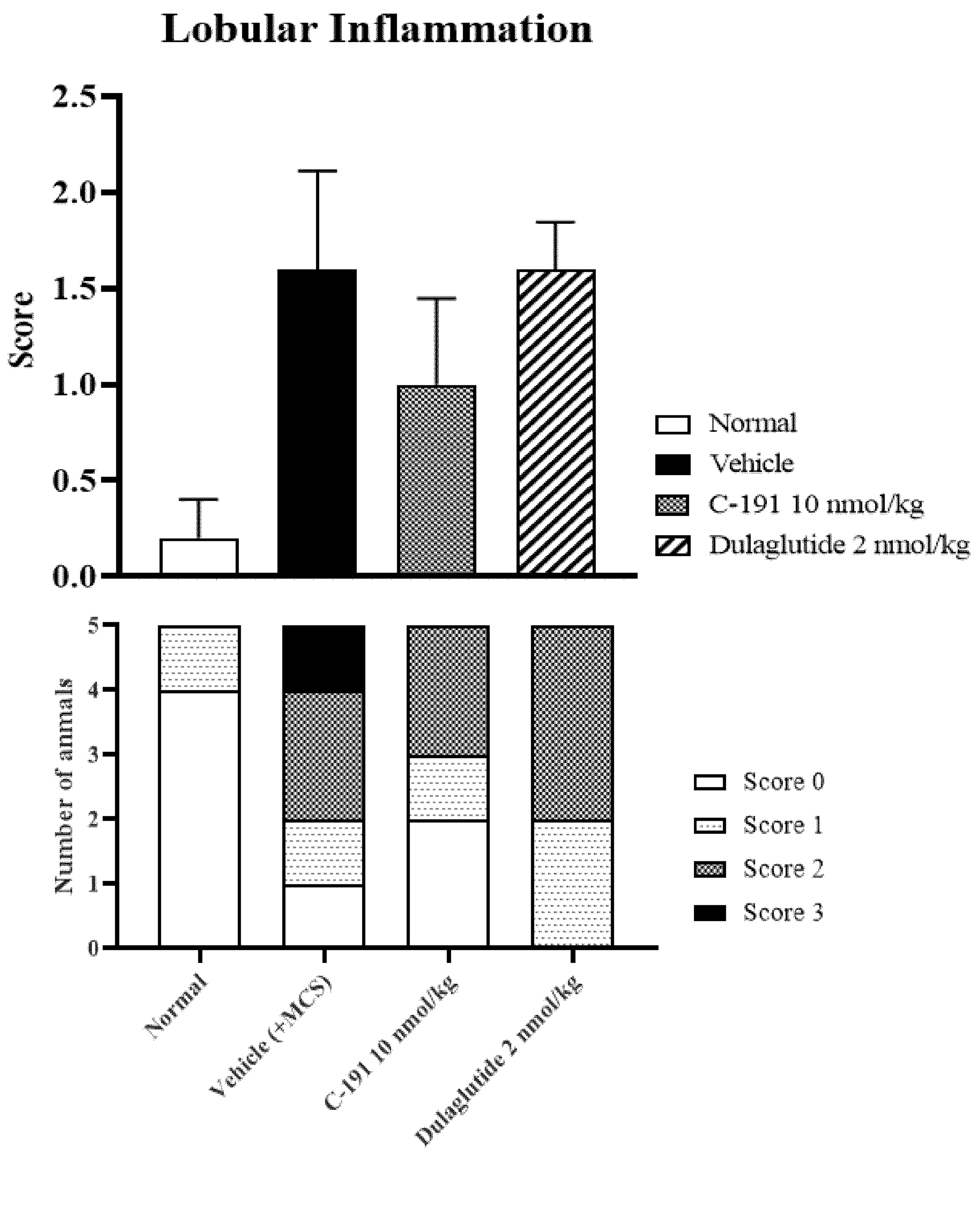
Lobular Inflammation
2.5
2.0
1.5
1.0
0.5
0.0
Score
Normal
Vehicle
C-191 10 nmol/kg
Dulaglutide 2 nmol/kg
5
4
3
2
1
0
Number of animals
Score 0
Score 1
Score 2
Score 3
Normal
Vehicle (+MCS)
C-191 10 nmol/kg
Dulaglutide 2 nmol/kg

【Fig. 34】

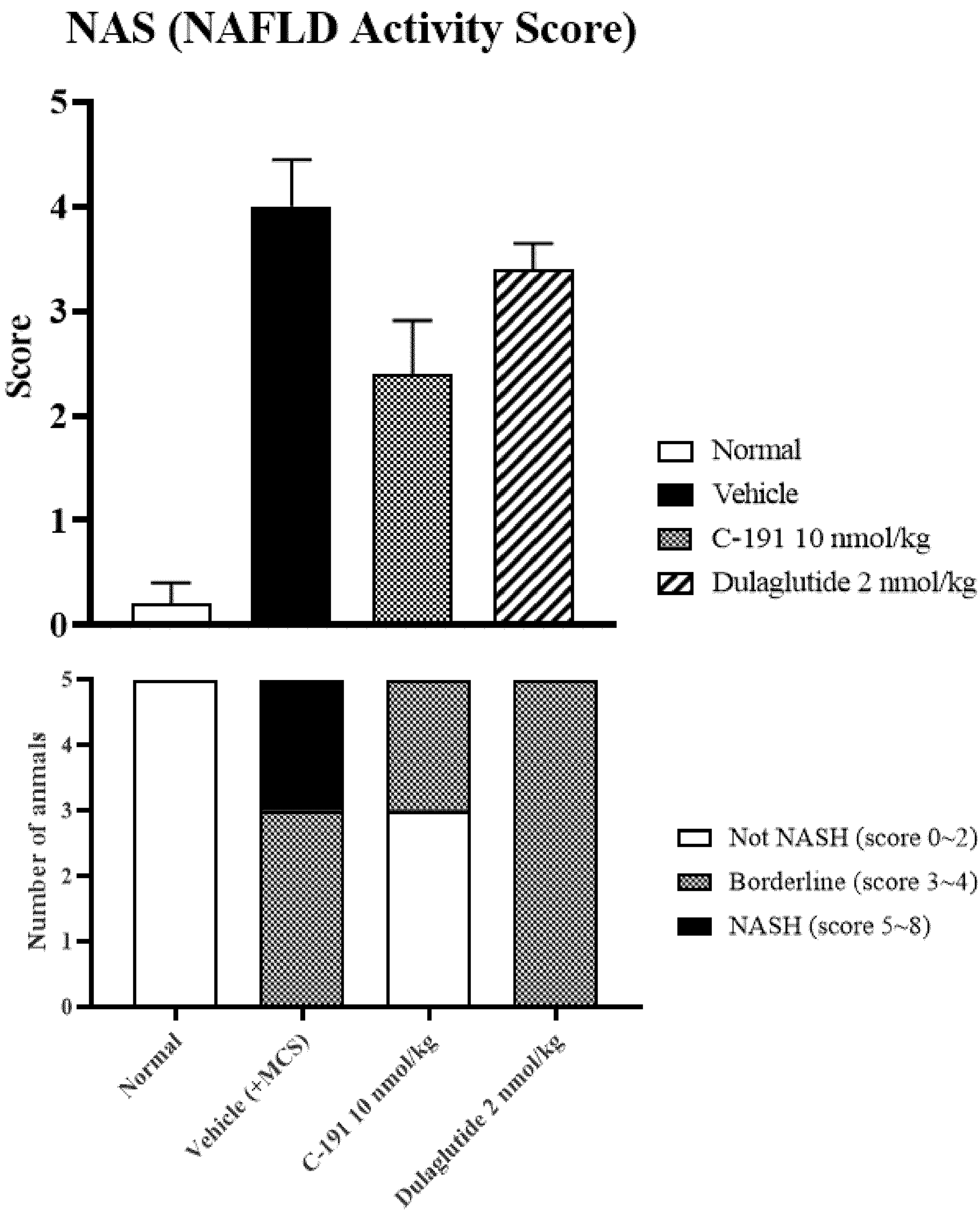
NAS (NAFLD Activity Score)
Score
5
4
3
2
1
0
Normal
Vehicle
C-191 10 nmol/kg
Dulaglutide 2 nmol/kg
Number of animals
Not NASH (score 0~2)
Borderline (score 3~4)
NASH (score 5~8)
Normal
Vehicle (+MCS)
C-191 10 nmol/kg
Dulaglutide 2 nmol/kg

【Fig. 35】

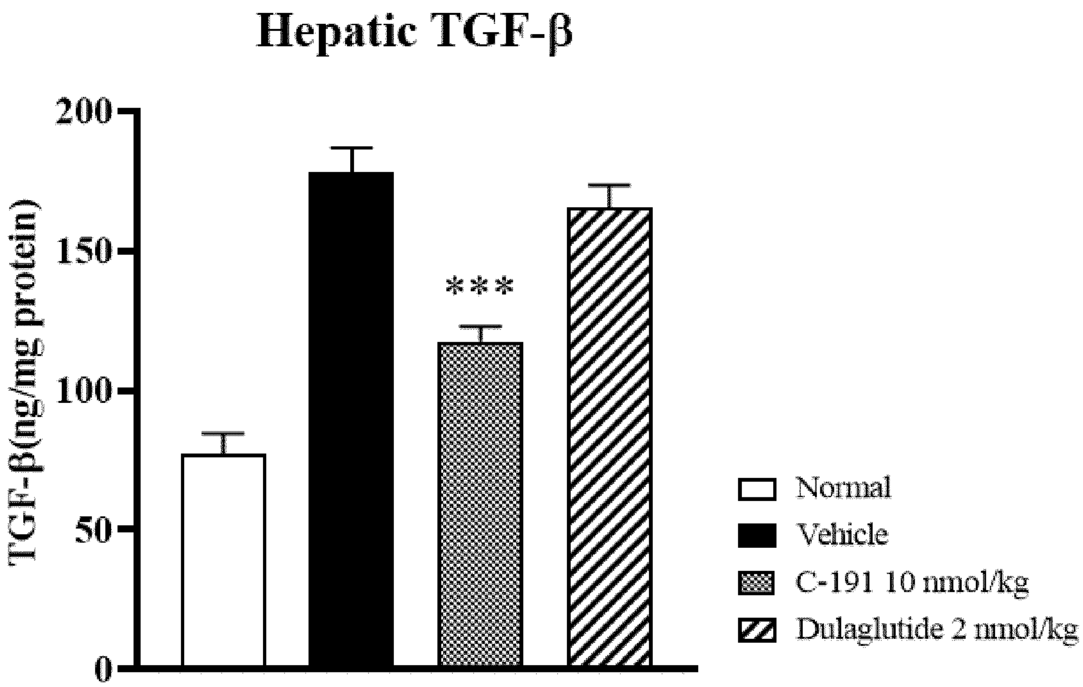


Data were expressed as mean ± SEM. One-way repeated measurement ANOVA and post-hoc Dunnett's test. ***$p < 0.001$, compared to vehicle group.

【Fig. 36】

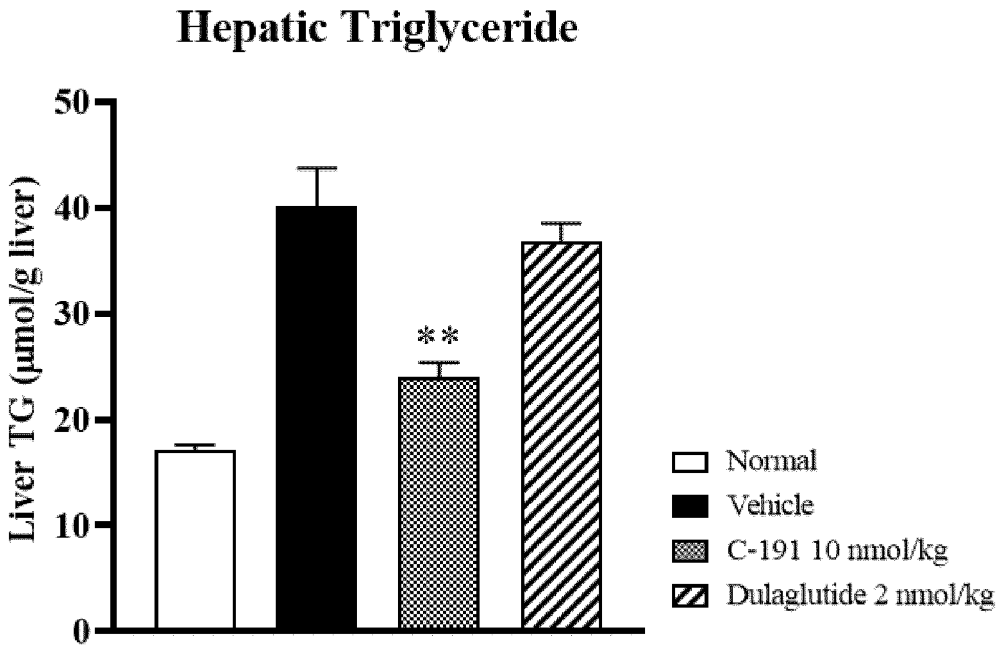


Data were expressed as mean ± SEM. One-way repeated measurement ANOVA and post-hoc Dunnett's test. **$p < 0.05$, compared to vehicle group.

【Fig. 37】

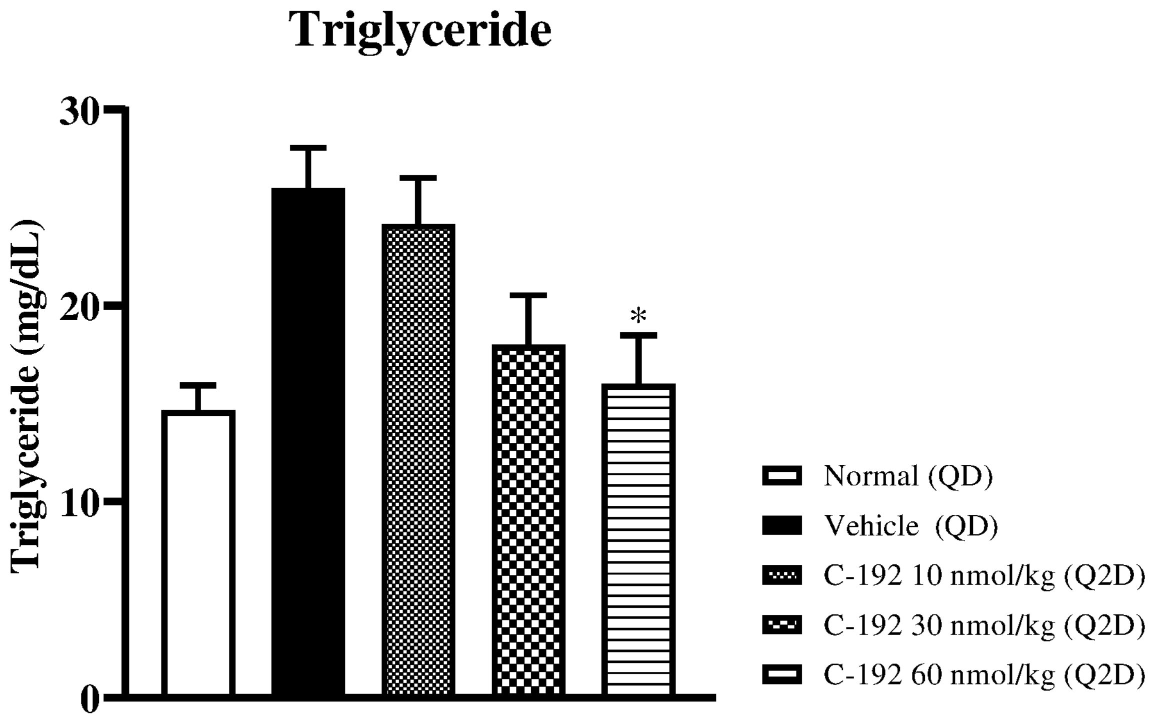


Data were expressed as mean ± SEM of tyiglyceride. One-way ANOVA and post-hoc Dunnett's test. *$p < 0.05$, compared to vehicle group. n = 6.

【Fig. 38】

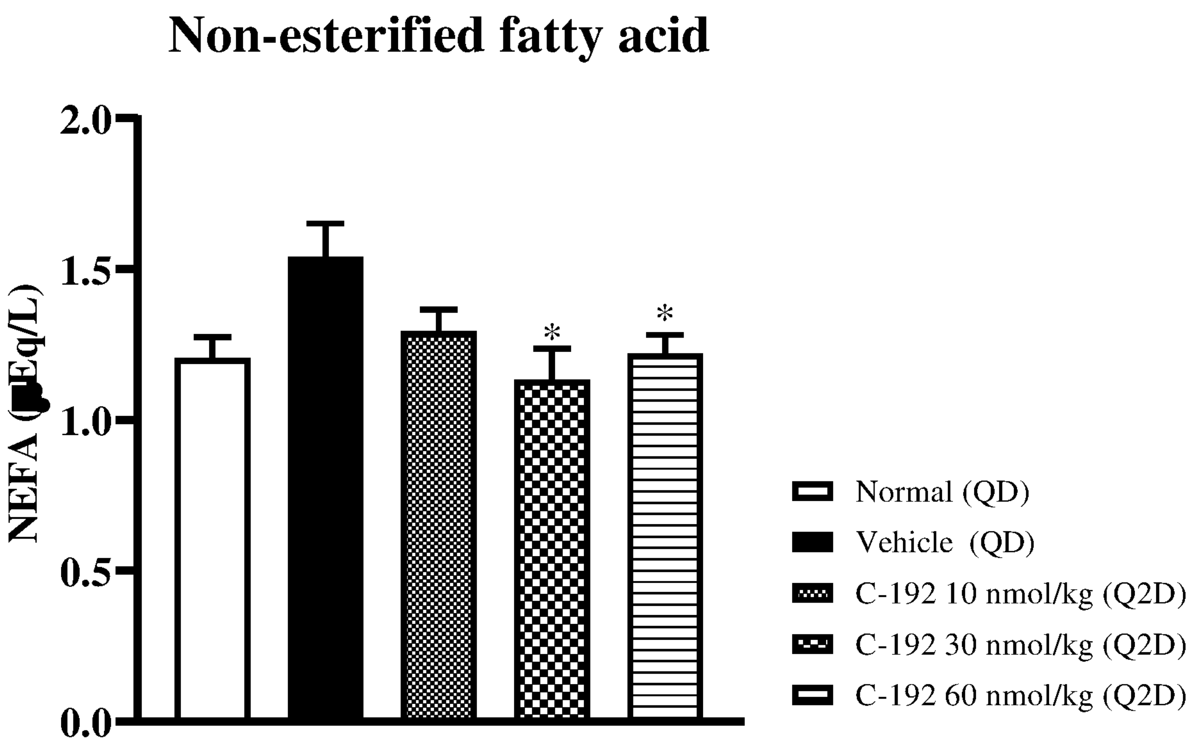


Data were expressed as mean ± SEM of non-esterified fatty acid. One-way ANOVA and post-hoc Dunnett's test. *$p < 0.05$, compared to vehicle group. n = 6.

【Fig. 39】

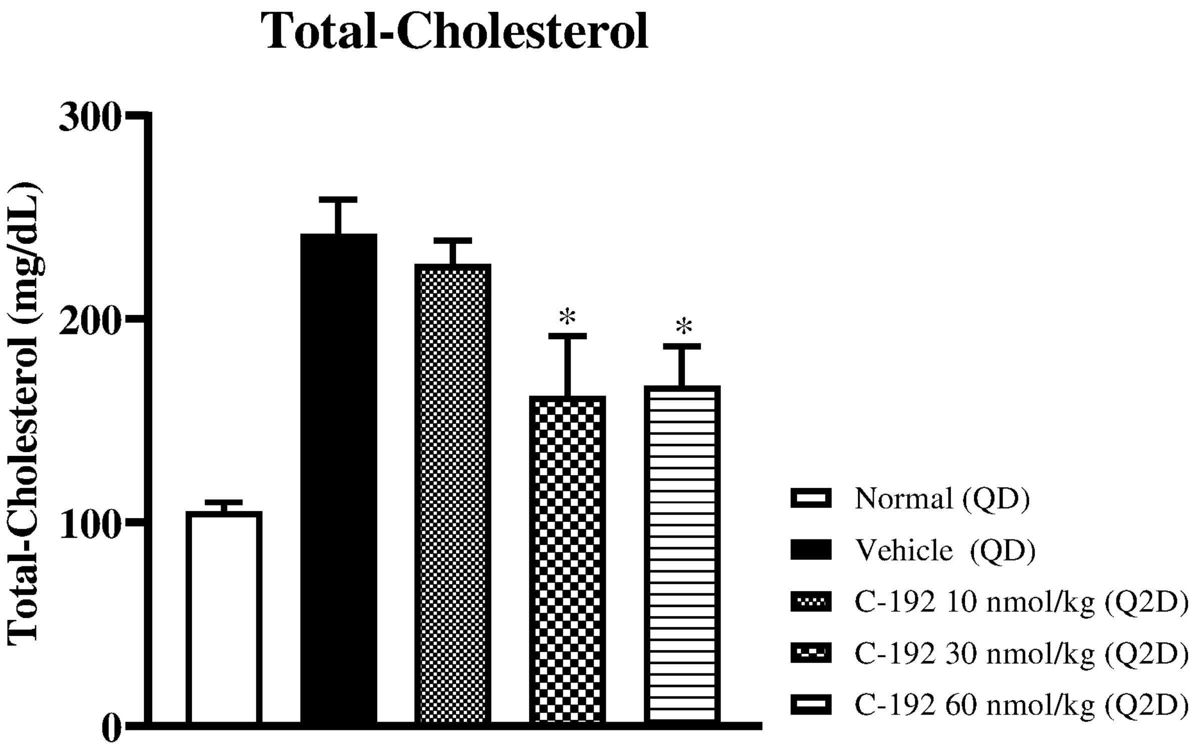


Data were expressed as mean ± SEM of total-cholesterol. One-way ANOVA and post-hoc Dunnett's test. *$p < 0.05$, compared to vehicle group. $n = 6$.

【Fig. 40】

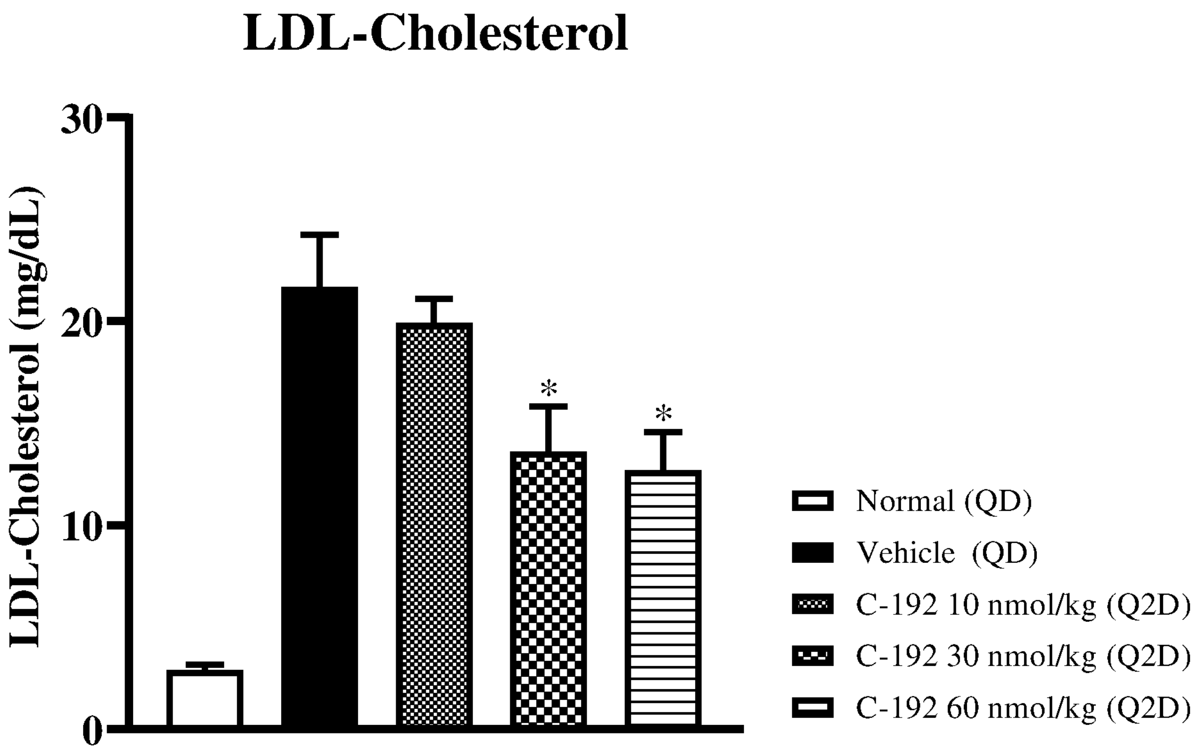


Data were expressed as mean ± SEM of LDL-cholesterol. One-way ANOVA and post-hoc Dunnett's test. *$p < 0.05$, compared to vehicle group. $n = 6$.

【Fig. 41】

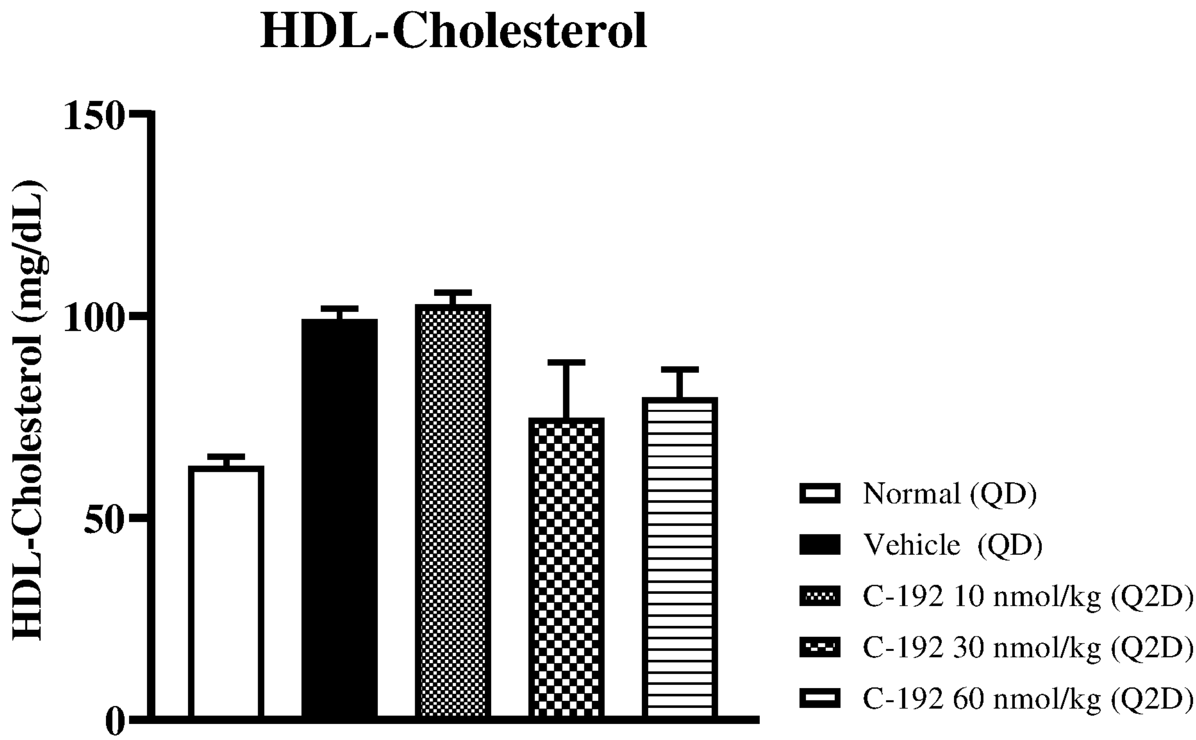


Data were expressed as mean ± SEM of HDL-cholesterol. n = 6.

【Fig. 42】

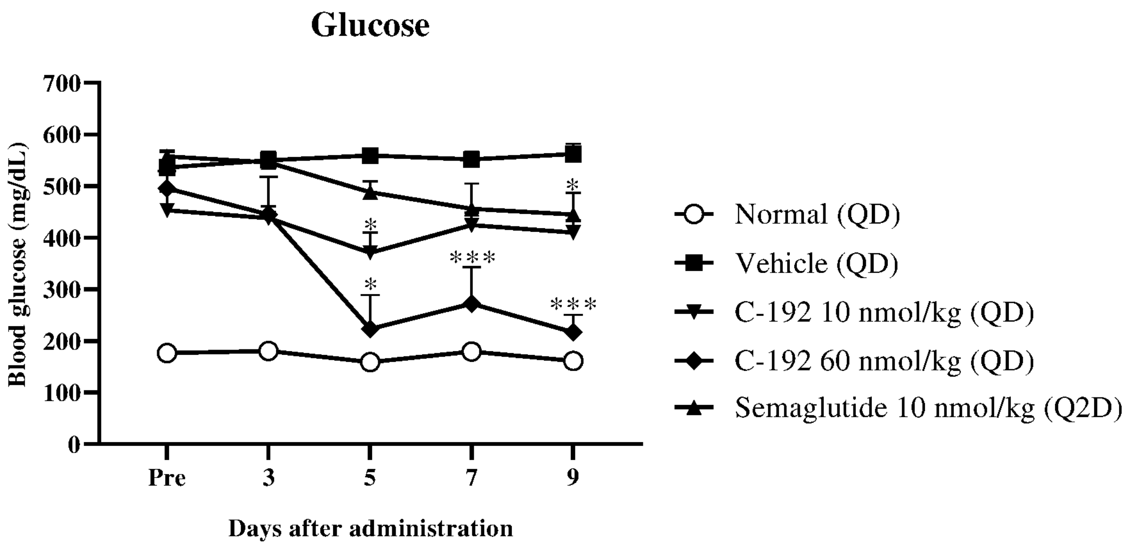


Data were expressed as mean ± SEM of blood glucose. Two-way repeated measurement ANOVA and post-hoc Dunnett's test. *$p < 0.05$, compared to vehicle group. ***$p < 0.001$, compared to vehicle group. n = 5.

【Fig. 43】

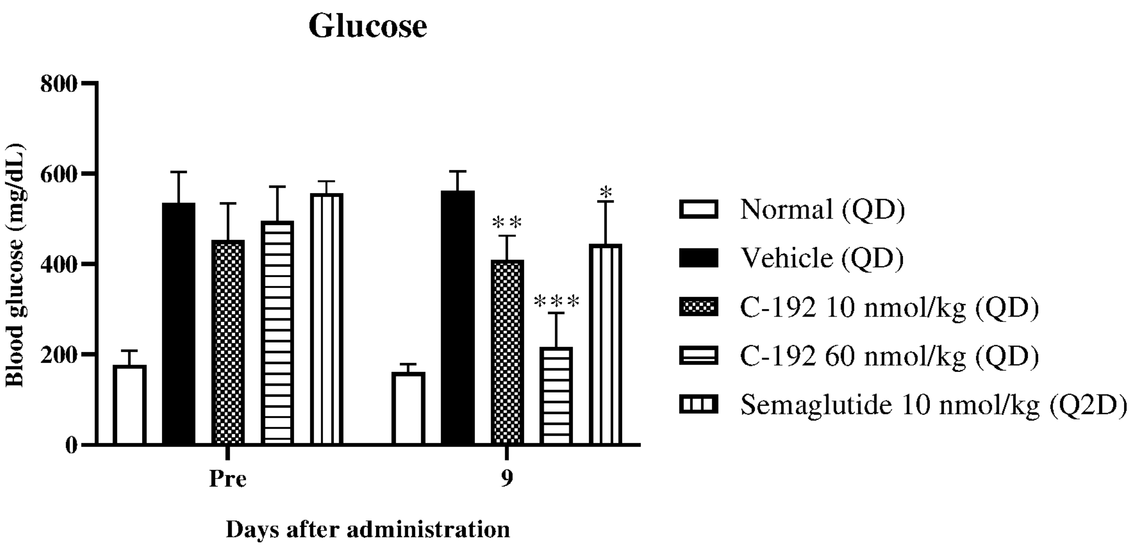


Data were expressed as mean ± SEM of blood glucose. Two-way repeated measurement ANOVA and post-hoc Dunnett's test. *$p < 0.05$, compared to vehicle group. **$p < 0.01$, compared to vehicle group. ***$p < 0.001$, compared to vehicle group. n = 5.

【Fig. 44】

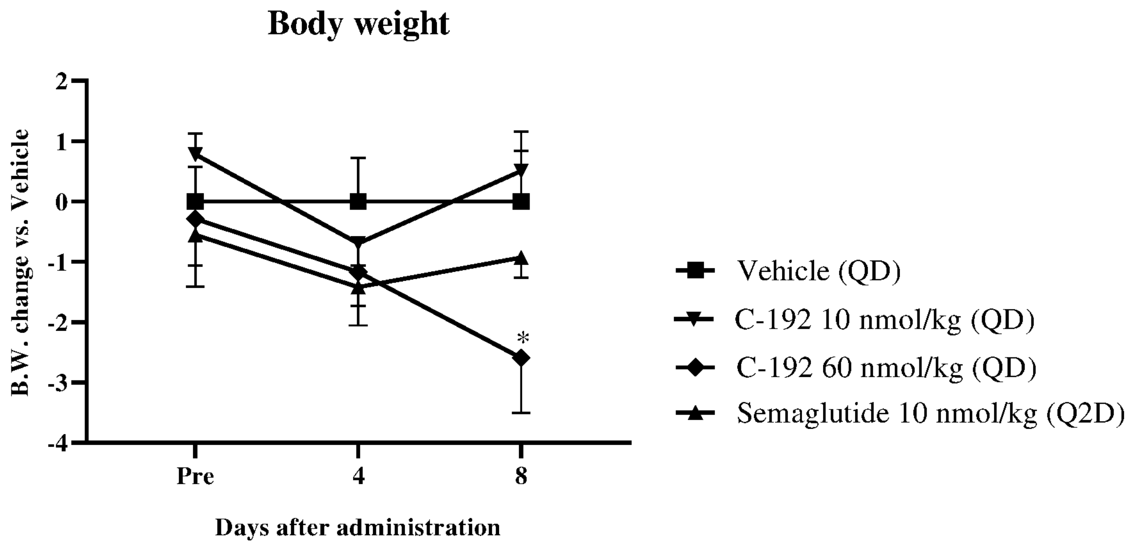


Data were expressed as mean ± SEM of body weight change verse vehicle. Two-way repeated measurement ANOVA and post-hoc Dunnett's test. *$p < 0.05$, compared to vehicle group. n = 5.

【Fig. 45】

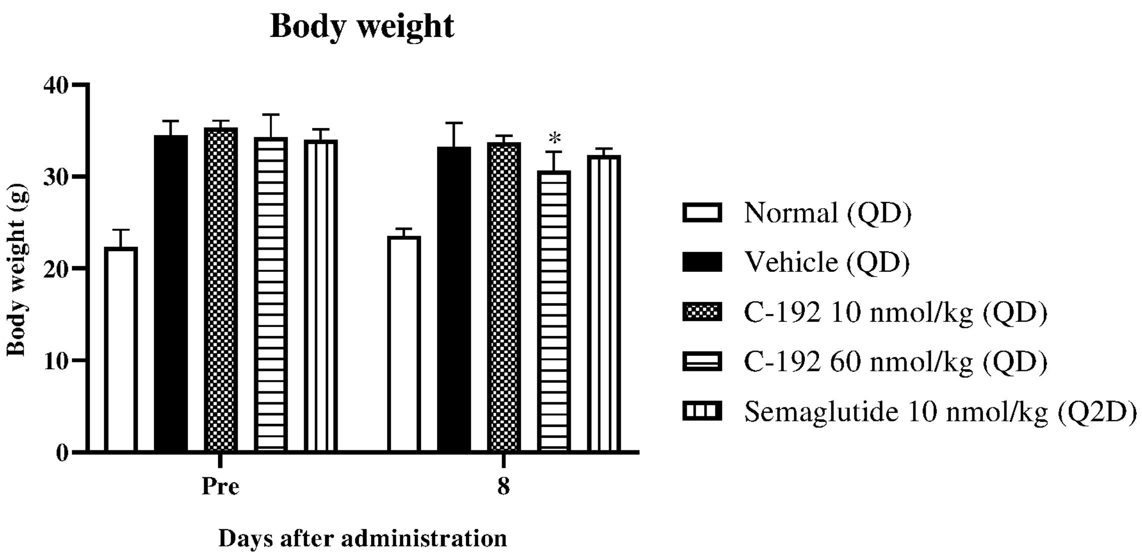


Data were expressed as mean ± SEM of body weight change verse vehicle. Two-way repeated measurement ANOVA and post-hoc Dunnett's test. *$p < 0.05$, compared to vehicle group. n = 5.

【Fig. 46】

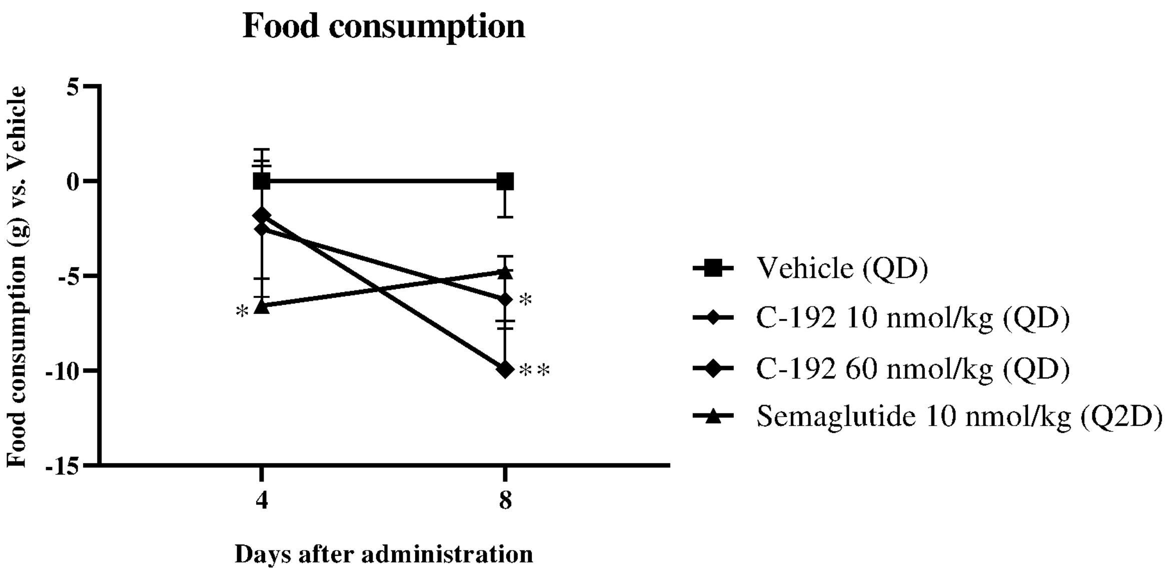


Data were expressed as mean ± SEM of food consumption. Two-way repeated measurement ANOVA and post-hoc Dunnett's test. *$p < 0.05$, compared to vehicle group. **$p < 0.01$, compared to vehicle group. n = 5.

【Fig. 47】

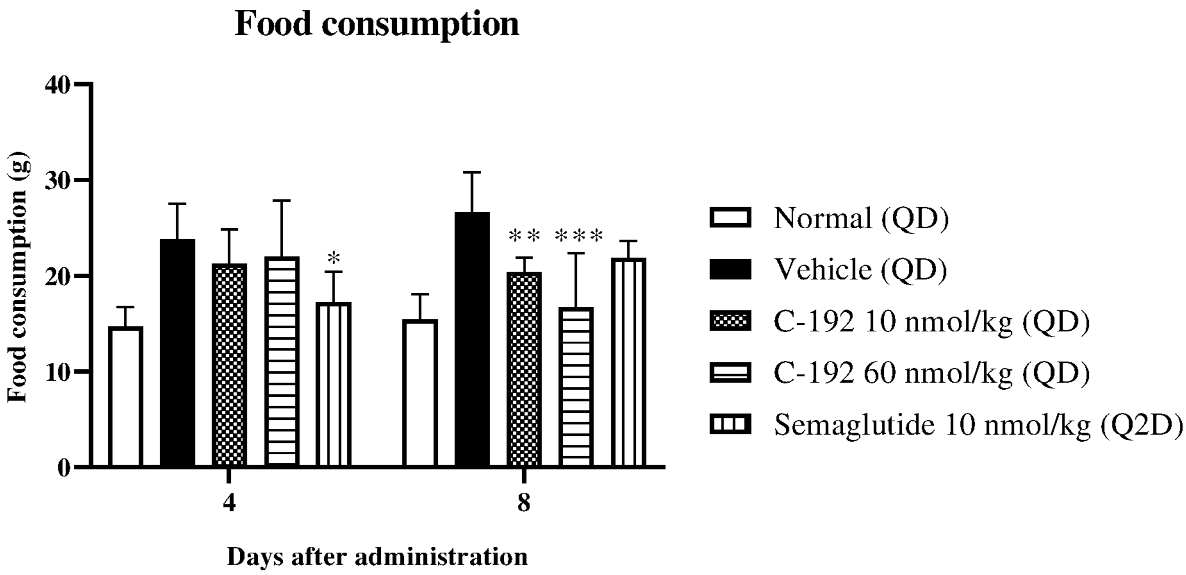


Data were expressed as mean ± SEM of food consumption. Two-way repeated measurement ANOVA and post-hoc Dunnett's test. *$p < 0.05$, compared to vehicle group. **$p < 0.01$, compared to vehicle group. ***$p < 0.001$, compared to vehicle group. n = 5.

【Fig. 48】

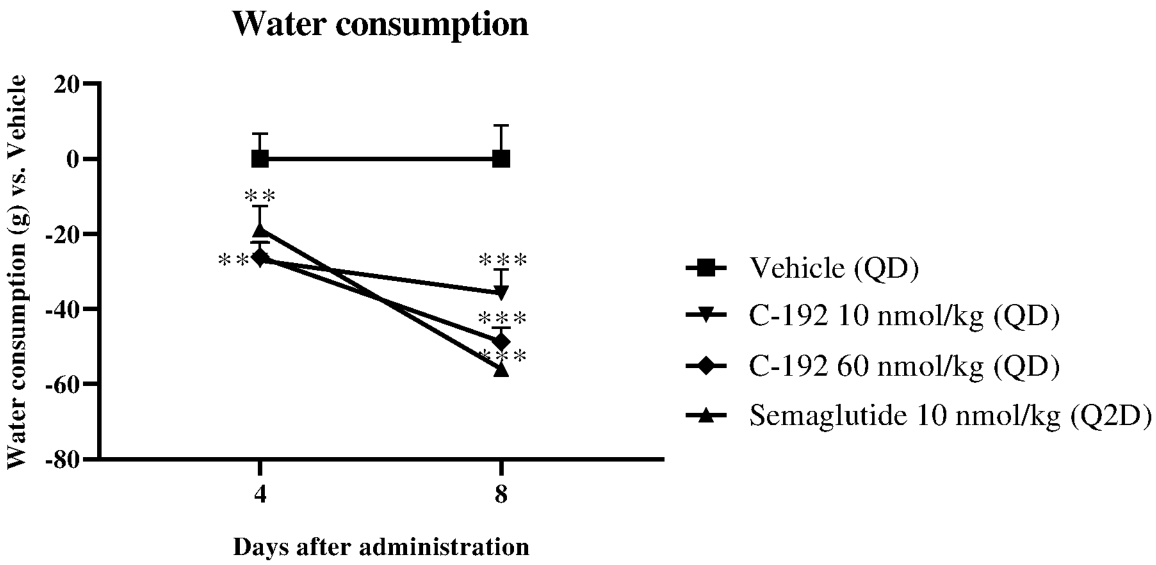


Data were expressed as mean ± SEM of water consumption. Two-way repeated measurement ANOVA and post-hoc Dunnett's test. **p < 0.01, compared to vehicle group. ***p < 0.001, compared to vehicle group. n = 5.

【Fig. 49】

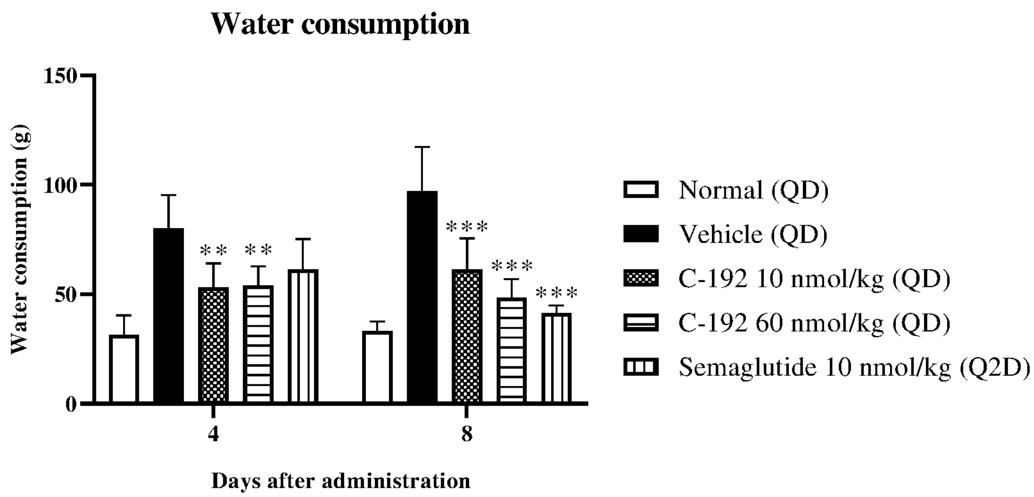


Data were expressed as mean ± SEM of water consumption. Two-way repeated measurement ANOVA and post-hoc Dunnett's test. **p < 0.01, compared to vehicle group. ***p < 0.001, compared to vehicle group. n = 5.

【Fig. 50】

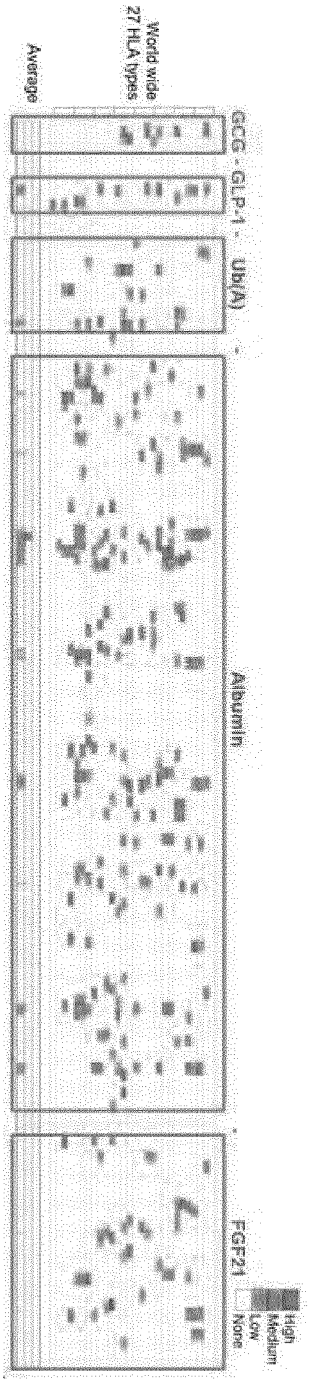
World wide
27 HLA types
Average
GCG
GLP-1
Ub(A)
Albumin
FGF21
High
Medium
Low
None

【Fig. 51】

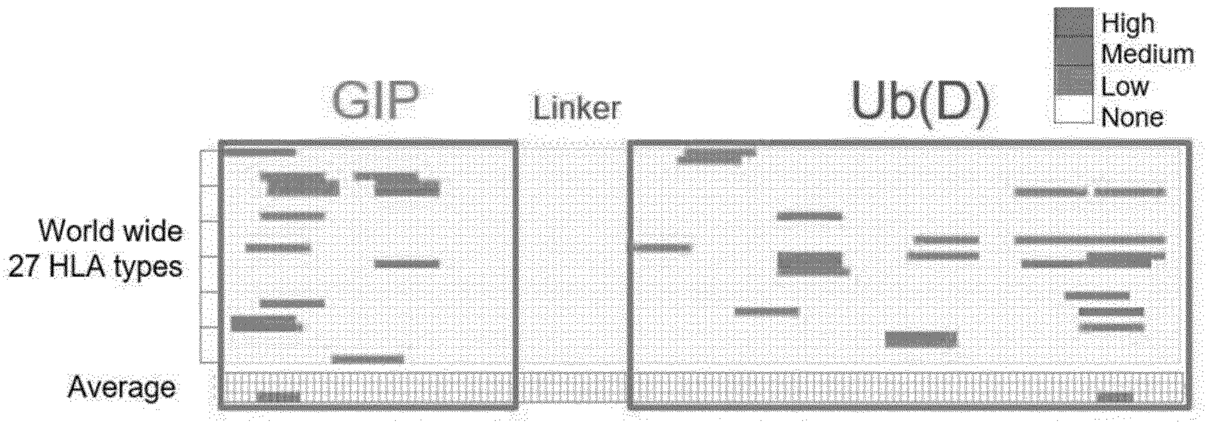

High
Medium
Low
None
GIP
Linker
Ub(D)
World wide
27 HLA types
Average

【Fig. 52】

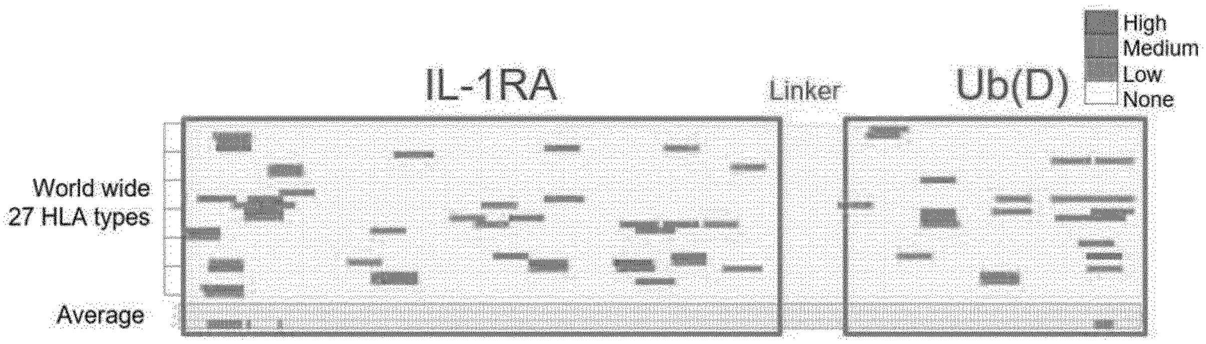
High
Medium
Low
None
IL-1RA
Linker
Ub(D)
World wide
27 HLA types
Average

【Fig. 53】

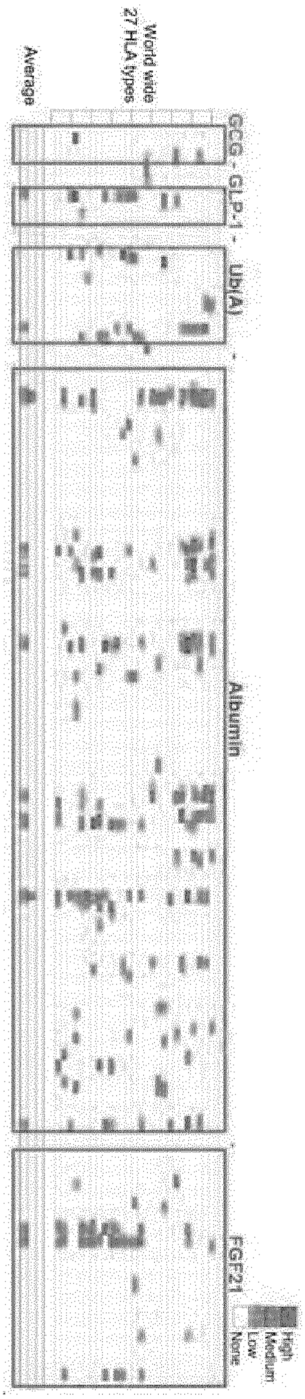
World wide
27 HLA types
Average
GCG
GLP-1
Lib(A)
Albumin
FGF21
High
Medium
Low
None

【Fig. 54】

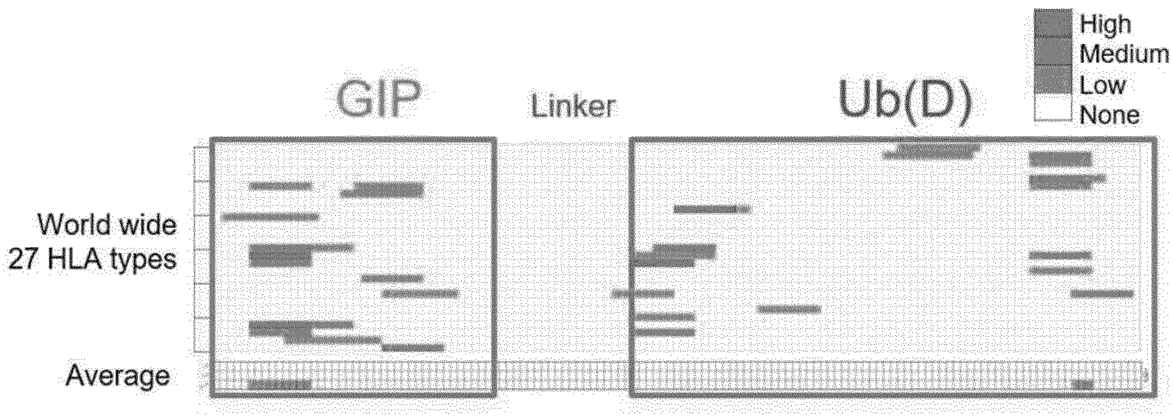
GIP
Linker
Ub(D)
High
Medium
Low
None
World wide
27 HLA types
Average

【Fig. 55】

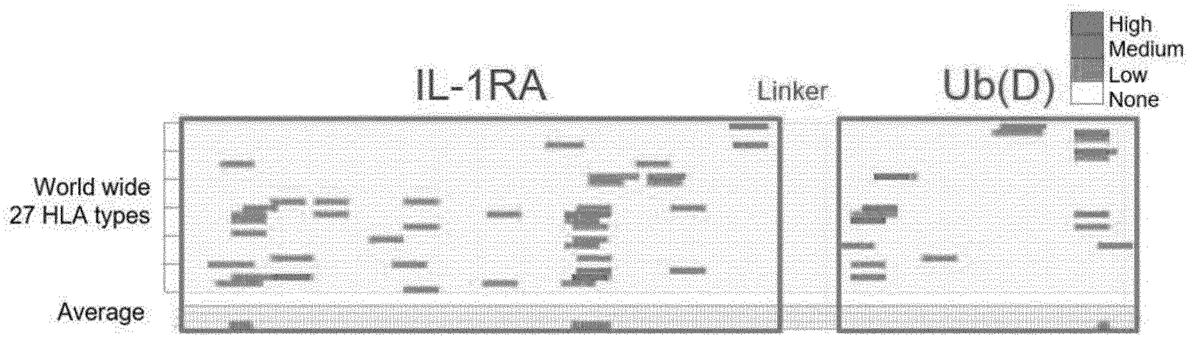
IL-1RA
Linker
Ub(D)
High
Medium
Low
None
World wide
27 HLA types
Average

## INTERNATIONAL SEARCH REPORT

International application No.
**PCT/KR2021/005481**

**A. CLASSIFICATION OF SUBJECT MATTER**

**A61K 47/64**(2017.01)i; **A61K 38/16**(2006.01)i; **A61K 38/26**(2006.01)i; **A61K 38/18**(2006.01)i; **A61K 38/22**(2006.01)i; **A61K 38/20**(2006.01)i; **A61K 47/65**(2017.01)i; **A61K 47/68**(2017.01)i; **A61K 47/60**(2017.01)i

According to International Patent Classification (IPC) or to both national classification and IPC

**B. FIELDS SEARCHED**

Minimum documentation searched (classification system followed by classification symbols)

A61K 47/64(2017.01); A61K 38/00(2006.01); A61K 38/18(2006.01); A61K 38/26(2006.01); A61P 3/04(2006.01); A61P 3/10(2006.01); C07K 1/14(2006.01); C07K 14/00(2006.01); C07K 14/47(2006.01); C07K 14/50(2006.01); C07K 14/605(2006.01); C07K 17/00(2006.01); G01N 33/48(2006.01); G01N 33/50(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리유비퀴틴(polyubiquitin), 링커(linker), 캐리어(carrier), 생체분자(biomolecule), 라이신(lysine), 아르기닌(arginine), 치환(substitution)

**C. DOCUMENTS CONSIDERED TO BE RELEVANT**

| Category* | Citation of document, with indication, where appropriate, of the relevant passages | Relevant to claim No. |
|---|---|---|
| A | KR 10-2019-0139841 A (ONEGENE BIOTECHNOLOGY INC) 18 December 2019 (2019-12-18) See claims 16-18 and 22. | 1-26 |
| A | KR 10-2014-0142293 A (GENENTECH, INC.) 11 December 2014 (2014-12-11) See paragraphs [0135]-[0139]. | 1-26 |
| A | US 8680049 B2 (MEIER, E. et al.) 25 March 2014 (2014-03-25) See claim 1; and SEQ ID NO: 4. | 1-26 |
| A | US 8263554 B2 (TATARKIEWICZ, K. et al.) 11 September 2012 (2012-09-11) See column 7, lines 14-47; and SEQ ID NO: 29. | 1-26 |
| A | US 8454971 B2 (DAY, J. et al.) 04 June 2013 (2013-06-04) See column 44, example 16; and SEQ ID NO: 81. | 1-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 September 2021** | **08 September 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No. **PCT/KR2021/005481**

**C. DOCUMENTS CONSIDERED TO BE RELEVANT**

| Category* | Citation of document, with indication, where appropriate, of the relevant passages | Relevant to claim No. |
|---|---|---|
| A | US 7491697 B2 (BEALS, J. M. et al.) 17 February 2009 (2009-02-17) See claim 1; and SEQ ID NO: 1. | 1-26 |
| A | US 9074014 B2 (DONG, Z. X.) 07 July 2015 (2015-07-07) See column 6, lines 29-34; and SEQ ID NO: 122. | 1-26 |
| A | US 7430476 B2 (CARR, F. J. et al.) 30 September 2008 (2008-09-30) See column 25; and SEQ ID NO: 46. | 1-26 |

Form PCT/ISA/210 (second sheet) (July 2019)

**INTERNATIONAL SEARCH REPORT**

International application No.
**PCT/KR2021/005481**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2021/005481**

| Patent document cited in search report | Publication date (day/month/year) | Patent family member(s) | Publication date (day/month/year) |
|---|---|---|---|
| KR 10-2019-0139841 A | 18 December 2019 | AU 2019-276622 A1 | 24 December 2020 |
| | | CA 3101185 A1 | 05 December 2019 |
| | | CN 112204046 A | 08 January 2021 |
| | | EP 3805256 A1 | 14 April 2021 |
| | | KR 10-2019-0135393 A | 06 December 2019 |
| | | KR 10-2155982 B1 | 15 September 2020 |
| | | WO 2019-231208 A1 | 05 December 2019 |
| KR 10-2014-0142293 A | 11 December 2014 | CA 2866835 A1 | 19 September 2013 |
| | | CN 104254541 A | 31 December 2014 |
| | | EP 2825549 A1 | 21 January 2015 |
| | | EP 2825549 B1 | 10 October 2018 |
| | | HK 1205524 A1 | 18 December 2015 |
| | | JP 2015-520730 A | 23 July 2015 |
| | | JP 6198807 B2 | 20 September 2017 |
| | | KR 10-2012-0014154 A | 16 February 2012 |
| | | MX 2014010943 A | 26 November 2014 |
| | | RU 2014141617 A | 10 May 2016 |
| | | US 2013-0281314 A1 | 24 October 2013 |
| | | US 9139863 B2 | 22 September 2015 |
| | | WO 2013-137920 A1 | 19 September 2013 |
| US 8680049 B2 | 25 March 2014 | AU 2008-365559 A1 | 21 July 2011 |
| | | AU 2008-365559 B2 | 25 February 2016 |
| | | BR PI0823377 A2 | 27 September 2016 |
| | | CA 2747197 A1 | 24 June 2010 |
| | | CN 102292348 A | 21 December 2011 |
| | | CN 102292348 B | 08 July 2015 |
| | | DK 2370462 T3 | 08 September 2014 |
| | | EA 201190048 A1 | 28 February 2012 |
| | | EP 2370462 A1 | 05 October 2011 |
| | | EP 2370462 B1 | 16 July 2014 |
| | | ES 2502218 T3 | 03 October 2014 |
| | | IL 213478 A | 31 July 2011 |
| | | JP 2012-511902 A | 31 May 2012 |
| | | JP 5635532 B2 | 03 December 2014 |
| | | KR 10-1593406 B1 | 12 February 2016 |
| | | KR 10-2011-0126590 A | 23 November 2011 |
| | | MX 2011006315 A | 22 September 2011 |
| | | NZ 593813 A | 22 February 2013 |
| | | PL 2370462 T3 | 30 January 2015 |
| | | US 2011-0286981 A1 | 24 November 2011 |
| | | WO 2010-070255 A1 | 24 June 2010 |
| | | ZA 201104593 B | 28 January 2015 |
| US 8263554 B2 | 11 September 2012 | US 2011-0306549 A1 | 15 December 2011 |
| | | US 2012-0295850 A1 | 22 November 2012 |
| | | US 8481490 B2 | 09 July 2013 |
| | | WO 2011-156407 A2 | 15 December 2011 |
| | | WO 2011-156407 A3 | 26 April 2012 |
| US 8454971 B2 | 04 June 2013 | AU 2008-216265 A1 | 21 August 2008 |
| | | AU 2008-216265 B2 | 03 April 2014 |
| | | AU 2013-204126 A1 | 02 May 2013 |

Form PCT/ISA/210 (patent family annex) (July 2019)

**INTERNATIONAL SEARCH REPORT**
**Information on patent family members**

International application No.
**PCT/KR2021/005481**

| Patent document cited in search report | Publication date (day/month/year) | Patent family member(s) | Publication date (day/month/year) |
|---|---|---|---|
| | | BR PI0807728 A2 | 17 April 2012 |
| | | CA 2677932 A1 | 21 August 2008 |
| | | CL 2008000479 A1 | 22 August 2008 |
| | | CL 4792008 A1 | 22 August 2008 |
| | | CN 101790538 A | 28 July 2010 |
| | | CN 101790538 B | 30 July 2014 |
| | | CR 11028 A | 29 October 2009 |
| | | EP 2111414 A2 | 28 October 2009 |
| | | EP 2111414 B1 | 02 July 2014 |
| | | EP 2487184 A1 | 15 August 2012 |
| | | GT 200900229 A | 21 January 2011 |
| | | HK 1135993 A1 | 18 June 2010 |
| | | HN 2009001593 A | 13 August 2012 |
| | | IL 200396 A | 29 April 2010 |
| | | IL 200396 B | 29 February 2016 |
| | | JP 2011-511753 A | 14 April 2011 |
| | | JP 2014-141500 A | 07 August 2014 |
| | | JP 6017754 B2 | 02 November 2016 |
| | | KR 10-2009-0119876 A | 20 November 2009 |
| | | KR 10-2015-0116465 A | 15 October 2015 |
| | | MA 31242 B1 | 01 March 2010 |
| | | MX 2009008241 A | 08 October 2009 |
| | | NI 200900158 A | 07 September 2010 |
| | | NZ 578948 A | 30 March 2012 |
| | | PA 8769301 A1 | 18 December 2008 |
| | | PE 01082009 A1 | 15 February 2009 |
| | | PE 01592014 A1 | 08 February 2014 |
| | | PE 20090108 A1 | 15 February 2009 |
| | | PE 20140159 A1 | 08 February 2014 |
| | | TN 2009000313 A1 | 31 December 2010 |
| | | TW 200848423 A | 16 December 2008 |
| | | TW I547499 B | 01 September 2016 |
| | | UA 104842 C2 | 25 March 2014 |
| | | US 2010-0190701 A1 | 29 July 2010 |
| | | US 2013-0203660 A1 | 08 August 2013 |
| | | US 2015-0126440 A1 | 07 May 2015 |
| | | US 8900593 B2 | 02 December 2014 |
| | | US 9447162 B2 | 20 September 2016 |
| | | WO 2008-101017 A2 | 21 August 2008 |
| | | WO 2008-101017 A3 | 18 December 2008 |
| | | ZA 200905521 B | 26 October 2011 |
| US 7491697 B2 | 17 February 2009 | AR 046877 A1 | 28 December 2005 |
| | | AU 2004-303783 A1 | 07 July 2005 |
| | | BR PI0416683 A | 30 January 2007 |
| | | CA 2549249 A1 | 07 July 2005 |
| | | CN 1890371 A | 03 January 2007 |
| | | CN 1890371 C | 03 January 2007 |
| | | EA 200601121 A1 | 27 October 2006 |
| | | EP 1702067 A1 | 20 September 2006 |
| | | EP 2270163 A1 | 05 January 2011 |

Form PCT/ISA/210 (patent family annex) (July 2019)

**INTERNATIONAL SEARCH REPORT**
**Information on patent family members**

International application No.
**PCT/KR2021/005481**

| Patent document cited in search report | Publication date (day/month/year) | Patent family member(s) | Publication date (day/month/year) |
|---|---|---|---|
| | | JP 2007-535306 A | 06 December 2007 |
| | | JP 4477013 B2 | 09 June 2010 |
| | | KR 10-2006-0135648 A | 29 December 2006 |
| | | NO 20062662 L | 07 August 2006 |
| | | TW 200520772 A | 01 July 2005 |
| | | US 2007-0142278 A1 | 21 June 2007 |
| | | US 2009-0118190 A1 | 07 May 2009 |
| | | WO 2005-061712 A1 | 07 July 2005 |
| US 9074014 B2 | 07 July 2015 | AU 2009-280017 A1 | 11 February 2010 |
| | | AU 2009-280017 B2 | 10 January 2013 |
| | | BR PI0916890 A2 | 24 September 2019 |
| | | CA 2732949 A1 | 11 February 2010 |
| | | CA 2732949 C | 20 December 2016 |
| | | CN 102171244 A | 31 August 2011 |
| | | CN 102171244 B | 13 May 2015 |
| | | CN 104829706 A | 12 August 2015 |
| | | EP 2328922 A2 | 08 June 2011 |
| | | EP 2987805 A2 | 24 February 2016 |
| | | EP 2987805 A3 | 13 April 2016 |
| | | JP 2011-530508 A | 22 December 2011 |
| | | JP 2014-028846 A | 13 February 2014 |
| | | JP 5865324 B2 | 17 February 2016 |
| | | KR 10-1417873 B1 | 09 July 2014 |
| | | KR 10-2011-0043686 A | 27 April 2011 |
| | | KR 10-2013-0093692 A | 22 August 2013 |
| | | MX 2011001031 A | 26 April 2011 |
| | | US 10756918 B2 | 25 August 2020 |
| | | US 2011-0136733 A1 | 09 June 2011 |
| | | US 2012-0131158 A1 | 24 May 2012 |
| | | US 2015-0252092 A1 | 10 September 2015 |
| | | US 8271629 B1 | 18 September 2012 |
| | | US 8667102 B1 | 04 March 2014 |
| | | US 9497261 B1 | 15 November 2016 |
| | | US 9497272 B1 | 15 November 2016 |
| | | WO 2010-016940 A2 | 11 February 2010 |
| | | WO 2010-016940 A3 | 15 April 2010 |
| US 7430476 B2 | 30 September 2008 | AU 2002-249224 B2 | 03 May 2007 |
| | | AU 2002-256624 B2 | 06 December 2007 |
| | | AU 2002-256624 B8 | 12 September 2002 |
| | | CA 2437214 A1 | 15 August 2002 |
| | | CA 2437263 A1 | 15 August 2002 |
| | | CA 2437265 A1 | 15 August 2002 |
| | | CA 2437270 A1 | 15 August 2002 |
| | | CA 2437272 A1 | 15 August 2002 |
| | | CA 2437287 A1 | 03 October 2002 |
| | | CA 2438513 A1 | 29 August 2002 |
| | | CA 2438513 C | 13 August 2013 |
| | | CA 2438652 A1 | 06 September 2002 |
| | | CN 1491231 A | 21 April 2004 |
| | | CN 1491232 A | 21 April 2004 |

Form PCT/ISA/210 (patent family annex) (July 2019)

**INTERNATIONAL SEARCH REPORT**
**Information on patent family members**

International application No.
**PCT/KR2021/005481**

| Patent document cited in search report | Publication date (day/month/year) | Patent family member(s) | Publication date (day/month/year) |
|---|---|---|---|
| | | CN 1492767 A | 28 April 2004 |
| | | CN 1493052 A | 28 April 2004 |
| | | CN 1493052 C | 23 July 2008 |
| | | CN 1514733 A | 21 July 2004 |
| | | CN 1514844 A | 21 July 2004 |
| | | CN 1527839 A | 08 September 2004 |
| | | CN 1549723 A | 24 November 2004 |
| | | EP 1357933 A2 | 05 November 2003 |
| | | EP 1357934 A1 | 05 November 2003 |
| | | EP 1360200 A2 | 12 November 2003 |
| | | EP 1360201 A1 | 12 November 2003 |
| | | EP 1361893 A1 | 19 November 2003 |
| | | EP 1361893 B1 | 24 October 2012 |
| | | EP 1366455 A2 | 03 December 2003 |
| | | EP 1366455 B1 | 02 July 2008 |
| | | EP 1387856 A2 | 11 February 2004 |
| | | EP 1392731 A2 | 03 March 2004 |
| | | EP 1392731 B1 | 02 July 2008 |
| | | EP 1998266 A2 | 03 December 2008 |
| | | EP 1998266 A3 | 11 February 2009 |
| | | ES 2309167 T3 | 16 December 2008 |
| | | ES 2309168 T3 | 16 December 2008 |
| | | ES 2398099 T3 | 13 March 2013 |
| | | JP 2004-519230 A | 02 July 2004 |
| | | JP 2004-519233 A | 02 July 2004 |
| | | JP 2004-522445 A | 29 July 2004 |
| | | JP 2004-523754 A | 05 August 2004 |
| | | JP 2004-526437 A | 02 September 2004 |
| | | JP 2004-527243 A | 09 September 2004 |
| | | JP 2004-532617 A | 28 October 2004 |
| | | JP 2004-532618 A | 28 October 2004 |
| | | JP 4279554 B2 | 17 June 2009 |
| | | KR 10-0899970 B1 | 28 May 2009 |
| | | KR 10-2003-0074784 A | 19 September 2003 |
| | | KR 10-2003-0074785 A | 19 September 2003 |
| | | KR 10-2003-0074788 A | 19 September 2003 |
| | | KR 10-2003-0074789 A | 19 September 2003 |
| | | KR 10-2003-0074790 A | 19 September 2003 |
| | | KR 10-2003-0074791 A | 19 September 2003 |
| | | KR 10-2003-0074839 A | 19 September 2003 |
| | | KR 10-2003-0075201 A | 22 September 2003 |
| | | PL 362375 A1 | 18 October 2004 |
| | | PL 362396 A1 | 02 November 2004 |
| | | PL 362397 A1 | 02 November 2004 |
| | | PL 362411 A1 | 02 November 2004 |
| | | PL 362412 A1 | 02 November 2004 |
| | | PL 362413 A1 | 02 November 2004 |
| | | PL 362414 A1 | 02 November 2004 |
| | | PL 363501 A1 | 29 November 2004 |
| | | PT 1361893 E | 24 January 2013 |

Form PCT/ISA/210 (patent family annex) (July 2019)

**INTERNATIONAL SEARCH REPORT**
**Information on patent family members**

International application No.
**PCT/KR2021/005481**

| Patent document cited in search report | Publication date (day/month/year) | Patent family member(s) | Publication date (day/month/year) |
|---|---|---|---|
| | | US 2004-0062749 A1 | 01 April 2004 |
| | | US 2004-0063634 A1 | 01 April 2004 |
| | | US 2004-0063917 A1 | 01 April 2004 |
| | | US 2004-0072219 A1 | 15 April 2004 |
| | | US 2004-0072291 A1 | 15 April 2004 |
| | | US 2004-0076991 A1 | 22 April 2004 |
| | | US 2004-0096442 A1 | 20 May 2004 |
| | | US 2004-0180386 A1 | 16 September 2004 |
| | | US 7132511 B2 | 07 November 2006 |
| | | US 7392141 B2 | 24 June 2008 |
| | | WO 02-062375 A1 | 15 August 2002 |
| | | WO 02-062832 A2 | 15 August 2002 |
| | | WO 02-062832 A3 | 13 March 2003 |
| | | WO 02-062833 A2 | 15 August 2002 |
| | | WO 02-062833 A3 | 30 October 2003 |
| | | WO 02-062842 A1 | 15 August 2002 |
| | | WO 02-062843 A2 | 15 August 2002 |
| | | WO 02-062843 A3 | 12 December 2002 |
| | | WO 02-066058 A1 | 29 August 2002 |
| | | WO 02-069232 A2 | 06 September 2002 |
| | | WO 02-069232 A3 | 13 February 2003 |
| | | WO 02-077034 A2 | 03 October 2002 |
| | | WO 02-077034 A3 | 11 December 2003 |
| | | ZA 200306924 A | 02 September 2004 |
| | | ZA 200306924 B | 02 September 2004 |
| | | ZA 200306926 A | 09 September 2004 |
| | | ZA 200306926 B | 09 September 2004 |
| | | ZA 200306930 A | 14 September 2004 |
| | | ZA 200306930 B | 14 September 2004 |
| | | ZA 200306931 A | 14 September 2004 |
| | | ZA 200306931 B | 14 September 2004 |
| | | ZA 200306933 A | 09 September 2004 |
| | | ZA 200306933 B | 09 September 2004 |
| | | ZA 200306934 A | 09 September 2004 |
| | | ZA 200306934 B | 09 September 2004 |
| | | ZA 200307324 A | 20 December 2004 |
| | | ZA 200307324 B | 23 February 2005 |
| | | ZA 200307329 A | 20 December 2004 |
| | | ZA 200307329 B | 23 February 2005 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020160032699
- KR 102034607

### Non-patent literature cited in the description

- *Metabolism Clinical and Experimental,* 2016, vol. 65, 1038-1048
- **LOW et al.** *Cooke and Bloom,* 2009